# EUROPEAN PATENT SPECIFICATION

(11) **EP 1 575 963 B1**
(45) Date of publication and mention of the grant of the patent: **07.05.2008**
(21) Application number: 03786136.6
(22) Date of filing: 19.12.2003
(51) Int. Cl.: C07D 513/04, C07D 471/04, C07D 403/06, C07D 401/06, A61K 31/519, A61P 35/00

(54) **ANTI-ANGIOGENETIC THERAPEUTIC AGENTS**
ANTI-ANGIOGENETISCHE ARZNEIMITTEL
AGENTS THERAPEUTIQUES ANTI-ANGIOGENIQUES

(30) Priority: 24.12.2002 GB 0230089
(43) Date of publication of application: 21.09.2005
(73) Proprietor: AstraZeneca AB, 151 85 Södertälje (SE)
(72) Inventor: LUKE, Richard W. A., AstraZeneca R & D Alderley, Macclesfield, Cheshire SK10 4TG (GB)
(86) International application number: PCT/GB2003/005568
(87) International publication number: WO 2004/058776

(56) References cited:
- WO-A-01/19829
- WO-A-01/37835
- WO-A-02/39954
- WO-A-20/04013141
- DATABASE CHEMCATS [Online] CHEMICAL ABSTRACTS SERVICE COLUMBUS, OHIO, US; XP002277085 retrieved from STN Database accession no. 2002:2408531 & "Interchim Intermediates" 9 July 2002 (2002-07-09), INTERCHIM , MONTLUCON, FRANCE

## Description

This invention relates to compounds, or pharmaceutically acceptable salts thereof, which possess anti-angiogentic activity and are accordingly useful in methods of treatment of disease states associated with angiogenesis in the animal or human body. The invention also concerns processes for the preparation of the compounds, pharmaceutical compositions containing the compounds as active ingredient, methods the use of the compounds in the manufacture of medicaments for use in the production of anti-angiogenic effects in warm-blooded animals such as humans.

4-[2-(6-phenylimidazo[2,1-b][1,3]thiazol-5-yl)vinyl]pyrimidin-2-amine is disclosed in Chemical abstracts Service Columbus, Ohio, US, XP002277085 & "Interchim Intermediate", Montlucan, France, 9 July 2002,

The Tie2 receptor tyrosine kinase (also known as TEK) is expressed predominantly in endothelial and haematopoietic cells and is essential for vessel formation and maintenance (Jones, N. et al. Nature Reviews Molecular Cell Biology. 2001: 2, 257-67).

Angiogenesis is a fundamental process defined as the generation of new blood vessels from existing vasculature. It is a vital yet complex biological process required for the formation and physiological functions of virtually all the organs. Normally it is transient in nature and is controlled by the local balance of angiogenic and angiostatic factors in a multi-step process involving vessel sprouting, branching and tubule formation by endothelial cells (involving processes such as activation of endothelial cells (ECs), vessel destabilisation, synthesis and release of degradative enzymes, EC migration, EC proliferation, EC organisation and differentiation and vessel maturation).

Normal angiogenesis plays an important role in a variety of processes and is under stringent control. In the adult, physiological angiogenesis is largely confined to wound healing and several components of female reproductive function and embryonic development. In undesirable or pathological angiogenesis, the local balance between angiogenic and angiostatic factors is dysregulated leading to inappropriate and/or structurally abnormal blood vessel formation. Pathological angiogenesis has been associated with disease states including diabetic retinopathy, psoriasis, cancer, rheumatoid arthritis, atheroma, Kaposi's sarcoma and haemangioma (Fan et al, 1995, Trends Pharmacology. Science. 16: 57-66; Folkman, 1995, Nature Medicine 1: 27-31). In cancer, growth of primary and secondary tumours beyond 1-2 mm³ requires angiogenesis (Folkman, J. New England Journal of Medicine 1995; 33, 1757-1763).

In diseases such as cancer in which progression is dependant on aberrant angiogenesis, blocking the process can lead to prevention of disease advancement (Folkman, J. 1995, Nature Medicine. 1: 27-31). Many factors are described in the scientific literature that are believed to play important critical roles in the regulation of angiogenesis. Two major classes of angiogenic factors are the vascular endothelial growth factor (VEGF) and the angiopoietins. These polypeptide moieties interact with their respective receptors (transmembrane tyrosine kinases which are predominantly endothelial cell specific) and induce cellular responses via ligand mediated signal transduction. It has been speculated that VEGF and the angiopoietins co-operate to regulate various aspects of the angiogenic process during both normal and pathological angiogenesis via signalling through their respective receptors.

Receptor tyrosine kinases (RTKs) are important in the transmission of biochemical signals across the plasma membrane of cells. These transmembrane molecules characteristically consist of an extracellular ligand-binding domain connected through a segment in the plasma membrane to an intracellular tyrosine kinase domain. Binding of ligand to the receptor results in stimulation of the receptor-associated tyrosine kinase activity that leads to phosphorylation of tyrosine residues on both the receptor and other intracellular molecules. These changes in tyrosine phosphorylation initiate a signalling cascade leading to a variety of cellular responses. To date, at least nineteen distinct RTK subfamilies, defined by amino acid sequence homology, have been identified. One of these subfamilies is presently comprised by the fms-like tyrosine kinase receptor, Flt or Flt1, the kinase insert domain-containing receptor, KDR (also referred to as Flk-1), and another fms-like tyrosine kinase receptor, Flt4. Two of these related RTKs, Flt and KDR, have been shown to bind VEGF with high affinity (De Vries et al, 1992, Science 255: 989-991; Terman et al, 1992, Biochem. Biophys. Res. Comm. 1992, 187: 1579-1586). Binding of VEGF to these receptors expressed in heterologous cells has been associated with changes in the tyrosine phosphorylation status of cellular proteins and calcium fluxes.

Recently a second family of predominantly endothelial cell specific receptors that regulate vessel destabilisation and maturation have been identified. The Tie receptors and their ligands, the angiopoietins, co-operate closely with VEGF during both normal and pathological angiogenesis. The transmembrane receptors Tie1 and Tie2, constitute a family of endothelial cell specific tyrosine kinase receptors involved in maintenance of blood vessel integrity and which are involved in angiogenic outgrowth and vessel remodelling. Structurally Tie1 and Tie2 share a number of features (e.g. the intracellular domains of both these receptors each contain a tyrosine kinase domain interrupted by a kinase insert region) and thus constitute a distinct RTK subfamily. Overall sequence identity between Tie1 and Tie2 receptors at the amino acid level is 44% while their intracellular domains exhibit 76% homology. Targeted disruption of the Tie1 gene results in a lethal phenotype characterised by extensive haemorrhage and poor microvessel integrity (Puri, M. et al. 1995 EMBO Journal:14:5884-5891). Transgenic mice deficient in Tie2 display defects in vessel sprouting and remodelling and display a lethal phenotype in mid gestation (E9.5-10.5) caused by severe defects in embryonic vasculature (Sato, T. et al. 1995 Nature 370: 70-74).

To date no ligands have been identified for Tie1 and little is known regarding its signalling abilities. However, Tie1 is believed to influence Tie2 signalling via heterodimerisation with the Tie2 receptor (hence potentially modulating the ability of Tie2 to autophosphorylate (Marron, M. et al. 2000 Journal of Biological Chemistry : 275, 39741-39746) and recent chimaeric Tie1 receptor studies have indicated that Tie-1 may inhibit apoptosis via the PI 3 kinase/Akt signal transduction pathway (Kontos, C.D., et al., 2002 Molecular and Cellular Biology: 22, 1704-1713). In contrast, a number of ligands, designated the angiopoietins have been identified for Tie2 of which Angiopoietin 1 (Ang1) is the best characterised. Binding of Ang1 induces tyrosine phosphorylation of the Tie2 receptor via autophosphorylation and subsequently activation of its signalling pathways via signal transduction. Ang2 has been reported to antagonise these effects in endothelial cells (Maisonpierre, P. et al. 1997 Science:277, 55-60). The knock-out and transgenic manipulation of Tie2 and its ligands suggest that stringent spatial and temporal control of Tie2 signalling is imperative for the correct development of new vasculature. There are also reports of at least another two ligands (Ang3 and Ang4) as well as the possibility of heterodimerisation between the angiopoietin ligands that has the potential to modify their activity (agonistic/antagonistic) on association with the receptor. Activation of the Tie2 receptor by Ang1 inhibits apoptosis (Papapetropoulos, A., et al., 2000 Journal of Biological Chemistry: 275 9102-9105), promotes sprouting in vascular endothelial cells (Witzenbicher, B., et al., 1998 Journal of Biological Chemistry : 273, 18514-18521) and in vivo promotes blood vessel maturation during angiogenesis and reduces the permeability and consequent leakage from adult microvessels (Thurston, G. et al., 2000 Nature Medicine : 6, 460-463). Thus activated Tie2 receptor is reported to be involved in the branching, sprouting and outgrowth of new vessels and recruitment and interaction of periendothelial support cells important in maintaining vessel integrity and overall appears to be consistent with promoting microvessel stability. Absence of Tie2 activation or inhibition of Tie2 auto phosphorylation may lead to a loss of vascular structure and matrix/cell contacts (Brindle, N., in press, 2002) and in turn may trigger endothelial cell death, especially in the absence of survival or growth stimuli. On the basis of the above reported effects due to Tie2 kinase activity, inhibiting Tie2 kinase may provide an anti-angiogenic effect and thus have application in the therapy of disease states associated with pathological angiogenesis. Tie2 expression has been shown to be up-regulated in the neovasculature of a variety of tumours (e.g. Peters, K.G. et al, (British Journal of Cancer 1998 ; 77,51-56) suggesting that inhibiting Tie2 kinase activity will result in anti-angiogenic activity. In support of this hypothesis, studies with soluble Tie2 receptor (extracellular domain) (Pengnian, L. et al., 1997, Journal of Clinical Investigation 1997 : 100, 2072-2078 and Pengnian, L. et al., 1998, Proceedings of the National Academy of Sciences 1998 : 95, 8829-8834) have shown anti-tumour activity in *in vivo* tumour models. In addition these experiments also indicate that disruption of the Tie2 signalling pathways in a normal healthy individual may be well tolerated as no adverse toxicities were observed in these studies.

Examination of human primary breast cancer samples and human and murine breast cancer cell lines (Stratmann, A., et al., 2001, International Journal of Cancer :91,273-282) indicate that Tie2 dependant pathways of tumour angiogenesis may exist alongside KDR dependant pathways and, in fact, may operate both independently (Siemeister G., et al., 1999 Cancer Research : 59,3185-3191) as well as in concert with each other (e.g. VEGF A and Ang1 reported to collaborate to induce angiogenesis and produce non-leaky mature vessels Thurston, G, et al., 1999 Science: 286,2511-2514). It is quite possible that a mix of such angiogenic processes even exist within a single tumour.

Tie2 has also been shown to play a role in the vascular abnormality called venous malformation (VM) (Mulliken, J.B. & Young, A.E. 1998, Vascular Birthmarks: W. B. Saunders, Philadelphia). Such defects can either be inherited or can arise sporadically. VM's are commonly found in the skin or mucosal membranes but can affect any organ. Typically lesions appear as a spongy, blue to purple vascular masses composed of numerous dilated vascular channels lined by endothelial cells. Among the inherited forms of this disease the most common defect appears to be a Tie2 kinase mutation C2545T in the Tie2 coding sequence (Calvert, J.T., et al., 1999 Human Molecular genetics: 8, 1279-1289), which produces a R849W amino acid substitution in the kinase domain.. Analysis of this Tie2 mutant indicates that it is constitutively activated even in the absence of ligand (Vikkula, M., et al., 1996 Cell : 87,1181-1190).

Upregulation of Tie2 expression has also been found within the vascular synovial pannus of arthritic joints in humans, which is consistent with the role of inappropriate neovascularisation.

Such examples provide further indications that inhibition of Tie2 phosphorylation and subsequent signal transduction will be useful in treating disorders and other occurrences of inappropriate neovascularisation. To date only a few inhibitors of Tie2 are known in the art. There is thus a need to identify additional Tie2 inhibitors that could exploit the full therapeutic potential of inhibiting/ modulating the Tie2 signalling pathways.

We have found that certain compounds possess inhibitory activity for the Tie2 receptor tyrosine kinase and accordingly have value in the treatment of disease states associated with pathological angiogenesis such as cancer, rheumatoid arthritis, and other diseases where active angiogenesis is undesirable.

According to a first aspect of the present invention there is provided a compound of the Formula I: 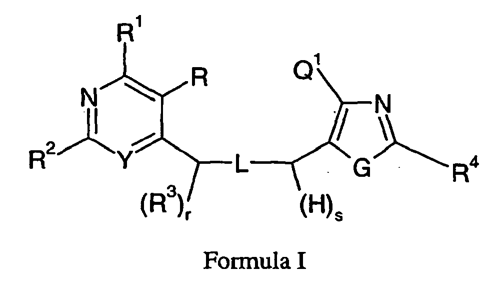 wherein:
- **-L-**: represents a double bond and r and s each represent 1 or -L- represents a triple bond and
- r and s: each represent 0;
- **G**: is selected from O, S and NR⁵;
- **Y**: is selected from N and CR⁶;
- **Q¹**: is selected from aryl and heteroaryl,
and wherein **Q¹** is optionally substituted by one or more substituents, which may be the same or different, selected from halogeno, trifluoromethyl, trifluoromethoxy, cyano, nitro, hydroxy, amino, carboxy, carbamoyl, (1-6C)alkyl, (2-8C)alkenyl, (2-8C)alkynyl, (1-6C)alkoxy, (2-6C)alkenyloxy, (2-6C)alkynyloxy, (1-6C)alkylthio, (1-6C)alkylsulfsulfinyl, (1-6C)alkylsulfsulfonyl, (1-6C)alkylamino, di-[(1-6C)alkyl]amino, (1-6C)alkoxycarbonyl, N-(1-6C)alkylcarbamoyl, N,N-di-[(1-6C)alkyl]carbamoyl, (2-6C)alkanoyl, (2-6C)alkanoyloxy, (2-6C)alkanoylamino, N-(1-6C)alkyl-(2-6C)alkanoylamino, (3-6C)alkenoylamino, N-(1-6C)alkyl-(3-6C)alkenoylamino, (3-6C)alkynoylamino, N-(1-6C)alkyl-(3-6C)alkynoylamino, N-(1-6C)alkylsulfsulfamoyl, N,N-di-[(1-6C)alkyl]sulfsulfamoyl, (1-6C)alkanesulfsulfonylamino, N-(1-6C)alkyl-(1-6C)alkanesulfsulfonylamino, from a group of the formula:

-X¹-R⁷

wherein X¹ is a direct bond or is selected from O and N(R⁸), wherein R⁸ is hydrogen or (1-6C)alkyl, and R⁷ is halogeno-(1-6C)alkyl, hydroxy-(1-6C)alkyl, (1-6C)alkoxy-(1-6C)alkyl, cyano-(1-6C)alkyl, amino-(1-6C)alkyl, (1-6C)alkylamino-(1-6C)alkyl or di-[(1-6C)alkyl]amino-(1-6C)alkyl, and from a group of the formula :

-X²-Q²

wherein X² is a direct bond or is selected from O, S, SO, SO₂, N(R⁹), CO, CH(OR⁹), CON(R⁹), N(R⁹)CO, N(R⁹)CON(R⁹), SO₂N(R⁹), N(R⁹)SO₂, C(R⁹)₂O, C(R⁹)₂S and N(R⁹)C(R⁹)₂, wherein R⁹ is hydrogen or (1-6C)alkyl, and Q² is aryl, aryl-(1-6C)alkyl, heteroaryl, heteroaryl-(1-6C)alkyl, heterocyclyl or heterocyclyl-(1-6C)alkyl which optionally bears 1, 2 or 3 substituents, which may be the same or different, selected from trifluoromethyl, trifluoromethoxy, cyano, nitro, hydroxy, amino, carboxy, carbamoyl; (1-6C)alkyl, (2-8C)alkenyl, (2-8C)alkynyl, (1-6C)alkoxy, (2-6C)alkenyloxy, (2-6C)alkynyloxy, (1-6C)alkylthio, (1-6C)alkylsulfinyl, (1-6C)alkylsulfonyl, (1-6C)alkylamino, di-[(1-6C)alkyl]amino, (1-6C)alkoxycarbonyl, N-(1-6C)alkylcarbamoyl, N,N-di-[(1-6C)alkyl]carbamoyl, (2-6C)alkanoyl, (2-6C)alkanoyloxy, (2-6C)alkanoylamino, N-(1-6C)alkyl-(2-6C)alkanoylamino, N-(1-6C)alkylsulfamoyl, N,N-di-[(1-6C)alkyl]sulfamoyl, (1-6C)alkanesulfonylamino and N-(1-6C)alkyl-(1-6C)alkanesulfonylamino, or from a group of the formula:

-X³-R¹⁰

wherein X³ is a direct bond or is selected from O and N(R¹¹), wherein R¹¹ is hydrogen or (1-6C)alkyl, and R¹⁰ is halogeno-(1-6C)alkyl, hydroxy-(1-6C)alkyl, (1-6C)alkoxy-(1-6C)alkyl, cyano-(1-6C)alkyl, amino-(1-6C)alkyl, (1-6C)alkylamino-(1-6C)alkyl or di-[(1-6C)alkyl]amino-(1-6C)alkyl,
and any heterocyclyl group within Q² optionally bears 1 or 2 oxo or thioxo substituents;
- **R**: is selected from hydrogen, amino, hydroxy, halogeno, (1-6C)alkyl, (1-6C)alkoxy, (1-6C)alkylamino, di-[(1-6C)alkyl]amino, carboxy, (1-6C)alkoxycarbonyl and N-(heterocyclyl(3-8C)cycloalkyl)carbamoyl;
- **R¹**: is selected from hydrogen, halogeno, trifluoromethyl, trifluoromethoxy, cyano, nitro, hydroxy, amino, mercapto, carbamoyl, (1-6C)alkyl, (2-8C)alkenyl, (2-8C)alkynyl, (1-6C)alkoxy, (2-6C)alkenyloxy, (2-6C)alkynyloxy, (1-6C)alkylthio, (1-6C)alkylsulfinyl, (1-6C)alkylsulfonyl, (1-6C)alkylamino, di-[(1-6C)alkyl]amino, (1-6C)alkoxycarbonyl, N-(1-6C)alkylcarbamoyl, N,N-di-[(1-6C)alkyl]carbamoyl, (2-6C)alkanoyl, (2-6C)alkanoyloxy, (2-6C)alkanoylamino, N-(1-6C)alkyl-(2-6C)alkanoylamino, (3-6C)alkenoylamino, N-(1-6C)alkyl-(3-6C)alkenoylamino, (3-6C)alkynoylamino, N-(1-6C)alkyl-(3-6C)alkynoylamino, N-(1-6C)alkylsulfamoyl, N,N-di-[(1-6C)alkyl]sulfamoyl, (1-6C)alkanesulfonylamino and N-(1-6C)alkyl-(1-6C)alkanesulfonylamino;
- **R²**: is selected from hydrogen, halogeno, amino, hydroxy, halogeno, (1-6C)alkyl, (1-6C)alkoxy, (1-6C)alkylthio, (1-6C)alkylamino, di-[(1-6C)alkyl]amino, aryl(1-6C)alkylamino, arylamino, heterocyclyl and (2-6C)alkanoylamino;
- **R³**: is selected from hydrogen, (1-6C)alkyl, hydroxy(1-6C)alkyl, carboxy, (1-6C)alkoxycarbonyl, carbamoyl, N-(1-6C)alkylcarbamoyl, N,N-di-[(1-6C)alkyl]carbamoyl and *N*-(heterocyclyl(3-8C)cycloalkyl)carbamoyl;
- **R⁵**: is, independently, as defined for R⁴ and R⁶, provided that R⁵ is not halogeno;
- **R⁴** and **R⁶**: which may be the same or different, are selected from hydrogen, halogeno, trifluoromethyl, trifluoromethoxy, cyano, isocyano, nitro, hydroxy, mercapto, amino, formyl, carboxy, carbamoyl, sulfamoyl, (1-6C)alkyl, (2-8C)alkenyl, (2-8C)alkynyl, (1-6C)alkoxy, (2-6C)alkenyloxy, (2-6C)alkynyloxy, (1-6C)alkylthio, (1-6C)alkylsulfinyl, (1-6C)alkylsulfonyl, (1-6C)alkylamino, di-[(1-6C)alkyl]amino, (1-6C)alkoxycarbonyl, N-(1-6C)alkylcarbamoyl, N,N-di-[(1-6C)alkyl]carbamoyl, (2-6C)alkanoyl, (2-6C)alkanoyloxy, (2-6C)alkanoylamino, N-(1-6C)alkyl-(2-6C)alkanoylamino, (3-6C)alkenoylamino, N-(1-6C)alkyl-(3-6C)alkenoylamino, (3-6C)alkynoylamino, N-(1-6C)alkyl-(3-6C)alkynoylamino, N-(1-6C)alkylsulfamoyl, N,N-di-[(1-6C)alkyl]sulfamoyl, (1-6C)alkanesulfonylamino and N-(1-6C)alkyl-(1-6C)alkanesulfonylamino, or from a group of the formula :

Q⁴-X⁵-
wherein X⁵ is a direct bond or is selected from O, S, SO, SO₂, N(R¹²), CO, CH(OR¹²), CON(R¹²), N(R¹²)CO, SO₂N(R¹²), N(R¹²)SO₂, OC(R¹²)₂, SC(R¹²)₂ and N(R¹²)C(R¹²)₂, wherein R¹² is hydrogen or (1-6C)alkyl, and Q⁴ is aryl, aryl-(1-6C)alkyl, (3-7C)cycloalkyl, (3-7C)cycloalkyl-(1-6C)alkyl, (3-7C)cycloalkenyl, (3-7C)cycloalkenyl-(1-6C)alkyl, heteroaryl, heteroaryl-(1-6C)alkyl, heterocyclyl or heterocyclyl-(1-6C)alkyl,
and wherein adjacent carbon atoms in any (2-6C)alkylene chain within an R⁴, R⁵ or R⁶ substituent are optionally separated by the insertion into the chain of a group selected from O, S, SO, SO₂, N(R¹³), CO, CR(OR¹³), CON(R¹³), N(R¹³)CO, SO₂N(R¹³), N(R¹³)SO₂, CH=CH and C≡C wherein R¹³ is hydrogen or (1-6C)alkyl,
and wherein any CH₂=CH- or HC≡C- group within an R⁴, R⁵ or R⁶ substituent optionally bears at the terminal CH₂= or HC≡ position a substituent selected from halogeno, carboxy, carbamoyl, (1-6C)alkoxycarbonyl, N-(1-6C)alkylcarbamoyl, N,N-di-[(1-6C)alkyl]carbamoyl, amino-(1-6C)alkyl, (1-6C)alkylamino-(1-6C)alkyl and di-[(1-6C)alkyl]amino-(1-6C)alkyl or from a group of the formula :

Q⁵-X⁶-

wherein X⁶ is a direct bond or is selected from CO and N(R¹⁴)CO, wherein R¹⁴ is hydrogen or (1-6C)alkyl, and Q⁵ is aryl, aryl-(1-6C)alkyl, heteroaryl, heteroaryl-(1-6C)alkyl, heterocyclyl or heterocyclyl-(1-6C)alkyl,
and wherein any CH₂ or CH₃ group within a R⁴, R⁵ or R⁶ substituent optionally bears on each said CH₂ or CH₃ group one or more halogeno or (1-6C)alkyl substituents or a substituent selected from hydroxy, cyano, amino, carboxy, carbamoyl, (1-6C)alkoxy, (1-6C)alkylthio, (1-6C)alkylsulfinyl, (1-6C)alkylsulfonyl, (1-6C)alkylamino, di-[(1-6C)alkyl]amino, (1-6C)alkoxycarbonyl, N-(1-6C)alkylcarbamoyl, N,N-di-[(1-6C)alkyl]carbamoyl, (2-6C)alkanoyl, (2-6C)alkanoyloxy, (2-6C)alkanoylamino, N-(1-6C)alkyl-(2-6C)alkanoylamino, N-(1-6C)alkylsulfamoyl, N,N-di-[(1-6C)alkyl]sulfamoyl, (1-6C)alkanesulfonylamino and N-(1-6C)alkyl-(1-6C)alkanesulfonylamino, or from a group of the formula :

-X⁷-Q⁶

wherein X⁷ is a direct bond or is selected from O, S, SO, SO₂, N(R¹⁵), CO, CH(OR¹⁵), CON(R¹⁵), N(R¹⁵)CO, SO₂N(R¹⁵), N(R¹⁵)SO₂, C(R¹⁵)₂O, C(R¹⁵)₂S and N(R¹⁵)C(R¹⁵)₂, wherein R¹⁵ is hydrogen or (1-6C)alkyl, and Q⁶ is aryl, aryl-(1-6C)alkyl, (3-7C)cycloalkyl, (3-7C)cycloalkyl-(1-6C)alkyl, (3-7C)cycloalkenyl, (3-7C)cycloalkenyl-(1-6C)alkyl, heteroaryl, heteroaryl-(1-6C)alkyl, heterocyclyl or heterocyclyl-(1-6C)alkyl,
and wherein any aryl, heteroaryl, heterocyclyl, cycloalkyl or cycloalkenyl group within a substituent on R⁴, R⁵ or R⁶ optionally bears 1 or more substituents, which may be the same or different, selected from halogeno, trifluoromethyl, trifluoromethoxy, cyano, nitro, hydroxy, amino, carboxy, carbamoyl, (1-6C)alkyl, (2-8C)alkenyl, (2-8C)alkynyl, (1-6C)alkoxy, (2-6C)alkenyloxy, (2-6C)alkynyloxy, (1-6C)alkylthio, (1-6C)alkylsulfmyl, (1-6C)alkylsulfonyl, (1-6C)alkylamino, di-[(1-6C)alkyl]amino, (1-6C)alkoxycarbonyl, N-(1-6C)alkylcarbamoyl, N,N-di-[(1-6C)alkyl]carbamoyl, (2-6C)alkanoyl, (2-6C)alkanoyloxy, (2-6C)alkanoylamino, N-(1-6C)alkyl-(2-6C)alkanoylamino, N-(1-6C)alkylsulfamoyl, N,N-di-[(1-6C)alkyl]sulfamoyl, (1-6C)alkanesulfonylamino, N-(1-6C)alkyl-(1-6C)alkanesulfonylamino, from a group of the formula :

-X⁸-R¹⁶

wherein X⁸ is a direct bond or is selected from O and N(R¹⁷), wherein R¹⁷ is hydrogen or (1-6C)alkyl, and R¹⁶ is halogeno-(1-6C)alkyl, hydroxy-(1-6C)alkyl, (1-6C)alkoxy-(1-6C)alkyl, cyano-(1-6C)alkyl, amino-(1-6C)alkyl, (1-6C)alkylamino-(1-6C)alkyl, di-[(1-6C)alkyl]amino-(1-6C)alkyl, (2-6C)alkanoylamino-(1-6C)alkyl or (1-6C)alkoxycarbonylamino-(1-6C)alkyl, and from a group of the formula :

-X⁹-Q⁷

wherein X⁹ is a direct bond or is selected from O, S, SO, SO₂, N(R¹⁸), CO, CH(OR¹⁸), CON(R¹⁸), N(R¹⁸)CO, SO₂N(R¹⁸), N(R¹⁸)SO₂, C(R¹⁸)₂O, C(R¹⁸)₂S and N(R¹⁸)C(R¹⁸)₂, wherein R¹⁸ is hydrogen or (1-6C)alkyl, and Q⁷ is aryl, aryl-(1-6C)alkyl, (3-7C)cycloalkyl, (3-7C)cycloalkyl-(1-6C)alkyl, (3-7C)cycloalkenyl, (3-7C)cycloalkenyl-(1-6C)alkyl, heteroaryl, heteroaryl-(1-6C)alkyl, heterocyclyl or heterocyclyl-(1-6C)alkyl which optionally bears 1 or 2 substituents, which may be the same or different, selected from halogeno, trifluoromethyl, trifluoromethoxy, cyano, nitro, hydroxy, amino, carboxy, carbamoyl, (1-6C)alkyl, (2-8C)alkenyl, (2-8C)alkynyl, (1-6C)alkoxy, (2-6C)alkenyloxy, (2-6C)alkynyloxy, (1-6C)alkylthio, (1-6C)alkylsulfinyl, (1-6C)alkylsulfonyl, (1-6C)alkylamino, di-[(1-6C)alkyl]amino, (1-6C)alkoxycarbonyl, N-(1-6C)alkylcarbamoyl, N,N-di-[(1-6C)alkyl]carbamoyl, (2-6C)alkanoyl, (2-6C)alkanoyloxy, (2-6C)alkanoylamino, N-(1-6C)alkyl-(2-6C)alkanoylamino, N-(1-6C)alkylsulfamoyl, N,N-di-[(1-6C)alkyl]sulfamoyl, (1-6C)alkanesulfonylamino and N-(1-6C)alkyl-(1-6C)alkanesulfonylamino,
or when G is NR⁵, R⁴ and R⁵ together with the atoms to which they are attached form a fused 5- or 6- membered heteroaryl or heterocyclyl ring, and wherein said fused 5- or 6-membered ring optionally bears one or more substituents as defined for R⁴,
and any fused 5- or 6- membered heterocyclyl ring so formed optionally bears 1 or 2 oxo or thioxo substituents,
and wherein any heterocyclyl group within any R⁴, R⁵ or R⁶ substituent optionally bears 1 or 2 oxo or thioxo substituents;
or a pharmaceutically-acceptable salt thereof.

In this specification the generic term "alkyl" includes both straight-chain and branched-chain alkyl groups such as propyl, isopropyl and tert-butyl. However references to individual alkyl groups such as "propyl" are specific for the straight-chain version only, references to individual branched-chain alkyl groups such as "isopropyl" are specific for the branched-chain version only. An analogous convention applies to other generic terms, for example (1-6C)alkoxy includes methoxy, ethoxy, (1-6C)alkylamino includes methylamino and ethylamino, and di-[(1-6Calkyl]amino includes dimethylamino and diethylamino.

It is to be understood that, insofar as certain of the compounds of Formula I defined above may exist in optically active or racemic forms by virtue of one or more asymmetric carbon atoms, the invention includes in its definition any such optically active or racemic form which possesses the above-mentioned activity. The synthesis of optically active forms may be carried out by standard techniques of organic chemistry well known in the art, for example by synthesis from optically active starting materials or by resolution of a racemic form. Similarly, the above-mentioned activity may be evaluated using the standard laboratory techniques referred to hereinafter.

It is to be understood that, insofar as certain of the compounds of Formula I defined above may exist in geometrically isomeric forms by virtue of one or more carbon carbon double bonds, the invention includes in its definition any and all such geometrically isomeric forms for example E and Z forms which possess the above-mentioned activity.

Suitable values for the generic radicals referred to above include those set out below.

A suitable value for any one of the 'Q' groups (Q¹ to Q⁷) when it is aryl or for the aryl group within a 'Q' group is, for example, phenyl or naphthyl, preferably phenyl.

A suitable value for any one of the 'Q' groups (Q⁴, Q⁶ or Q⁷) when it is (3-7C)cycloalkyl or for the (3-7C)cycloalkyl group within a 'Q' group is, for example, cyclopropyl, cyclobutyl, cyclopentyl, cyclohexyl, cycloheptyl or bicyclo[2.2.1]heptyl and a suitable value for any one of the 'Q' groups (Q⁴, Q⁶ or Q⁷) when it is (3-7C)cycloalkenyl or for the (3-7C)cycloalkenyl group within a 'Q' group is, for example, cyclobutenyl, cyclopentenyl, cyclohexenyl or cycloheptenyl.

A suitable value for any one of the 'Q' groups (Q¹, Q² or Q⁴ to Q⁷) when it is heteroaryl or for the heteroaryl group within a 'Q' group is, for example, an aromatic 5- or 6-membered monocyclic ring or unless specified otherwise, a 9- or 10-membered bicyclic ring, with up to five ring heteroatoms selected from oxygen, nitrogen and sulfur, which may, unless otherwise specified be carbon or nitrogen linked. Preferably heteroaryl is an aromatic 5- or 6-membered monocyclic ring with up to five ring heteroatoms selected from oxygen, nitrogen and sulfur, which may, unless otherwise specified be carbon or nitrogen linked. Suitable heteroaryl rings include, for example furyl, pyrrolyl, thienyl, oxazolyl, isoxazolyl, imidazolyl, pyrazolyl, thiazolyl, isothiazolyl, oxadiazolyl, thiadiazolyl, triazolyl, tetrazolyl, pyridyl, pyridazinyl, pyrimidinyl, pyrazinyl, 1,3,5-triazenyl, benzofuranyl, indolyl, benzothienyl, benzoxazolyl, benzimidazolyl, benzothiazolyl, indazolyl, benzofurazanyl, quinolyl, isoquinolyl, quinazolinyl, quinoxalinyl, cinnolinyl or naphthyridinyl. Preferably furyl, pyrrolyl, thienyl, oxazolyl, isoxazolyl, imidazolyl, pyrazolyl, thiazolyl, isothiazolyl, oxadiazolyl, thiadiazolyl, triazolyl, tetrazolyl, pyridyl, pyridazinyl, pyrimidinyl, pyrazinyl or 1,3,5-triazenyl.

The term "heterocyclyl" as used herein, for example for any one of the 'Q' groups (Q² to Q⁷) when it is heterocyclyl or for the heterocyclyl group within a 'Q' group means a non-aromatic saturated or partially saturated 3 to 10 membered monocyclic or bicyclic ring with up to five heteroatoms selected from oxygen, nitrogen and sulfur, which may, unless specified otherwise, be carbon or nitrogen linked, wherein a ring sulfur atom may be oxidized to form the S-oxide(s). Preferably a heterocyclyl is a non-aromatic saturated or partially saturated 5 or 6 membered monocyclic ring with 1, 2, 3 or 4 heteroatoms selected from oxygen, nitrogen and sulfur, which may, unless specified otherwise, be carbon or nitrogen linked, wherein a ring sulfur atom may be oxidized to form the S-oxide(s). Suitable heterocyclyls include, for example oxiranyl, oxetanyl, tetrahydrofuranyl, tetrahydropyranyl, 2,3-dihydro-1,3-thiazolyl, 1,3-thiazolidinyl, oxepanyl, pyrrolinyl, pyrrolidinyl, morpholinyl, tetrahydro-1,4-thiazinyl, 1-oxotetrahydrothienyl, 1,1-dioxotetrahydro-1,4-thiazinyl, piperidinyl, homopiperidinyl, piperazinyl, homopiperazinyl, dihydropyridinyl, tetrahydropyridinyl, dihydropyrimidinyl or tetrahydropyrimidinyl, preferably tetrahydrofuranyl, tetrahydropyranyl, pyrrolidinyl, morpholinyl, 1,1-dioxotetrahydro-4H-1,4-thiazinyl, piperidinyl or piperazinyl, more preferably tetrahydrofuran-3-yl, tetrahydropyran-4-yl, pyrrolidin-3-yl, morpholino, 1,1-dioxotetrahydro-4H-1,4-thiazin-4-yl, piperidino, piperidin-4-yl or piperazin-1-yl. A suitable value for such a group which bears 1 or 2 oxo or thioxo substituents is, for example, 2-oxopyrrolidinyl, 2-thioxopyrrolidinyl, 2-oxoimidazolidinyl, 2-thioxoimidazolidinyl, 2-oxopiperidinyl, 2,5-dioxopyrrolidinyl, 2,5-dioxoimidazolidinyl or 2,6-dioxopiperidinyl.

A suitable value for a 'Q' group when it is heteroaryl-(1-6C)alkyl is, for example, heteroarylmethyl, 2-heteroarylethyl and 3-heteroarylpropyl. The invention comprises corresponding suitable values for 'Q' groups when, for example, rather than a heteroaryl-(1-6C)alkyl group, an aryl-(1-6C)alkyl (such as phenyl-(1-6C)alkyl for example benzyl or phenylethyl), (3-7C)cycloalkyl-(1-6C)alkyl, (3-7C)cycloalkenyl-(1-6C)alkyl or heterocyclyl-(1-6C)alkyl group is present.

A suitable value when G is NR⁵ and R⁴ and R⁵ together with the atoms to which they are attached form a fused 5- or 6- membered heteroaryl or heterocyclyl ring, include for example the divalent derivatives of the 5- and 6- membered heteroaryl and heterocyclyl rings mentioned hereinbefore for the "Q" groups that contain at least 1 nitrogen atom, for example, thiazolo, isothiazolo, 1,3-thiazolidino, pyrrolidino, pyrrolino, oxazolo, isoxazolo, pyrazolino, pyridino, pyrimidino or pyridazino. As will be understood the ring formed by R⁴ and R⁵ is fused to the ring containing G in formula I to form a 5,5 or 5,6 bicyclic ring structure, by way of example R⁴ and R⁵ together with the atoms to which they are attached may form, for example an imidazo[2,1-*b*][1,3]thiazolyl, 2,3-dihydroimidazo[2,1-*b*][1,3]thiazolyl fused bicyclic ring or imidazo[1,2-*a*]pyridinyl fused bicyclic ring.

For the avoidance of any doubt there are no substituents labelled X⁴ or Q³ included in this specification.

Suitable values for any of the 'R' groups (R¹ to R¹⁸), or for various groups within an R¹ to R⁶ substituent, or for various groups within Q¹:-

| | |
|---|---|
| for halogeno | fluoro, chloro, bromo and iodo; |
| for (1-6C)alkyl: | methyl, ethyl, propyl, isopropyl and tert-butyl; |
| for (2-8C)alkenyl: | vinyl, isopropenyl, allyl and but-2-enyl; |
| for (2-8C)alkynyl: | ethynyl, 2-propynyl and but-2-ynyl; |
| for (1-6C)alkoxy: | methoxy, ethoxy, propoxy, isopropoxy and butoxy; |
| for (2-6C)alkenyloxy: | vinyloxy and allyloxy; |
| for (2-6C)alkynyloxy: | ethynyloxy and 2-propynyloxy; |
| for (1-6C)alkylthio: | methylthio, ethylthio and propylthio; |
| for (1-6C)alkylsulfinyl: | methylsulfinyl and ethylsulfinyl; |
| for (1-6C)alkylsulfonyl: | methylsulfonyl and ethylsulfonyl; |
| for (1-6C)alkylamino: | methylamino, ethylamino, propylamino, isopropylamino and butylamino; |
| for di-[(1-6C)alkyl]amino: | dimethylamino, diethylamino, N-ethyl-N-methylamino and diisopropylamino; |
| for (1-6C)alkoxycarbonyl: | methoxycarbonyl, ethoxycarbonyl, propoxycarbonyl and tert-butoxycarbonyl; |
| for N-(1-6C)alkylcarbamoyl: | N-methylcarbamoyl, N-ethylcarbamoyl and N-propylcarbamoyl; |
| for N,N-di-[(1-6C)alkyl]carbamoyl: | N,N-dimethylcarbamoyl, N-ethyl-N-methylcarbamoyl and N,N-diethylcarbamoyl; |
| for (2-6C)alkanoyl: | acetyl and propionyl; |
| for (2-6C)alkanoyloxy: | acetoxy and propionyloxy; |
| for (2-6C)alkanoylamino: | acetamido and propionamido; |
| for N-(1-6C)alkyl-(2-6C)alkanoylamino: | N-methylacetamido and N-methylpropionamido; |
| for N-(1-6C)alkylsulfamoyl: | N-methylsulfamoyl and N-ethylsulfamoyl; |
| for N,N-di-[(1-6C)alkyl]sulfamoyl: | N,N-dimethylsulfamoyl; |
| for (1-6C)alkanesulfonylamino: | methanesulfonylamino and ethanesulfonylamino; |
| for N-(1-6C)alkyl-(1-6C)alkanesulfonylamino: | N-ethylmethanesulfonylamino and N-methylethanesulfonylamino; |
| for (3-6C)alkenoylamino: | acrylamido, methacrylamido and crotonamido; |
| for N-(1-6C)alkyl-(3-6C)alkenoylamino: | N-methylacrylamido and N-methylcrotonamido; |
| for (3-6C)alkynoylamino: | propiolamido; |
| for N-(1-6C)alkyl-(3-6C)alkynoylamino: | N-methylpropiolamido; |
| for amino-(1-6C)alkyl: | aminomethyl, 2-aminoethyl, 1-aminoethyl and 3- |
| | aminopropyl; |
| for (1-6C)alkylamino-(1-6C)alkyl: | methylaminomethyl, ethylaminomethyl, 1-methylaminoethyl, 2-methylaminoethyl, 2-ethylaminoethyl and 3-ethylaminopropyl; |
| for di-[(1-6C)alkyl]amino-(1-6C)alkyl: | dimethylaminomethyl, diethylaminomethyl, |
| | 1-dimethylaminoethyl, 2-dimethylaminoethyl and 3-dimethylaminopropyl; |
| for halogeno-(1-6C)alkyl: | chloromethyl, 2-chloroethyl, 1-chloroethyl and 3-chloropropyl; |
| for hydroxy-(1-6C)alkyl: | hydroxymethyl, 2-hydroxyethyl, 1-hydroxyethyl and 3-hydroxypropyl; |
| for (1-6C)alkoxy-(1-6C)alkyl: | methoxymethyl, ethoxymethyl, 1-methoxyethyl, 2-methoxyethyl, 2-ethoxyethyl and 3-methoxypropyl; |
| for cyano-(1-6C)alkyl: | cyanomethyl, 2-cyanoethyl, 1-cyanoethyl and 3-cyanopropyl; |
| for (1-6C)alkylthio-(1-6C)alkyl: | methylthiomethyl, ethylthiomethyl, 2-methylthioethyl, 1-methylthioethyl and 3-methylthiopropyl; |
| for (1-6C)alkylsulfinyl-(1-6C)alkyl: | methylsulfinylmethyl, ethylsulfinylmethyl, 2-methylsulfinylethyl, 1-methylsulfinylethyl and 3-methylsulfinylpropyl; |
| for (1-6C)alkylsulfonyl-(1-6C)alkyl: | methylsulfonylmethyl, ethylsulfonylmethyl, 2-methylsulfonylethyl, 1-methylsulfonylethyl and 3-methylsulfonylpropyl; |
| for (2-6C)alkanoylamino-(1-6C)alkyl: | acetamidomethyl, propionamidomethyl and 2-acetamidoethyl; and |
| for (1-6C)alkoxycarbonylamino-(1-6C)alkyl: | methoxycarbonylaminomethyl, methoxycarbonylaminomethyl, tert-butoxycarbonylaminomethyl and 2-methoxycarbonylaminoethyl. |

When, as defined hereinbefore, an R⁶ group forms a group of the Formula Q⁴-X⁵- and, for example, X⁵ is a OC(R¹²)₂ linking group, it is the carbon atom, not the oxygen atom, of the OC(R¹²)₂ linking group which is attached to the pyridine ring and the oxygen atom is attached to the Q⁴ group. Similarly, when, for example a CH₃ group within an R⁴, R⁵ or R⁶ substituent bears a group of the formula -X⁷-Q⁶ and, for example, X⁷ is a C(R¹⁵)₂O linking group, it is the carbon atom, not the oxygen atom, of the C(R¹⁵)₂O linking group which is attached to the CH₃ group and the oxygen atom is linked to the Q⁶ group.

As defined hereinbefore, adjacent carbon atoms in any (2-6C)alkylene chain within an R⁴, R⁵ or R⁶ substituent may be optionally separated by the insertion into the chain of a group such as O, CON(R¹³) or C≡C. For example, insertion of a C≡C group into the ethylene chain within a 2-morpholinoethoxy group gives rise to a 4-morpholinobut-2-ynyloxy group and, for example, insertion of a CONH group into the ethylene chain within a 3-methoxypropoxy group gives rise to, for example, a 2-(2-methoxyacetamido)ethoxy group.

When, as defined hereinbefore, any CH₂=CH- or HC≡C- group within an R⁴ R⁵ or R⁶ substituent optionally bears at the terminal CH₂= or HC≡ position a substituent such as a group of the formula Q⁵-X⁶ - wherein X⁶ is, for example, NHCO and Q⁵ is a heterocyclyl-(1-6C)alkyl group, suitable R³, R⁴, R⁵ or R⁶ substituents so formed include, for example, N-[heterocyclyl-(1-6C)alkyl]carbamoylvinyl groups such as N-(2-pyrrolidin-1-ylethyl)carbamoylvinyl or N-[heterocyclyl-(1-6C)alkyl]carbamoylethynyl groups such as N-(2-pyrrolidin-1-ylethyl)carbamoylethynyl.

When, as defined hereinbefore, any CH₂ or CH₃ group within an R⁴, R⁵ or R⁶ substituent optionally bears on each said CH₂ or CH₃ group one or more halogeno or (1-6C)alkyl substituents, there are suitably 1 or 2 halogeno or (1-6C)alkyl substituents present on each said CH₂ group and there are suitably 1, 2 or 3 such substituents present on each said CH₃ group.

When, as defined hereinbefore, any CH₂ or CH₃ group within a R⁴, R⁵ or R⁶ substituent optionally bears on each said CH₂ or CH₃ group a substituent as defined hereinbefore, suitable substituents so formed include, for example, hydroxy-substituted heterocyclyl-(1-6C)alkoxy groups such as 2-hydroxy-3-piperidinopropoxy and 2-hydroxy-3-morpholinopropoxy, hydroxy-substituted amino-(2-6C)alkoxy groups such as 3-amino-2-hydroxypropoxy, hydroxy-substituted (1-6C)alkylamino-(2-6C)alkoxy groups such as 2-hydroxy-3-methylaminopropoxy, hydroxy-substituted di-[(1-6C)alkyl]amino-(2-6C)alkoxy groups such as 3-dimethylamino-2-hydroxypropoxy, hydroxy-substituted heterocyclyl-(1-6C)alkylamino groups such as 2-hydroxy-3-piperidinopropylamino and 2-hydroxy-3-morpholinopropylamino, hydroxy-substituted amino-(2-6C)alkylamino groups such as 3-amino-2-hydroxypropylamino, hydroxy-substituted (1-6C)alkylamino-(2-6C)alkylamino groups such as 2-hydroxy-3-methylaminopropylamino, hydroxy-substituted di-[(1-6C)alkyl]amino-(2-6C)alkylamino groups such as 3-dimethylamino-2-hydroxypropylamino, hydroxy-substituted (1-6C)alkoxy groups such as 2-hydroxyethoxy, (1-6C)alkoxy-substituted (1-6C)alkoxy groups such as 2-methoxyethoxy and 3-ethoxypropoxy, (1-6C)alkylsulfonyl-substituted (1-6C)alkoxy groups such as 2-methylsulfonylethoxy and heterocyclyl-substituted (1-6C)alkylamino-(1-6C)alkyl groups such as 2-morpholinoethylaminomethyl, 2-piperazin-1-ylethylaminomethyl and 3-morpholinopropylaminomethyl.

When, as defined hereinbefore, any cycloalkyl or cycloalkenyl group within an R⁴, R⁵ or R⁶ substituent optionally bears one or more substituent(s) as defined hereinbefore, the substituent may be present on any CH₂ or CH group within the cycloalkyl or cycloalkenyl group. Suitable substituents so formed include, for example, hydroxy-substituted (3-7C)cycloalkyl groups such as 1-hydroxycyclohex-1-yl or 1-hydroxycycloprop-1-yl, (3-7C)cycloalkyl-(1-6C)alkyl groups such as 2-(1-hydroxycyclohex-1-yl)ethyl, 2-(1-hydroxycyclohex-4-yl)ethyl 3-(1-hydroxycyclohex-1-yl)propyl or 3-(1-hydroxycyclopent-1-yl)propyl, or (3-7C)cycloalkyl-(1-6C)alkoxy groups such as 2-(1-hydroxycyclohex-1-yl)ethoxy or 3-(1-hydroxycyclohex-1-yl)propoxy.

A particular group of compounds of Formula I is represented by Formula II 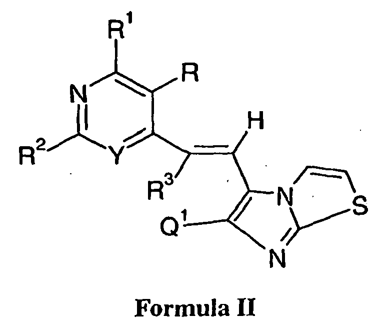 in which of R, R¹, R², R³, Y, and Q¹ are as previously defined.

A further particular group of compounds of Formula I is represented by Formula III 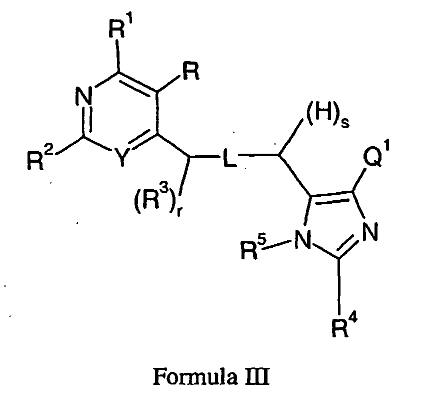 in which of R, R¹, R², R³, R⁴, R⁵, r, s, L, Y, and Q¹ are as previously defined.

Particular novel compounds of the invention include, for example, compounds of the Formula I, II or III, or pharmaceutically-acceptable salts thereof, wherein, unless otherwise stated, each of R, R¹, R², R³, R⁴, R⁵, R⁶, L, Y, G, and Q¹ has any of the meanings defined hereinbefore or in paragraphs (a) to (s):
(a) R is selected from hydrogen, halogeno, carboxy, (1-6C)alkoxycarbonyl and *N-*(heterocyclyl(3-8C)cycloalkyl)carbamoyl;
(b) R is selected from hydrogen, bromo, carboxy, ethoxycarbonyl, *N*-[4-(4-methylpiperazin-1-yl)cyclohexyl]carbamoyl,
(c) R¹ is selected from hydrogen, amino and (1-6C)alkyl;
(d) R¹ is selected from hydrogen, amino and methyl;
(e) R² is selected from hydrogen, halogeno, hydroxy, amino, (1-6C)alkylthio, (1-6C)alkylamino, di-[(1-6C)alkyl]amino, aryl(1-6C)alkylamino, arylamino, heterocyclyl, (2-6C)alkanoylamino;
(f) R² is selected from hydrogen, chloro, hydroxy, amino, methylthio, methylamino, benzylamino, phenethylamino, anilino, morpholino, acetylamino, and 2,2,dimethylpropanoylamino;
(g) R³ is selected from hydrogen, carboxy, (1-6C)alkoxycarbonyl, hydroxy(1-6C)alkyl, N-(1-6C)alkylcarbamoyl and N-(heterocyclyl(3-8C)cycloalkyl)carbamoyl;
(g) R³ is selected from hydrogen, carboxy, methoxycarbonyl, hydroxymethyl, *N-*methylcarbamoyl and *N*-[4-(4-methylpiperazin-1-yl)cyclohexyl]carbamoyl;
(h) R⁴ is hydrogen;
(i) R⁵ is selected from hydrogen, (1-6C)alkyl, aryl(1-6C)alkyl, carboxy(1-6C)alkyl, heterocyclyl(1-6C)alkyl and amino(1-6C)alkyl wherein the amino group is optionally substituted by one or more (1-6C)alkyl;
(j) R⁵ is selected from hydrogen, methyl, benzyl, carboxymethyl and 2-pyrrolidinoethyl;
(k) R⁴ and R⁵ together represent S-CH=CH, S-CH₂-CH₂, or CH=CH-CH=CH wherein the S is attached to the carbon which bears R⁴ in the ring;
(l) R⁶ is selected from hydrogen and (1-6C)alkyl;
(m) G is NR⁵;
(n) Y is N; and
(o) Q¹ is selected from phenyl optionally substituted by halogeno or trifluoromethyl.

A further particular group of compounds of Formula I is represented by Formula III wherein R R¹, R², R³, R⁴, R⁵, r and s are as previously defined and .
R⁶ is hydrogen,
R⁷ is selected from hydrogen, (1-6C)alkyl, aryl(1-6C)alkyl, carboxy(1-6C)alkyl and hetercyclyl(1-6C)alkyl, and
Q is selected from phenyl optionally substituted by halogeno or trifluoromethyl.

Specific compounds of the present invention comprise one or more of the following:
4-[(*E*)-2-(6-phenylimidazo[2,1-*b*][1,3]thiazol-5-yl)vinyl]pyrimidin-2-amine
2,2-dimethyl-*N*-{4-[(*E*)-2-(6-phenylimidazo[2,1-*b*][1,3]thiazol-5-yl)vinyl]pyrimidin-2-yl}propanamide
*N*-{4-[(*E*)-2-(6-phenylimidazo[2,1-*b*][1,3]thiazol-5-yl)vinyl]pyrimidin-2-yl}acetamide
5-[(*E*)-2-(2-chloropyrimidin-4-yl)vinyl]-6-phenylimidazo[2,1-*b*][1,3]thiazole
5-[(*E*)-2-(2-morpholin-4-ylpyrimidin-4-yl)vinyl]-6-phenylimidazo[2,1-*b*][1,3]thiazole
4-{(*E*)-2-[6-(4-fluorophenyl)imidazo[2,1-b][1,3]thiazol-5-yl]vinyl}pyrimidin-2-ol
4-{(*E*)-2-[6-(4-fluorophenyl)imidazo[2,1-b][1,3]thiazol-5-yl]vinyl}pyrimidin-2-amine
4-{(*E*)-2-[6-(4-chlorophenyl)imidazo[2,1-b][1,3]thiazol-5-yl]vinyl}pyrimidin-2-ol
4-{(*E*)-2-[6-(4-chlorophenyl)imidazo[2,1-b][1,3]thiazol-5-yl]vinyl}pyrimidin-2-amine
4-{(*E*)-2-[6-(4-bromophenyl)imidazo[2,1-b][1,3]thiazol-5-yl]vinyl}pyrimidin-2-ol
4-{(*E*)-2-[6-(4-bromophenyl)imidazo[2,1-b][1,3]thiazol-5-yl]vinyl}pyrimidin-2-amine
4-((*E*)-2-{6-[3-(trifluoromethyl)phenyl]imidazo[2,1-*b*][1,3]thiazol-5-yl}vinyl)pyrimidin-2-amine
5-bromo-4-[(*E*)-2-(6-phenylimidazo[2,1-*b*][1,3]thiazol-5-yl)vinyl]pyrimidin-2-amine
*N*-{4-[(*E*)-2-(6-phenyl-2,3-dihydroimidazo[2,1-*b*][1,3]thiazol-5-yl)vinyl]pyrimidin-2-yl}acetamide
4-[(*E*)-2-(6-phenyl-2,3-dihydroimidazo[2,1-*b*][1,3]thiazol-5-yl)vinyl]pyrimidin-2-amine
4-[(*E*)-2-(2-phenylimidazo[1,2-a]pyridin-3-yl)vinyl]pyrimidin-2-amine
4-[(*E*)-2-(4-phenyl-1*H-*imidazol-5-yl)vinyl]pyrimidin-2-amine
Ethyl 2-amino-4-[(*E*)-2-(6-phenylimidazo[2,1-*b*][1,3]thiazol-5-yl)vinyl]pyrimidine-5-carboxylate
2-amino-4-[(*E*)-2-(6-phenylimidazol[2,1-*b*][1,3]thiazol-5-yl)vinyl]pyrimidine-5-carboxylic acid
2-amino-*N-*[4-(4-methylpiperazin-1-yl)cyclohexyl]-4-[(*E*)-2-(6-phenylimidazo[2,1-*b*][1,3]thiazol-5-yl)vinyl]pyrimidine-5-carboxamide
4-[(*E*)-2-(1-methyl-4-phenyl-1*H*-imidazol-5-yl)vinyl]pyrimidin-2-amine
4-[(*E*)-2-(1-benzyl-4-phenyl-1*H*-imidazol-5-yl)vinyl]pyrimidin-2-amine
{5-[(*E*)-2-(2-aminopyrimidin-4-yl)vinyl]-4-phenyl-1*H*-imidazol-1-yl}acetic acid
4-{(*E*)-2-[4-phenyl-1-(2-pyrrolidin-1-ylethyl)-1*H*-imidazol-5-yl]vinyl}pyrimidin-2-amine
*N-*benzyl-4-[(*E*)-2-(6-phenylimidazo[2,1-*b*][1,3]thiazol-5-yl)vinyl]pyrimidin-2-amine
*N*-methyl-4-[(*E*)-2-(6-phenylimidazo[2,1-*b*][1,3]thiazol-5-yl)vinyl]pyrimidin-2-amine
*N*-(1-phenylethyl)-4-[(*E*)-2-(6-phenylimidazo[2,1-*b*][1,3]thiazol-5-yl)vinyl]pyrimidin-2 amine
*N-*phenyl-4-[(*E*)-2-(6-phenylimidazo[2,1-*b*][1,3]thiazol-5-yl)vinyl]pyrimidin-2-amine
4-[(*E)*-2-(6-phenylimidazo[2,1-*b*][1,3]thiazol-5-yl)vinyl]pyrimidin-2-ol
4-[(*E*)-2-(6-phenylimidazo[2,1-*b*][1,3]thiazol-5-yl)vinyl]pyrimidine
4-methyl-6-[(*E*)-2-(6-phenylimidazo[2,1-*b*][1,3]thiazol-5-yl)vinyl]pyrimidin-2-amine
6-[(*E*)-2-(6-phenylimidazo[2,1-*b*][1,3]thiazol-5-yl)vinyl]pyrimidin-4-amine
methyl (2Z)-2-(2-aminopyrimidin-4-yl)-3-(6-phenylimidazo[2,1-*b*][1,3]thiazol-5-yl)prop-2-enoate
(2*Z*)-2-(2-aminopyrimidin-4-yl)-3-(6phenylimidazo[2,1-*b*][1,3]thiazol-5-yl)prop-2-enoic acid
(2*Z*)-2-(2-aminopyrimidin-4-yl)-*N*-[4-(4-methylpiperazin-1-yl)cyclohexyl]-3-(6-phenylimidazo[2,1-*b*][1,3]thiazol-5-yl)prop-2-enamide
2-(2-aminopyrimidin-4-yl)-*N*-methyl-3-(6-phenylimidazo[2,1-*b*][1,3]thiazol-5-yl)acrylamide
2-(2-aminopyrimidin-4-yl)-3-(6-phenylimidazo[2,1-*b*][1,3]thiazol-5-yl)prop-2-en-1-ol
4-[(*E*)-2-(4-phenyl-1*H*-imidazol-5-yl)ethenyl]pyridine
4-[(*E*)-2-(4-phenyl-1*H*-imidazol-5-yl)ethenyl]pyrimidine
4-[(*E*)-2-(1-methyl-4-phenyl-1*H*-imidazol-5-yl)ethenyl]pyrimidine
4-[(*Z*)-2-(1-methyl-4-pheny]-1*H*-imidazol-5-yl)vinyl]pyridine
4-[(*E*)-2-(1-methyl-4-phenyl-1*H*-imidazol-5-yl)ethenyl]pyrimidin-2-ol
*N-*methyl-4-[(*E*)-2-(1-methyl-4-phenyl-1-*H*-imidazol-5-yl)ethenyl]pyrimidin-2-amine
4-[(1-methyl-4-phenyl-1*H*-imidazol-5-yl)ethynyl]-2-(methylthio)pyrimidine
4-(1-Methyl-4-phenyl-1*H*-imidazoyl-5-yl)ethynyl)pyrimidin-2-amine
*N*-methyl-4-[(1-methyl-4-phenyl-1*H*-imidazol-5-yl)ethynyl]pyrimidin-2-amine or
4-[(1-methyl-4-phenyl-1*H-*imidazol-5-yl)ethynyl]pyrimidine ;
and pharmaceutically acceptable salts thereof.

A suitable pharmaceutically-acceptable salt of a compound of the Formula I is, for example, an acid-addition salt of a compound of the Formula I, for example an acid-addition salt with an inorganic or organic acid such as hydrochloric, hydrobromic, sulfuric, trifluoroacetic, citric or maleic acid; or, for example, a salt of a compound of the Formula I which is sufficiently acidic, for example an alkali or alkaline earth metal salt such as a calcium or magnesium salt, or an ammonium salt, or a salt with an organic base such as methylamine, dimethylamine, trimethylamine, piperidine, morpholine or tris-(2-hydrokyethyl)amine.

A particular compound according to the present invention is any one of the compounds described in the Examples or a pharmaceutically acceptable salt thereof.

A compound of the Formula I, or a pharmaceutically-acceptable salt thereof, may be prepared by any process known to be applicable to the preparation of chemically-related compounds. Such processes, when used to prepare a compound of the Formula I are provided as a further feature of the invention and are illustrated by the following representative process variants. Necessary starting materials may be obtained by standard procedures of organic chemistry. The preparation of such starting materials is described in conjunction with the following representative process variants and within the accompanying Examples. Alternatively necessary starting materials are obtainable by analogous procedures to those illustrated which are within the ordinary skill of an organic chemist.

According to a further aspect of the present invention provides a process for preparing a compound of Formula I or a pharmaceutically acceptable salt thereof (wherein R¹, R², R³, R⁴, R⁵, R⁶, A,Q, G,Y, r and s are, unless otherwise specified, as defined in formula I) which process comprises:
(a) for the preparation of those compounds of the Formula I in which L is a double bond, and r is 1 reacting a compound of Formula IV 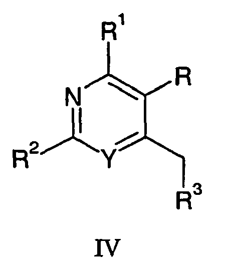 in which R¹, R², R³, R⁴, Y and r are as defined in Formula I with a compound of Formula V 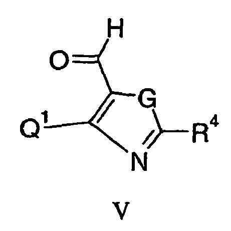 in which A, G, Q, R⁵ and R⁶ are as defined in Formula I in the presence of a base for example, lithium diisopropylamide, n-butyl lithium or sodium ethoxide in the presence of a suitable inert solvent such as an ether, for example, tetrahydofuran or 1,4-dioxan, or an alcohol such as ethanol at a temperature of 0-150°C, preferably at a temperature of 60-110°C,
   or alternatively
(b) reacting a compound of Formula IV with a compound of Formula V in the presence of an acid for example, hydrochloric acid, acetic acid or sulphuric acid, with or without the presence of acetic anhydride or
(c) for the preparation of those compounds of the Formula I in which L is a triple bond and r and s are each 0, reacting a compound of Formula VI 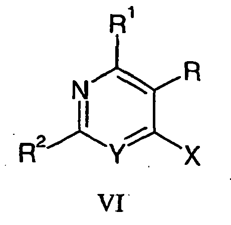 in which R, R¹, R² and Y are as previously defined and X represents a leaving group, for example halogeno, e.g. iodo or bromo, with a compound of Formula VII 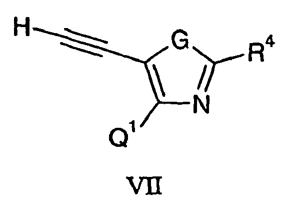 in which G, Q¹ and R⁴ are as previously defined in the presence of a coupling system, for example a copper I / palladium II system for example copper I iodide and dichlorobis(triphenylphosphine)palladium(II), optionally in the presence of a base, for example triethylamine, and in the presence of an inert solvent, for example an ether e.g. THF at a temperature of 0-150°C, preferably at a temperature of 10-60°C.

In the process described in (A) above an intermediate of Formula 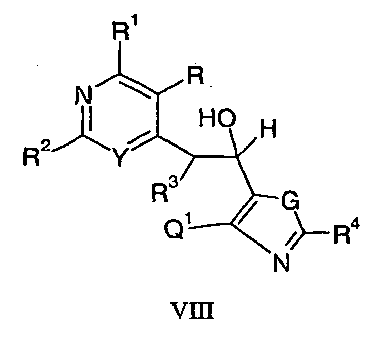 may be isolated and then dehydrated to give a compound of Formula I for example by reacting with strong acid, for example trifluoroacetic acid or hydrochloric acid.

For the preparation of compounds of the formula I in which R¹ is amino, the compound of the formula I may have R¹ as an amino or as an amino protecting group, for example t-butylamido. The protecting group is removed in the dehydration process. This process is conveniently performed at a temperature of 20 to 120°C, preferably at 100°C. Compounds of the formula I where R¹ is hydroxy are also formed in this process.
The compounds of the formula I may be purified using a conventional technique, such as HPLC or recrystallisation

Certain compounds of Formula I may be converted into other compounds of Formula I by means of standard chemical reactions known to those skilled in the art of organic chemistry for example:
(i) esters may be hydrolysed to acids;
(ii) acids may be reacted with amines to give amides;
(iii) activated halogens may be displaced by amines;
(iv) acids may be esterified;
(v) esters may be reduced to alcohols; and
(vi) heterocyclic sulfoxides and sulfones may be displaced with amines.

Certain compounds of Formula I are capable of existing in stereoisomeric forms. It will be understood that the invention encompasses all geometric and optical isomers of the compounds of formula I and mixtures thereof including racemates. Tautomers and mixtures thereof also form an aspect of the present invention.

Isomers may be resolved or separated by conventional techniques, e.g. chromatography or fractional crystallisation. Enantiomers may be isolated by separation of a racemic or other mixture of the compounds using conventional techniques (e.g. chiral High Performance Liquid Chromatography (HPLC)). Alternatively the desired optical isomers may be made by reaction of the appropriate optically active starting materials under conditions which will not cause racemisation, or by derivatisation, for example with a homochiral acid followed by separation of the diastereomeric derivatives by conventional means (e.g. HPLC, chromatography over silica) or may be made with achiral starting materials and chiral reagents. All stereoisomers are included within the scope of the invention.

The compounds of the invention may be isolated from their reaction mixtures using conventional techniques.

It will be appreciated that in some of the reactions mentioned herein it may be necessary/desirable to protect any sensitive groups in the compounds. The instances where protection is necessary or desirable and suitable methods for protection are known to those skilled in the art. Conventional protecting groups may be used in accordance with standard practice (for illustration see T.W. Green, Protective Groups in Organic Synthesis, John Wiley and Sons, 1991). Thus, if reactants include groups such as amino, carboxy or hydroxy it may be desirable to protect the group in some of the reactions mentioned herein. Protecting groups may be removed by any convenient method as described in the literature or known to the skilled chemist as appropriate for the removal of the protecting group in question, such methods being chosen so as to effect removal of the protecting group with minimum disturbance of groups elsewhere in the molecule.

Specific examples of protecting groups are given below for the sake of convenience, in which "lower", as in, for example, lower alkyl, signifies that the group to which it is applied preferably has 1-4 carbon atoms. It will be understood that these examples are not exhaustive. Where specific examples of methods for the removal of protecting groups are given below these are similarly not exhaustive. The use of protecting groups and methods of deprotection not specifically mentioned are, of course, within the scope of the invention.

A carboxy protecting group may be the residue of an ester-forming aliphatic or arylaliphatic alcohol or of an ester-forming silanol (the said alcohol or silanol preferably containing 1-20 carbon atoms). Examples of carboxy protecting groups include straight or branched chain (1-12C)alkyl groups (for example isopropyl, and tert-butyl); lower alkoxylower alkyl groups (for example methoxymethyl, ethoxymethyl and isobutoxymethyl); lower acyloxy-lower alkyl groups, (for example acetoxymethyl, propionyloxymethyl, butyryloxymethyl and pivaloyloxymethyl); lower alkoxycarbonyloxy-lower alkyl groups (for example -methoxycarbonyloxyethyl and 1-ethoxycarbonyloxyethyl); aryl-lower alkyl groups (for example benzyl, 4-methoxybenzyl, 2-nitrobenzyl, 4-nitrobenzyl, benzhydryl and phthalidyl); tri(lower alkyl)silyl groups (for example trimethylsilyl and tert-butyldimethylsilyl); tri(lower alkyl)silyl-lower alkyl groups (for example trimethylsilylethyl); and (2-6C)alkenyl groups (for example allyl). Methods particularly appropriate for the removal of carboxyl protecting groups include for example acid-, base-, metal- or enzymically-catalysed cleavage.

Examples of hydroxy protecting groups include lower alkyl groups (for example tert-butyl), lower alkenyl groups (for example allyl); lower alkanoyl groups (for example acetyl); lower alkoxycarbonyl groups (for example tert-butoxycarbonyl); lower alkenyloxycarbonyl groups (for example allyloxycarbonyl); aryl-lower alkoxycarbonyl groups (for example benzyloxycarbonyl, 4-methoxybenzyloxycarbonyl, 2-nitrobenzyloxycarbonyl and 4-nitrobenzyloxycarbonyl); tri(lower alkyl)silyl (for example trimethylsilyl and tert-butyldimethylsilyl) and aryl-lower alkyl (for example benzyl) groups.

Examples of amino protecting groups include formyl, aryl-lower alkyl groups (for example benzyl and substituted benzyl, 4-methoxybenzyl, 2-nitrobenzyl and 2,4-dimethoxybenzyl, and triphenylmethyl); di-4-anisylmethyl and furylmethyl groups; lower alkoxycarbonyl (for example tert-butoxycarbonyl); lower alkenyloxycarbonyl (for example allyloxycarbonyl); aryl-lower alkoxycarbonyl groups (for example benzyloxycarbonyl, 4-methoxybenzyloxycarbonyl, 2-nitrobenzyloxycarbonyl and 4-nitrobenzyloxycarbonyl); trialkylsilyl (for example trimethylsilyl and tert-butyldimethylsilyl); alkylidene (for example methylidene) and benzylidene and substituted benzylidene groups.

Methods appropriate for removal of hydroxy and amino protecting groups include, for example, acid-, base-, metal- or enzymically-catalysed hydrolysis for groups such as 2-nitrobenzyloxycarbonyl, hydrogenation for groups such as benzyl and photolytically for groups such as 2-nitrobenzyloxycarbonyl.

It will also be appreciated that certain of the various ring substituents in the compounds of the present invention may be introduced by standard aromatic substitution reactions or generated by conventional functional group modifications either prior to or immediately following the processes mentioned above, and as such are included in the process aspect of the invention. Such reactions and modifications include, for example, introduction of a substituent by means of an aromatic substitution reaction, reduction of substituents, alkylation of substituents and oxidation of substituents. The reagents and reaction conditions for such procedures are well known in the chemical art. Particular examples of aromatic substitution reactions include the introduction of a nitro group using concentrated nitric acid, the introduction of an acyl group using, for example, an acyl halide and Lewis acid (such as aluminium trichloride) under Friedel Crafts conditions; the introduction of an alkyl group using an alkyl halide and Lewis acid (such as aluminium trichloride) under Friedel Crafts conditions; and the introduction of a halogeno group. Particular examples of modifications include the reduction of a nitro group to an amino group by for example, catalytic hydrogenation with a nickel catalyst or treatment with iron in the presence of hydrochloric acid with heating; oxidation of alkylthio to alkylsulfinyl or alkylsulfonyl.

### Biological Assays

The following assays can be used to measure the effects of the compounds of the present invention as Tie2 inhibitors in vitro and as inhibitors of Tie2 autophosphorylation in whole cells.

### a. In vitro receptor tyrosine kinase inhibition assay

To test for inhibition of Tie2 receptor tyrosine kinase, compounds are evaluated in a non-cell based protein kinase assay by their ability to inhibit the protein kinase enzyme phosphorylation of a tyrosine containing polypeptide substrate in an ELISA based microtitre plate assay. In this particular case, the assay was to determine the IC₅₀, for three different recombinant human tyrosine kinases Tie2, KDR and Flt.

To facilitate production of the tyrosine kinases, recombinant receptor genes were produced using standard molecular biology cloning and mutagenesis techniques. These recombinant proteins fragments encoded within these genes consist of only the intracellular portion C-terminal portion of the respective receptor, within which is found the kinase domain. The recombinant genes encoding the kinase domain containing fragments were cloned and expressed in standard baculovirus/Sf21 system (or alternative equivalent).

Lysates were prepared from the host insect cells following protein expression by treatment with ice-cold lysis buffer (20mM N-2-hydroxyethylpiperizine-N'-2-ethanesulphonic acid (HEPES) pH7.5, 150mM NaCl, 10% glycerol, 1% Triton X-100, 1.5mM MgCl₂, 1mM ethylene glycol-bis (β-aminoethyl ether) N',N',N',N'- tetraacetic acid (EGTA), plus protease inhibitors and then cleared by centrifugation. Tie2, KDR and Flt1 lysates were stored in aliquots at -80°C.

Constitutive kinase activity of these recombinant proteins was determined by their ability to phosphorylate a synthetic peptide (made up of a random co-polymer of Glutamic Acid, Alanine and Tyrosine in the ratio of 6:3:1). Specifically, Nunc Maxisorb^{™} 96-well immunoplates were coated with 100 microlitres of synthetic peptide Sigma P3899 (1mg/ml stock solution in PBS diluted 1:500 in PBS prior to plate coating) and incubated at 4°C overnight. Plates were washed in 50mM HEPES pH 7.4 at room temperature to remove any excess unbound synthetic peptide.

Tie2, KDR or Flt1 activities were assessed by incubation of the appropriate freshly diluted lysates (1:200, 1:400 and 1:1000 respectively) in peptide coated plates for 60 minutes (Tie2) or 20 minutes for (KDR, Flt) at room temperature in 100mM HEPES pH 7.4 , adenosine trisphosphate (ATP) at 5 micromolar (or Km concentration for the respective enzyme, 10mM MnCl₂, 0.1mM Na₃VO_{4,} 0.2mM DL-dithiothreitol (DTT), 0.1% Triton X-100 together with the test compound(s) in dissolved in DMSO (final concentration of 2.5%) with final compound concentrations ranging from 0.05 micromolar -100 micromolar. Reactions were terminated by the removal of the liquid components of the assay followed by washing of the plates with PBS-T (phosphate buffered saline with 0.5% Tween 20) or an alternative equivalent wash buffer.

The immobilised phospho-peptide product of the reaction was detected by immunological methods. Firstly, plates were incubated for 4 hrs at room temperature with murine monoclonal anti-phosphotyrosin -HRP (Horseradish Peroxidase) conjugated antibodies (4G10 from Upstate Biotechnology UBI 16-105). Following extensive washing with PBS-T, HRP activity in each well of the plate was measured colorimetrically using 22'-Azino-di-[3-ethylbenzthiazoline sulfonate (6)] diammonium salt crystals ABTS (Sigma P4922 - prepared as per manufactures instructions) as a substrate incubated for 30-45 minutes to allow colour development, before 100ul of 1M H2SO4 was added to stop the reaction.

Quantification of colour development and thus enzyme activity was achieved by the measurement of absorbance at 405nm on a Molecular Devices ThermoMax microplate reader. Kinase inhibition for a given compound was expressed as an IC₅₀ value. This was determined by calculation of the concentration of compound that was required to give 50% inhibition of phosphorylation in this assay. The range of phosphorylation was calculated from the positive (vehicle plus ATP) and negative (vehicle minus ATP) control values.

### b. Cellular Tie2 autophosphorylation assay

This assay is based on measuring the ability of compounds to inhibit autophosphorylation of the Tie2 receptor which normally leads to the production of "activated" receptor that in turn initiates the particular signal transduction pathways associated with the receptor function.

Autophosphorylation can be achieved by a number of means. It is known that expression of recombinant kinase domains in baculoviral systems can lead to the production of phosphorylated and activated receptor. It is also reported that over expression of receptors in recombinant cell lines can itself lead to receptor autophosphorylation in the absence of the ligand (Heldin C-H. 1995 Cell : 80, 213-223; Blume-J. P, Hunter T. 2001 Nature: 411, 355-65). Furthermore, there are numerous literature examples in which chimaeric receptors have been constructed. In these cases the natural, external cell surface domain of the receptor has been replaced with that of a domain which is known to be readily dimerised via the addition of the appropriate ligand (e.g. TrkA-Tie2/NGF ligand (Marron, M.B., et al., 2000 Journal of Biological Chemistry : 275:39741-39746) or C-fms-Tie-1/CSF-1 ligand (Kontos, C.D., et al., 2002 Molecular and Cellular Biology : 22, 1704-1713). Thus when the chimaeric receptor expressed in a host cell line and the respective ligand is added, this induces autophosphorylation of the chimeric receptor's kinase domain. This approach has the advantage of often allowing a known (and often easily obtained) ligand to be used instead of having to identify and isolate the natural ligand for each receptor of interest.

Naturally if the ligand is available one can use natural cell lines or primary cells which are known to express the receptor of choice and simply stimulate with ligand to achieve ligand induced phosphorylation. The ability of compounds to inhibit autophosphorylation of the Tie2 receptor, which is expressed for example in EA.hy926/B3 cells (supplied by J. McLean/ B. Tuchi, Univ.of N. Carolina at Chapel Hill, CB- 4100, 300 Bynum Hall, Chapel Hill, N.C. 27599-41000, USA) or primary HUVEC (human umbilical vein endothelial cells - available from various commercial sources), can measured by this assay.

Natural Angl ligand can be isolated using standard purification technology from either tumour cell supernatants or alternatively the Ang1 gene can be cloned and expressed recombinantly using stand molecular biology techniques and expression systems. In this case one can either attempt to produce the ligand either in its native state or as recombinant protein which for example may have been genetically engineered to contain additional of purification tags (eg. polyhistidine peptides, antibody Fc domains) to facilitate the process.

Using the ligand stimulation of either EA.hy926/B3 or HUVEC cellular Tie2 receptor as the example, a Ang1 ligand stimulated cellular receptor phosphorylation assay can be constructed which can be used to analyse to determine the potential of compounds to inhibit this process. For example EA.hy926/B3 cells were grown in the appropriate tissue culture media plus 10% foetal calf serum (FCS) for two days in 6 well plates starting with an initial seeding density of 5x10⁵ cells/well. On the third day the cells were serum starved for a total of 2 hours by replacing the previous media with media containing only 1% FCS. After lhr.40 of serum starvation the media was removed and replace with 1 ml of the test compound dilutions (compound dilutions made in serum starvation media yet keeping the DMSO concentration below 0.8%). After 1hr 5 of serum starvation orthovanidate was added to a final concentration of 0.1 mM for the final 10 mins of serum starvation.

Following a total of 2 hrs of serum starvation, the ligand plus orthovandiate was added to stimulate autophosphorylation of the cellular Tie2 receptor (ligand can be added either as purified material diluted in serum starvation media or non-purified cell supernatant containing ligand e.g. when recombinantly expressed mammalian cells).
After 10 minutes incubation at 37°C with the ligand, the cells were cooled on ice washed with approximately 5mls with cold PBS containing 1 mM orthovanadate, after which 1 ml of ice cold lysis buffer ((20 mM Tris pH 7.6, 150 mM NaCl, 50 mM NaF, 0.1 % SDS, 1% NP40, 0.5 % DOC, 1 mM orthovanadate, 1mM EDTA, 1 mM PMSF, 30 µl / ml Aprotinin, 10 µg/ml Pepstatin, 10 µg/ml Leupeptin) was added the cells and left on ice for 10- 20 min. The lysate was removed and transferred to a 1.5 ml Eppendorf tube and centrifuged for 3 min at 13000 rpm at 4°C. 800 µl of each lysate was transferred to fresh 2 ml Eppendorf tubes for the immuno-precipitation. 3 mg = 15 µl of anti-phospho-tyrosine antibody (Santa Cruz PY99 - sc-7020) was added to the lysates and left to incubate for 2 hours at 4°C. 600 µl washed MagnaBind beads (goat anti-mouse IgG, Pierce 21354) were added to the lysates and the tubes left to rotate over night at 4°C.

Samples were treated for 1 min in the magnet before carefully removing the lysis supernatant. 1 ml of lysis buffer was then added to the beads and this step repeated twice more. The beads were suspended in 25 µl of 94°C hot 2x Laemmli loading buffer plus betamercaptoethanol and left to stand for 15 min at room temperature.

The beads were removed by exposing the tubes for 1 min in the magnet, and the total liquid separated from the beads from each immuno-precipitate loaded onto Polyacrylamide/SDS protein gels (pre-cast 4-12 % BisTris NuPAGE / MOPS 12 well gels from Novex). Protein gels were run at 200 V and then blotted onto NC membrane for 1 h 30 min at 50 V / 250 mA. All blots were treated with 5% Marvel in PBS-Tween for 1 hour at room temperature to reduce non-specific binding of the detection antibody. A rabbit anti-Tie2 (Santa Cruz sc-324) was added in a 1:500 dilution in 0.5 % Marvel / PBS-Tween and left to incubate overnight at 4°C. The blots were rigorously washed with PBS-Tween before adding the goat anti rabbit -POD conjugate (Dako P0448) at a 1:5000 dilution in 0.5 % Marvel / PBS-Tween. The antibody was left on for 1 hour at room temperature before subsequently washing the blots with PBS-Tween. The western blots of the various immuno-precipitated samples were developed the blots with LumiGLO (NEB 7003). And transferred to an X-Ray cassette and films exposed for 15 sec / 30 sec and 60 sec. The relative strength of the protein band which pertains to the phosphorylated Tie2 receptor was evaluated using a FluorS BioRad image analyser system. The percentage phosphorylation for each test compound dilution series was determined from which IC₅₀ values were calculated by standard methods using the appropriate control samples as reference.

Although the pharmacological properties of the compounds of the Formula I vary with structural change as expected, in general activity possessed by compounds of the Formula I, may be demonstrated at the following concentrations or doses in one or more of the above tests (a) and (b):-

| | |
|---|---|
| Test (a):- | IC₅₀ in the range, for example, < 500µM; |
| Test (b):- | IC₅₀ in the range, for example, < 50µM; |

According to a further aspect of the invention there is provided a pharmaceutical composition which comprises a compound of the Formula I, or a pharmaceutically acceptable salt thereof, as defined hereinbefore in association with a pharmaceutically-acceptable diluent or carrier.

The compositions of the invention may be in a form suitable for oral use (for example as tablets, lozenges, hard or soft capsules, aqueous or oily suspensions, emulsions, dispersible powders or granules, syrups or elixirs), for topical use (for example as creams, ointments, gels, or aqueous or oily solutions or suspensions), for administration by inhalation (for example as a finely divided powder or a liquid aerosol), for administration by insufflation (for example as a finely divided powder) or for parenteral administration (for example as a sterile aqueous or oily solution for intravenous, subcutaneous, intramuscular or intramuscular dosing or as a suppository for rectal dosing).

The compositions of the invention may be obtained by conventional procedures using conventional pharmaceutical excipients, well known in the art. Thus, compositions intended for oral use may contain, for example, one or more colouring, sweetening, flavouring and/or preservative agents.

The amount of active ingredient that is combined with one or more excipients to produce a single dosage form will necessarily vary depending upon the host treated and the particular route of administration. For example, a formulation intended for oral administration to humans will generally contain, for example, from 0.5 mg to 0.5 g of active agent (more suitably from 0.5 to 100 mg, for example from 1 to 30 mg) compounded with an appropriate and convenient amount of excipients which may vary from about 5 to about 98 percent by weight of the total composition.

The size of the dose for therapeutic or prophylactic purposes of a compound of the Formula I will naturally vary according to the nature and severity of the conditions, the age and sex of the animal or patient and the route of administration, according to well known principles of medicine.

In using a compound of the Formula I for therapeutic or prophylactic purposes it will generally be administered so that a daily dose in the range, for example, 0.1 mg/kg to 75 mg/kg body weight is received, given if required in divided doses. In general lower doses will be administered when a parenteral route is employed. Thus, for example, for intravenous administration, a dose in the range, for example, 0.1 mg/kg to 30 mg/kg body weight will generally be used. Similarly, for administration by inhalation, a dose in the range, for example, 0.05 mg/kg to 25 mg/kg body weight will be used. Oral administration is however preferred, particularly in tablet form. Typically, unit dosage forms will contain about 0.5 mg to 0.5 g of a compound of this invention.

The compounds according to the present invention as defined herein are of interest for, amongst other things, their antiangiogenic effect. The compounds of the invention are expected to be useful in the treatment or prophylaxis of a wide range of disease states associated with undesirable or pathological angiogenesis, including cancer, diabetes, psoriasis, rheumatoid arthritis, Kaposi's sarcoma, haemangioma, lymphoedema, acute and chronic nephropathies, atheroma, arterial restenosis, autoimmune diseases, acute inflammation, excessive scar formation and adhesions, endometriosis, dysfunctional uterine bleeding and ocular diseases with retinal vessel proliferation. Cancer may affect any tissue and includes leukaemia, multiple myeloma and lymphoma. In particular such compounds of the invention are expected to slow advantageously the growth of primary and recurrent solid tumours of, for example, the colon, breast, prostate, lungs and skin.

We believe that the antiangiogenic properties of the compounds according to the present invention arise from their Tie2 receptor tyrosine kinase inhibitory properties. Accordingly, the compounds of the present invention are expected be useful to produce a Tie2 inhibitory effect in a warm-blooded animal in need of such treatment. Thus the compounds of the present invention may be used to produce an antiangiogenic effect mediated alone or in part by the inhibition of Tie2 receptor tyrosine kinase.

More particularly the compounds of the invention are expected to inhibit any form of cancer associated with Tie2. For example, the growth of those primary and recurrent solid tumours which are associated with Tie2, especially those tumours which are significantly dependent on Tie2 receptor tyrosine kinase for their growth and spread.

According to a further aspect of the invention there is provided a compound of the Formula I, or a pharmaceutically-acceptable salt thereof, as defined hereinbefore, for use as a medicament.

According to another aspect of the invention, there is provided the use of a compound of the formula I, or a pharmaceutically acceptable salt thereof, as defined hereinbefore, in the manufacture of a medicament for use as a Tie2 receptor tyrosine kinase inhibitor in a warm-blooded animal such as man.

According to another aspect of the invention, there is provided the use of a compound of the formula I, or a pharmaceutically acceptable salt thereof, as defined hereinbefore, in the manufacture of a medicament for use in the production of an anti-angiogenic effect in a warm-blooded animal such as man.

According to another aspect of the invention, there is provided the use of a compound of the formula I, or a pharmaceutically acceptable salt thereof, as defined hereinbefore in the manufacture of a medicament for use in the treatment of cancers in a warm-blooded animal such as man.

According to another aspect of the invention, there is provided the use of a compound of the formula I, or a pharmaceutically acceptable salt thereof, as defined hereinbefore in the manufacture of a medicament for use in the treatment of a cancer selected from leukaemia, breast, lung, colon, rectal, stomach, prostate, bladder, pancreas, ovarian, lymphoma, testicular, neuroblastoma, hepatic, bile duct, renal cell, uterine, thyroid and skin cancer in a warm-blooded animal such as man.

According to another aspect of the invention there is provided a method of inhibiting Tie2 receptor tyrosine kinase in a warm-blooded animal, such as man, in need of such treatment, which comprises administering to said animal an effective amount of a compound of the formula I, or a pharmaceutically acceptable salt thereof, as defined hereinbefore.

According to another aspect of the invention there is provided a method for producing an anti-angiogenic effect in a warm-blooded animal, such as man, in need of such treatment, which comprises administering to said animal an effective amount of a compound of the formula I, or a pharmaceutically acceptable salt thereof, as defined hereinbefore.

According to another aspect of the invention there is provided a method of treating cancers in a warm-blooded animal, such as man, in need of such treatment, which comprises administering to said animal an effective amount of a compound of the formula I, or a pharmaceutically acceptable salt thereof, as defined hereinbefore.

According to another aspect of the invention there is provided a method of treating a cancer selected from leukaemia, breast, lung, colon, rectal, stomach, prostate, bladder, pancreas, ovarian, lymphoma, testicular, neuroblastoma, hepatic, bile duct, renal cell, uterine, thyroid or skin cancer, in a warm-blooded animal, such as man, in need of such treatment, which comprises administering to said animal an effective amount of a compound of the formula I, or a pharmaceutically acceptable salt thereof, as defined hereinbefore.

According to another aspect of the invention there is provided a compound of the formula I, or a pharmaceutically acceptable salt thereof, as defined hereinbefore, for use in inhibiting Tie2 receptor tyrosine kinase in a warm-blooded animal, such as man.

According to an another aspect of the invention there is provided a compound of the formula I, or a pharmaceutically acceptable salt thereof, as defined hereinbefore, for use in producing an anti-angiogenic effect in a warm-blooded animal, such as man.

According to another aspect of the invention there is provided a compound of the formula I, or a pharmaceutically acceptable salt thereof, as defined hereinbefore, for use in the treatment of cancer.

According to another aspect of the invention there is provided a compound of the formula I, or a pharmaceutically acceptable salt thereof, as defined hereinbefore, for use in the treatment of a cancer selected from leukaemia, breast, lung, colon, rectal, stomach, prostate, bladder, pancreas, ovarian, lymphoma, testicular, neuroblastoma, hepatic, bile duct, renal cell, uterine, thyroid or skin cancer.

As hereinbefore mentioned it is further expected that a compound of the present invention will possess activity against other diseases mediated by undesirable or pathological angiogenesis including psoriasis, rheumatoid arthritis, Kaposi's sarcoma, haemangioma, lymphoedema, acute and chronic nephropathies, atheroma, arterial restenosis, autoimmune diseases, acute inflammation, excessive scar formation and adhesions, endometriosis, dysfunctional uterine bleeding and ocular diseases with retinal vessel proliferation.

The anti-angiogenic activity defined herein may be applied as a sole therapy or may involve, in addition to a compound of the invention, one or more other substances and/or treatments. Such conjoint treatment may be achieved by way of the simultaneous, sequential or separate administration of the individual components of the treatment. In the field of medical oncology it is normal practice to use a combination of different forms of treatment to treat each patient with cancer. In medical oncology the other component(s) of such conjoint treatment in addition to the cell cycle inhibitory treatment defined hereinbefore may be: surgery, radiotherapy or chemotherapy. Such chemotherapy may include one or more of the following categories of anti-tumour agents:
(i) anti-invasion agents (for example metalloproteinase inhibitors like marimastat and inhibitors of urokinase plasminogen activator receptor function);
(ii) antiproliferative/antineoplastic drugs and combinations thereof, as used in medical oncology, such as alkylating agents (for example cis-platin, carboplatin, cyclophosphamide, nitrogen mustard, melphalan, chlorambucil, busulphan and nitrosoureas); antimetabolites (for example antifolates such as fluoropyrimidines like 5-fluorouracil and tegafur, raltitrexed, methotrexate, cytosine arabinoside and hydroxyurea, or, for example, one of the preferred antimetabolites disclosed in European Patent Application No. 562734 such as (2S)-2-{o-fluoro-p-[N-{2,7-dimethyl-4-oxo-3,4-dihydroquinazolin-6-ylmethyl)-N-(prop-2-ynyl)amino]benzamido}-4-(tetrazol-5-yl)butyric acid); antitumour antibiotics (for example anthracyclines like adriamycin, bleomycin, doxorubicin, daunomycin, epirubicin, idarubicin, mitomycin-C, dactinomycin and mithramycin); antimitotic agents (for example vinca alkaloids like vincristine, vinblastine, vindesine and vinorelbine and taxoids like taxol and taxotere); and topoisomerase inhibitors (for example epipodophyllotoxins like etoposide and teniposide, amsacrine, topotecan and camptothecin);
(iii) cytostatic agents such as antioestrogens (for example tamoxifen, toremifene, raloxifene, droloxifene and iodoxyfene), antiandrogens (for example bicalutamide, flutamide, nilutamide and cyproterone acetate), LHRH antagonists or LHRH agonists (for example goserelin, leuprorelin and buserelin), progestogens (for example megestrol acetate), aromatase inhibitors (for example as anastrozole, letrazole, vorazole and exemestane) and inhibitors of 5 α-reductase such as finasteride;
(iv) inhibitors of growth factor function, for example such inhibitors include growth factor antibodies, growth factor receptor antibodies, farnesyl transferase inhibitors, tyrosine kinase inhibitors and serine/threonine kinase inhibitors, for example inhibitors of the epidermal growth factor family (for example the EGFR tyrosine kinase inhibitors N-(3-chloro-4-fluorophenyl)-7-methoxy-6-(3-morpholinopropoxy)quinazolin-4-amine (ZD1839), N-(3-ethynylphenyl)-6,7-bis(2-methoxyethoxy)quinazolin-4-amine (CP 358774) and 6-acrylamido-N-(3-chloro-4-fluorophenyl)-7-(3-morpholinopropoxy)quinazolin-4-amine (CI 1033)), for example inhibitors of the platelet-derived growth factor family and for example inhibitors of the hepatocyte growth factor family;
(v) antiangiogenic agents that work by different mechanisms to those defined hereinbefore, such as those which inhibit vascular endothelial growth factor such as the compounds disclosed in International Patent Applications WO 97/22596, WO 97/30035, WO 97/32856 and WO 98/13354 and those that work by other mechanisms (for example linomide, inhibitors of integrin αvβ3 function and angiostatin);
(vi) antisense therapies, for example those which are directed to the targets listed above, such as ISIS 2503, an anti-ras antisense;
(vii) gene therapy approaches, including for example approaches to replace aberrant genes such as aberrant p53 or aberrant BRCA1 or BRCA2, GDEPT (gene-directed enzyme pro-drug therapy) approaches such as those using cytosine deaminase, thymidine kinase or a bacterial nitroreductase enzyme and approaches to increase patient tolerance to chemotherapy or radiotherapy such as multi-drug resistance gene therapy; and
(viii) immunotherapy approaches, including for example ex-vivo and in-vivo approaches to increase the immunogenicity of patient tumour cells, such as transfection with cytokines such as interleukin 2, interleukin 4 or granulocyte-macrophage colony stimulating factor, approaches to decrease T-cell anergy, approaches using transfected immune cells such as cytokine-transfected dendritic cells, approaches using cytokine-transfected tumour cell lines and approaches using anti-idiotypic antibodies.

Such conjoint treatment may be achieved by way of the simultaneous, sequential or separate dosing of the individual components of the treatment. Such combination products employ the compounds of this invention within the dosage range described hereinbefore and the other pharmaceutically-active agent within its approved dosage range.

According to this aspect of the invention there is provided a pharmaceutical product comprising a compound of the Formula I as defined hereinbefore and an additional anti-tumour substance as defined hereinbefore for the conjoint treatment of cancer,

In addition to their use in therapeutic medicine, the compounds of Formula I and their pharmaceutically acceptable salts, are also useful as pharmacological tools in the development and standardisation of in vitro and *in vivo* test systems for the evaluation of the effects of inhibitors of cell cycle activity in laboratory animals such as cats, dogs, rabbits, monkeys, rats and mice, as part of the search for new therapeutic agents.

The invention will now be illustrated by the following non limiting examples in which, unless stated otherwise:
(i) temperatures are given in degrees Celsius (°C); operations were carried out at room or ambient temperature, that is, at a temperature in the range of 18-25 °C;
(ii) organic solutions were dried over anhydrous magnesium sulfate; evaporation of solvent was carried out using a rotary evaporator under reduced pressure (600-4000 Pascals; 4.5-30 mmHg) with a bath temperature of up to 60°C;
(iii) chromatography means flash chromatography on silica gel; thin layer chromatoraphy (TLC) was carried out on silica gel plates;
(iv) in general, the course of reactions was followed by TLC and / or analytical LC-MS, and reaction times are given for illustration only;
(v) final products had satisfactory proton nuclear magnetic resonance (NMR) spectra and/or mass spectral data;
(vi) yields are given for illustration only and are not necessarily those which can be obtained by diligent process development; preparations were repeated if more material was required;
(vii) when given, NMR data is in the form of delta values for major diagnostic protons, given in parts per million (ppm) relative to tetramethylsilane (TMS) as an internal standard, determined at 300 MHz using perdeuterio dimethyl sulphoxide (DMSO-d₆) as solvent unless otherwise indicated; the following abbreviations have been used: s, singlet; d, doublet; t, triplet; q, quartet; m, multiplet; b, broad;
(viii) chemical symbols have their usual meanings; SI units and symbols are used;
(ix) solvent ratios are given in volume:volume (v/v) terms; and
(x) mass spectra (MS) were run with an electron energy of 70 electron volts in the chemical ionization (CI) mode using a direct exposure probe; where indicated ionization was effected by electron impact (EI), fast atom bombardment (FAB) or electrospray (ESP); values for m/z are given; generally, only ions which indicate the parent mass are reported; and unless otherwise stated, the mass ion quoted is MH⁺;
(xi) unless stated otherwise compounds containing an asymmetrically substituted carbon and/or sulphur atom have not been resolved;
(xii) where a synthesis is described as being analogous to that described in a previous example the amounts used are the millimolar ratio equivalents to those used in the previous example;
(xvi) the following abbreviations have been used:
   - THF: tetrahydrofuran;
   - DMF: *N,N*-dimethylformamide;
   - NMP: 1-methyl-2-pyrrolidinone;
   - DCM: dichloromethane;
   - DMSO: dimethyl sulfoxide;
   - TFA: trifluoroacetic acid
   - EtOAc: ethyl acetate
   - DIPEA: diisopropylethylamine
   - LDA: lithium diisopropylamide
   - MeOH: methanol
   - RPHPLC: reversed phase high peformance liquid chromatography
   - HATU: O-(7-azabenzotriazolyl-1- yl)-N,N,N',N'-tetramethyluronium hexafluorophosphate
   - Et₃N: triethylamine
   - TEA: triethylamine
   - MeCN: acetonitrile
   - DMA: dimethylamine
xvii) where a synthesis is described as leading to an acid addition salt (e.g. HCI salt), no comment is made on the stoichiometry of this salt. Unless otherwise stated, all NMR data is reported on free-base material, with isolated salts converted to the free-base form prior to characterisation. Where compounds are isolated by reverse phase HPLC using TFA as a component of the mobile phase it is likely that the product obtained will be a trifluoroacetate salt.

### Example 1

### 4-[(E)-2-(6-phenylimidazo[2,1-b][1,3]thiazol-5-yl)vinyl]pyrimidin-2-amine (M368100)

### General method A (Base Method)

LDA (as 2M in heptane/THF/ethylbenzene, 0.55 ml, 1.1 mmol) was added to a solution of 2,2-dimethyl-*N*-(4-methylpyrimidin-2-yl)propanamide (Intermediate 1) (100 mg, 0.52 mmol) in THF (2.5 ml) at room temperature. The resulting solution was stirred for 25 minutes, after which time a solution of 6-phenylimidazo[2,1-*b*][1,3]thiazole-5-carbaldehyde (114 mg, 0.5 mmol) in THF (2.5 ml) was added. After stirring for a further 4 hours the mixture was poured into water and acidified by addition of 2M HCI. The mixture was washed with EtOAc, back extracting with 2M HCI, and the combined aqueous phases basified by addition of 2M NaOH, extracted into EtOAc, washed water, dried and the solvent evaporated to give a yellow solid. Purification by flash chromatography on silica, eluting with 98:2 dichloromethane: methanol gave 2,2-dimethyl-*N*-{4-[(*E*)-2-(6-phenylimidazo[2,1-*b*][1,3]thiazol-5-yl)vinyl]pyrimidin-2-yl}propanamide (Example 2) as a yellow solid (168mg, 80%).
MS m/e MH⁺ 404

This was dissolved in 4M HCI (5 ml) and heated at 100°C for 70 minutes. After cooling, the mixture was poured into 1M NaOH, and extracted into EtOAc. The organic phase was washed with water, dried and the solvent removed to a yellow-brown solid. Trituration with EtOAc, ether and *iso*-hexane gave the title compound as a yellow solid. (56mg, 34%).
¹H NMR (DMSO-d₆): δ 4.96 (bs, 2H), 7.09 (d, 1H), 7.18 (d, 1H), 7.45-7.58 (m, 3H), 7.64-7.68 (m, 3H), 8.12 (d, 1H), 8.30 (d, 1H), 8.63 (d, 1H).
MS m/e MH⁺ 320.

The starting materials were prepared as follows:

### Intermediate 1

### 2,2-dimethyl-N-(4-methylpyrimidin-2-yl)propanamide

Pivaloyl chloride (8.6ml, 69.8mmol) was added dropwise to a suspension of 2-amino,-4-methylpyrimidine (7.53g, 68.7 mol) in dichloromethane (70ml)) and triethylamine (9.8ml, 70.3mmol) with ice bath cooling. The mixture was stirred for 16 hours, filtered, washed with water, saturated NaHCO₃, dried and the solvent evaporated to a pale solid. Recrystallisation from dichloromethane/*iso*-hexane gave the title compound as colourless needles (7.12g). Partial evaporation yielded 1.8 g (67% total) as a second crop.
¹H NMR (DMSO-d₆): δ 1.19 (s, 9H), 2.39 (s, 3H), 7.05 (d, 1H), 8.48 (d, 1H), 9.83 (bs, 1H). MS m/e (M-1) 192.

### General method B (Acid Method)

Conc. sulfuric acid (5 ml) was added to a solution of 6-phenylimidazo[2,1-*b*][1,3]thiazole-5-carbaldehyde (1.0 g, 4.39 mmol) and 2-amino-4-methylpyrimidine (480 mg, 4.4 mmol) in acetic acid. The solution was heated at 70°C for 2 days, cooled, poured carefully into saturated NaHCO₃, extracted into EtOAc, dried and the solvent evaporated. Purification by flash chromatography on silica, eluting with EtOAc:MeOH:Et₃N mixtures yielded the title compound as a yellow solid. (450 mg, 32%).

### General Method C (Acid/Anhydride method)

Acetic anhydride (0.3 ml) and conc. sulfuric acid (5 ml) were added to a solution of 6-phenylimidazo[2,1-*b*][1,3]thiazole-5-carbaldehyde (1342 mg, 1.5 mmol) and 2-amino-4-methylpyrimidine (164 mg, 1.5 mmol) in acetic acid (3.75 ml). The solution was heated at 70°C for 2 days, cooled, poured carefully into saturated NaHCO₃, extracted into EtOAc, dried and the solvent evaporated. Purification by flash chromatography on silica, eluting with EtOAc: MeOH: Et₃N mixtures yielded the title compound as a yellow solid (221mg, 46%) and *N*-{4-[(*E*)-2-(6-phenylimidazo[2,1-*b*][1,3]thiazol-5-yl)vinyl]pyrimidin-2-yl}acetamide (Example 3) as a yellow solid (4mg, 0.7%).
¹H NMR (DMSO-d₆): δ 2.23 (s, 3H), 7.13 -7.19 (m, 2H), 7.42 (t, 1H), 7.48 (t, 2H), 7.56 (d, 1H), 7.68 (d, 2H), 8.15 (d, 1H), 8.53-8.56 (m, 2H), 10.29 (bs, 1H). (Contains 32% cis isomer). MS m/e MH⁺ 362.

### Example 4

### 5-[(E)-2-(2-chloropyrimidin-4-yl)vinyl]-6-phenylimidazo[2,1-b][1,3]thiazole

Lithium hexamethyldisilazane (as 1.0M in THF, 15.75 ml, 15.75 mmol) was added to a solution of 2-chloro-4-methylpyrimidine (1.93 g, 15 mmol) in THF (75 ml) at -70°C. 6-phenylimidazo[2,1-*b*][1,3]thiazole-5-carbaldehyde (3.42 g, 15 mmol) as a suspension in THF (75 ml) was added to the resultant solution. The mixture was allowed to warm to room temperature and trifluoroacetic acid (15 ml) and water (0.75 ml) were added. The mixture was warmed to 50°C for 24 hours, cooled to room temperature and poured into water. Filtration gave the title compound as a yellow solid (2.14 g, 42%).
¹H NMR (CDCl₃):δ 6.75 (d, 1H), 7.06 (d, 1H), 7.16 (d, 1H), 7.41-7.54 (m, 3H), 7.70-7.73 (m, 2H), 7.89 (d, 1H), 8.06 (d, 1H), 8.48 (d, 1H).+
MS m/e MH⁺ 337, 339.

### General method D - (Chloride displacement)

### Example 5.

### 5-[(E)-2-(2-morpholin-4-ylpyrimidin-4-yl)vinyl]-6-phenylimidazo[2,1-b][1,3]thiazole

Morpholine (40 µl, 0.45 mmol) and one drop of HCI in ether were added to a solution of 5-[(*E*)-2-(2-chloropyrimidin-4-yl)vinyl]-6-phenylimidazo[2,1-*b*][1,3]thiazole (Example 4) (68 mg, 0.2 mmol) in DMA (1 ml). The solution was heated for 16 hours, partitioned between 2M HCI and EtOAc. The aqueous was basified by addition of 1M NaOH and extracted into EtOAc, washed with water, saturated brine, dried, solvent evaporated to give the title compound as a yellow solid. (74mg, 95%).
¹H NMR (CDCl₃): δ 3.77-3.84 (m, 8H), 6.51 (d, 1H), 6.66 (d, 1H), 7.00 (d, 1H), 7.39-7.50 (m, 3H), 7.74-7.87 (m, 3H), 8.11 (d, 1H), 8.28 (d, 1H).
MS m/e MH⁺ 391.

### Examples 6 & 7

### 4-{(E)-2-[6-(4-fluorophenyl)imidazo[2,1-b][1,3]thiazol-5-yl]vinyl}pyrimidin-2-ol and 4-{(E)-2-[6-(4-fluorophenyl)imidazo[2,1-b][1,3]thiazol-5-yl]vinyl}pyrimidin-2-amine

Prepared according to General method A from 6-(4-fluorophenyl)imidazo[2,1-*b*][1,3]thiazole-5-carbaldehyde (185mg, 0.75mmol) and 2,2-dimethyl-*N-*(4-methylpyrimidin-2-yl)propanamide (Intermediate 1) (145mg, 0.75mmol) to give a mixture of examples 6 and 7 which were purified by preparative reverse phase HPLC (Acetonitrile:water:TFA, 90:10;0.1) to give 4-{(*E*)-2-[6-(4-fluorophenyl)imidazo[2,1-*b*][1,3]thiazol-5-yl]vinyl}pyrimidin-2-ol (example 6) as a yellow solid. (10.3mg, 4%)
¹H NMR (DMSO-d₆): δ 4.95 (bs, 2H), 6.92 (d, 1H), 7.09 (d, 1H), 7.36 (t, 2H), 7.60 (d, 1H), 7.69 (dd, 2H), 8.00 (d, 1H), 8.24 (d, 1H), 8.54 (d, 1H).
MS m/e MH⁺ 339.
and 4-{(*E*)-2-[6-(4-fluorophenyl)imidazo[2,1-*b*][1,3]thiazol-5-yl]vinyl}pyrimidin-2-amine (example 7) as a yellow solid (7.8mg, 3%)
¹H NMR (DMSO-d₆): δ 4.30 (bs, 2H), 6.90 (d, 1H), 6.93 (d, 1H), 7.37 (t, 2H), 7.67 (d, 1H), 7.74 (dd, 2H), 8.09 (d, 1H), 8.21 (d, 1H), 8.47 (d, 1H).
MS m/e MH⁺ 338.

### Examples 8 & 9

### 4-{(E)-2-[6-(4-chlorophenyl)imidazo[2,1-b][1,3]thiazol-5-yl]vinyl}pyrimidin-2-ol and 4-{[E)-2-[6-(4-chlorophenyl)imidazo[2,1-b][1,3]thiazol-5-yl]vinyl}pyrimidin-2-amine

Prepared according to General method A from 6-(4-chlorophenyl)imidazo[2,1-*b*][1,3]thiazole-5-carbaldehyde (200mg, 0.76mmol) and 2,2-dimethyl-*N-*(4-methylpyrimidin-2-yl)propanamide (Intermediate 1) (145mg, 0.75mmol) to give a mixture of examples 8 and 9. Purification by preparative reverse phase HPLC (Acetonitrile:water:TFA, 90:10;0.1) gave 4-{(*E*)-2-[6-(4-chlorophenyl)imidazo[2,1-*b*][1,3]thiazol-5-yl]vinyl}pyrimidin-2-ol (Example 8) as a yellow solid. (6.7mg, 2.5%)
¹H NMR (DMSO-d₆): δ 3.95 (bs, 2H), 6.91 (d, 1H), 6.95 (d, 1H), 7.59 (d, 2H), 7.66-7.73 (m, 3H), 8.10 (d, 1H), 8.22 (d, 1H), 8.47 (d, 1H).
MS m/e MH⁺ 355, 357.
and 4-{(*E*)-2-[6-(4-chlorophenyl)imidazo[2,1-*b*][1,3]thiazol-5-yl]vinyl}pyrimidin-2-amine (Example 9) as a yellow solid. (18.7mg, 7%).
¹H NMR (DMSO-d₆): δ 5.10 (bs, 4H), 6.95 (d, 1H), 7.11 (d, 1H), 7.57-7.69 (m, 5H), 8.03 (d, 1H), 8.25 (d, 1H), 8.55 (d, 1H).
MS m/e MH⁺ 354, 356.

### Examples 10 & 11

### 4-{(E)-2-[6-(4-bromonhenyl)imidazo[2,1-b][1,3]thiazol-5-yl}]vinyl}pyrimidin-2-ol and 4-{(E)-2-[6-(4-bromophenyl)imidazo[2,1-b][1,3]thiazol-5-yl]vinyl}pyrimidin-2-amine

Prepared according to general method A from 6-(4-bromophenyl)imidazo[2,1-*b*][1,3]thiazole-5-carbaldehyde (230mg, 0.75mmol) and 2,2-dimethyl-*N*-(4-methylpyrimidin-2-yl)propanamide (Intermediate 1) (145mg, 0.75mmol) to give a mixture of examples 10 and 11. Purification on preparative reverse phase HPLC (Acetonitrile:water:TFA, 90:10;0.1) gave 4-{(*E*)-2-[6-(4-bromophenyl)imidazo[2,1-*b*][1,3]thiazol-5-yl]vinyl}pyrimidin-2-ol (Example 10) as a yellow solid. (3.5mg, 1.2%).
¹H NMR (DMSO-d₆): δ 4.30 (bs, 1H), 6.90 (d, 1H), 6.95 (d, 1H), 7.63-7.74 (m, 5H), 8.08 (d, 1H), 8.21 (d, 1H), 8.47 (d, 1H).
MS m/e MH⁺ 399, 401.
and 4-{(*E*)-2-[6-(4-bromophenyl)imidazo[2,1-*b*][1,3]thiazol-5-yl]vinyl}pyrimidin-2-amine (Example 11) as a yellow solid. (10.6mg, 3.6%).
¹H NMR (DMSO-d₆): δ 5.00 (bs, 2H), 6.95 (d, 1H), 7.12 (d, 1H), 7.59-7.62 (m, 3H), 7.72 (d, 2H), 8.03 (d, 1H), 8.25 (d, 1H), 8.55 (d, 1H).
MS m/e MH⁺ 398, 400.

### Example 12

### 4-((E)-2-{6-[3-(trifluoromethyl)phenyl]imidazo[2,1-b][1,3]thiazol-5-yl}vinyl)pyrimidin-2 amine

Prepared according to general method A from 6-[3-(trifluoromethyl)phenyl]imidazo[2,1-*b*][1,3]thiazole-5-carbaldehyde (148mg, 0.5mmol) and 2,2-dimethyl-*N*-(4-methylpyrimidin-2-yl)propanamide (Intermediate 1) (145mg, 0.75mmol). Purification on preparative reverse phase HPLC (Acetonitrile:water:TFA, 90:10;0.1) gave the title compound as a yellow solid. (16mg, 5.4%)
¹H NMR (DMSO-d₆): δ 4.44 (bs, 2H), 6.97 (d, 1H), 7.14 (d, 1H), 7.63 (d, 2H), 7.74-7.82 (m, 2H), 7.93 (d, 1H), 8.03 (d, 1H), 8.27 (d, 1H), 8.55 (d, 1H).
MS m/e MH⁺ 388.

### Example 13

### 5-bromo-4-[(E)-2-(6-phenylimidazo[2,1-b][1,3]thiazol-5-yl)vinyl]pyrimidin-2-amine

Prepared according to general method B from 6-phenylimidazo[2,1-*b*][1,3]thiazole-5-carbaldehyde (228mg, 1mmol) and 5-bromo-4-methylpyrimidin-2-amine (188mg, 1mmol). Purification by flash chromatography on silica eluting, with 1:1 EtOAc: :*iso*-hexane gave the title compound as a yellow solid. (56mg, 14%).
¹H NMR (CDCl₃): δ 4.94 (bs, 2H), 7.05 (d, 1H), 7.10 (d, 1H), 7.42 (t, 1H), 7.50 (t, 2H), 7.75 (d, 2H), 7.89 (d, 1H), 8.21 (d, 1H), 8.30 (s, 1H) (Contains 16% *cis* isomer).
MS m/e MH⁺ 398, 400.

### Examples 14 & 15

### N-{4-[(E)-2-(6-phenyl-2,3-dihydroimidazo[2,1-b][1,3]thiazol-5-yl)vinyl]pyrimidin-2-yl}acetamide and 4-[(E)-2-(6-phenyl-2,3-dihydroimidazo[2,1-b][1,3]thiazol-5-yl)vinyl]pyrimidin-2-amine

Prepared according to general method C from 6-phenyl-2,3-dihydroimidazo[2,1-*b*][1,3]thiazole-5-carbaldehyde (230mg, 1mmol) and 4-methylpyrimidin-2-amine (109mg, 1mmol) to give a mixture of examples 14 and 15 which were purified by flash chromatography on silica, eluting with 98:2 dichloromethane:methanol gave *N-*{4-[(*E*)-2-(6-phenyl-2,3-dihydroimidazo[2,1-*b*][1,3]thiazol-5-yl)vinyl]pyrimidin-2-yl}acetamide (Example 14) as a yellow solid. (25mg, 7%).
¹H NMR (CDCl₃): δ 2.50 (s, 3H), 3.97 (t, 2H), 4.52 (t, 2H), 6.58 (d, 1H), 6.82 (d, 1H), 7.37 (t, 1H), 7.44 (t, 2H), 7.63 (d, 2H), 7.93 (d, 1H), 8.31 (bs, 1H), 8.45 (d, 1H).
MS m/e MH⁺ 364.
and 4-[(*E*)-2-(6-phenyl-2,3-dihydroimidazo[2,1-*b*][1,3]thiazol-5-yl)vinyl]pyrimidin-2-amine (Example 15) as a yellow solid, (123mg, 38%)
¹H NMR (CDCl₃): δ 3.93 (t, 2H), 4.48 (t, 2H), 4.94 (bs, 2H), 6.51 (d, 1H), 6.56 (d, 1H), 7.35 (t, 1H), 7.43 (t, 2H), 7.64 (d, 2H), 7.77 (d, 1H), 8.21 (d, 1H).
MS m/e MH⁺ 322.

### Example 16

### 4-[(E)-2-(2-phenylimidazo[1,2-a]pyridin-3-yl)vinyl]pyrimidin-2-amine

Prepared according to general method C from 2-phenylimidazo[1,2-a]pyridine-3-carbaldehyde (92mg, 0.41mmol) and 4-methylpyrimidin-2-amine (60mg, 0.55mmol). Purification by flash chromatography on silica, eluting with 98:2 dichloromethane:methanol gave the title compound as a yellow solid foam. (67mg, 52%)
¹H NMR (CDCl₃): δ 4.98 (bs, 2H), 6.57 (d, 1H), 6.85 (d, 1H), 6.97 (dt, 1H), 7.26-7.35 (m, 1H), 7.43-7.53 (m, 3H), 7.72 (d, 1H), 7.78-7.84 (dd, 2H), 8.11 (d, 1H), 8.24 (d, 1H), 8.53 (d, 1H).
MS m/e MH⁺ 314.

### Example 17

### 4-[(E)-2-(4-phenyl-1H-imidazol-5-yl)vinyl]pyrimidin-2-amine

Prepared according to general method B from 4-phenyl-1*H*-imidazole-5-carbaldehyde (86mg, 0.5mmol) and 4-methylpyrimidin-2-amine (60mg, 0.55mmol). Purification by flash chromatography on silica, eluting with 98:2 dichloromethane: methanol gave the title compound as a yellow solid. (57mg, 44%).
¹H NMR (DMSO-d₆): δ 4.20 (bs, 2H), 7.01 (d, 1H), 7.53 (d, 1H), 7.55-7.68 (m, 5H), 7.79 (d, 1H), 8.37 (d, 1H), 9.20 (bs, 1H).
MS m/e MH⁺ 264.

### Example 18

### Ethyl 2-amino-4-[(E)-2-(6-phenylimidazo[2,1-b][1,3]thiazol-5-yl)vinyl]pyrimidine-5 carboxylate

Prepared according to general method C from phenylimidazo[2,1-*b*][1,3]thiazole-5-carbaldehyde (228mg, 1mmol) and ethyl 2-amino-4-methylpyrimidine-5-carboxylate (181mg, 1mmol). Purification by flash chromatography on silica, eluting with 98:2 dichloromethane:methanol gave the title compound as a yellow solid. (65mg, 16.6%)
¹H NMR (CDCl₃): δ 1.42 (t, 3H), 4.38 (q, 2H), 5.27 (bs, 2H), 7.04 (d, 1H), 7.41 (t, 1H), 7.50 (t, 2H), 7.77 (d, 2H), 8.11 (d, 1H), 8.16 (d, 1H), 8.34 (d, 1H), 8.92 (s, 1H).
MS m/e MH⁺ 392.

### Example 19

### 2-amino-4-[(E)-2-(6-phenylimidazo[2,1-b][1,3]thiazol-5-yl)vinyl]pyrimidine-5-carboxylic acid

A solution of ethyl 2-amino-4-[(*E*)-2-(6-phenylimidazo[2,1-*b*][1,3]thiazol-5-yl)vinyl]pyrimidine-5-carboxylate (Example 18), (2.37 g, 6.06 mmol) in methanol (100 ml) and 2M NaOH (20 ml) was heated to 60°C for 16 hours. The methanol was removed by evaporation and the aqueous residue acidified by addition of 1M HCI. The resultant solid was collected by filtration, washed with water and dried to give the title compound as a yellow solid. (1.93g, 88%).
¹H NMR (DMSO-d₆): δ 7.30 (bs, 3H), 7.43 (t, 1H), 7.51 (t, 2H), 7.59 (d, 1H), 7.67 (d, 2H), 8.09-8.14 (m, 3H), 8.74 (s,1H).
MS m/e MH⁺ 364.

### Example 20

### 2-amino-N-[4-(4-methylpiperazin-1-yl)cyclohexyl]-4-[(E)-2-(6-phenylimidazo[2,1-b][1,3]thiazol-5-yl)vinyl]pyrimidine-5-carboxamide

HATU (85 mg, 0.22 mmol) and DIPEA (140 µl, 0.8 mmol) were added to a solution of 4-(4-methylpiperazin-1-yl)cyclohexanamine (Intermediate 2) (39 mg, 0.2 mmol) and 2-amino-4-[(*E*)-2-(6-phenylimidazo[2,1-*b*][1,3]thiazol-5-yl)vinyl]pyrinfidine-5-carboxylic acid (Example 19) (73 mg, 0.2 mmol) in DMF (1 ml). The resulting solution was stirred for 4 hours, poured into water and the product collected by filtration to give the title compound as a yellow solid. (30mg, 28%)
¹H NMR (DMSO-d₆): δ 1.18-1.38 (m, 2H), 1.80-1.96 (m, 2H), 2.10-2.40 (m, 4H), 2.50 (s, 3H), 3.20-3.50. (m, 9H), 3.60-3.75 (m, 1H), 6.86 (bs, 2H), 7.36-7.67 (m, 7H), 7.98-8.03 (m, 2H), 8.15 (d, 1H), 8.35 (d, 1H).

### Intermediate 2

### 4-(4-methylpiperazin-1-yl)cyclohexanamine

Sodium trisacetoxyborohydride (5.1g, 24mmole) was added in portions over 30 minutes to a solution of *tert*-butyl (4-oxocyclohexyl)carbamate (5g, 24mmole) and methylpiperazine (2.66ml, 24mmole) in MeCN (500ml). After 16 hours at ambient temperature, the mixture was filtered and the solid was washed with MeCN (3x50ml). The combined filtrate and washes were evaporated to give an oil. Purification by flash chromatography on silica, eluting with 95:5 dichloromethane:methanol gave the product as an oil. (6.28g, 87%). This was deprotected with 4M HCl in dioxan (20ml, 4 equivalents) at ambient temperature for 2 hours. The slurry of white precipitate and solvent was carefully evaporated to remove HCl and the solid was dried, in vacuo, over NaOH pellets (6.04g, 82%).

One half of this salt was dissolved in water (100ml) and passed through a column of ion exchange resin (AG1X2, quaternary ammonium hydroxide, OH form). The column was washed with water and the solution was evaporated to give a colourless foam, which was dried, in vacuo, over phosphorus pentoxide to afford the free base as a colourless solid (1.97g, 100%.).
MS m/e MH⁺ 198.

### Example 21

### 4-[(E)-2-(1-methyl-4-phenyl-1H-imidazol-5-yl)vinyl]pyrimidin-2-amine

Prepared according to general method B by reacting 1-methyl-4-phenyl-1*H*-imidazole-5 carbaldehyde (Intermediate 3) (51.6mg, 0.28mmol) and 4-methylpyrimidin-2-amine (31mg, 0.28mmol). Purification by flash chromatography on silica, eluting with EtOAc gave the title compound as a yellow solid. (20mg, 23%).
¹H NMR (DMSO-d₆): δ 3.82 (s, 3H); 6.48 (bs, 2H), 6.65 (d, 1H), 6.80 (d, 1H), 7.34 (t, 1H), 7.46 (t, 2H), 7.58 (d, 2H), 7.77 (d, 1H), 7.83 (s, 1H), 8.20 (d, 1H).

### General method E (Alkylation)

### Intermediate 3

### 1-methyl-4-phenyl-1H-imidazole-5-carbaldehyde

Cesium carbonate (21.2 g, 65.1 mmol) was added to a suspension of 4-phenyl-1*H*-imidazole-5-carbaldehyde (10.38 g, 60 mmol) in DMF (240 ml) followed after 10 minutes by addition of iodomethane (3.9 ml, 61.8 mmol). The reaction mixture was stirred for 16 hours. Water (400 ml) was added and stirred for 1 hour. Filtration of the resulting precipitate gave the title compound as a yellow solid (5.8g). Extraction of the filtrate and purification by flash chromatography on silica, eluting with 1:1 EtOAc: iso-hexane gave a further 2.24 g (66%).
¹H NMR (DMSO-d₆): δ 3.89 (s, 3H), 7.41-7.46 (m, 3H), 7.72 (d, 2H), 8.02 (s, 1H), 9.82 (s, 1H).
MS m/e MH⁺ 187.

### Example 22

### 4-[(E)-2-(1-benzyl-4-phenyl-1H-imidazol-5-yl)vinyl]pyrimidin-2-amine

Prepared according to general method B from 1-benzyl-4-phenyl-1*H*-imidazole-5-carbaldehyde (Intermediate 4) (262mg, 1mmol) and 4-methylpyrimidin-2-amine (109mg, 1mmol). Purification by preparative HPLC (Acetonitrile:water:TFA, 90:10;0.1) gave the title compound as a yellow solid (40mg, 12%).
¹H NMR (DMSO-d₆): δ 5.58 (bs, 2H), 6.66 (d, 1H), 6.76 (d, 1H), 7.22-7.52 (m, 8H), 7.59 (d, 2H), 7.73 (d, 1H), 8.20 (d, 1H), 8.54 (s, 1H).
MS m/e MH⁺ 354.

### Intermediate 4

### 1-benzyl-4-phenyl-1H-imidazole-5-carbaldehyde

Prepared according to general method E from 4-phenyl-1*H*-imidazole-5-carbaldehyde (380mg, 2.2mmol) and benzyl bromide (0,265ml), 2.23mmol). Purification by flash chromatography on silica eluting with 1:1 EtOAc: iso-hexane gave the title compound as a pale yellow solid (427mg, 74%).
¹H NMR (DMSO-d₆): δ 5.23 (s, 2H), 6.89-6.92 (m, 2H), 7.23-7.27 (m, 3H), 7.39-7.50 (m, 5H), 8.12 (s, 1H), 9.58 (s, 1H).
MS m/e MH⁺ 263.

### Example 23

### {5-[(E)-2-(2-aminopyrimidin-4-yl)vinyl]-4-phenyl-1H-imidazol-1-yl}acetic acid

Prepared according to general method B from *tert*-butyl (5-formyl-4-phenyl-1*H*-imidazol-1-yl)acetate (Intermediate 5) (285mg, 1mmol) and 4-methylpyrimidin-2-amine (109mg, 1mmol). Purification by preparative HPLC (Acetonitrile:water:TFA, 90:10;0.1) gave the title compound as a yellow solid (24mg, 7%).
¹H NMR (DMSO-d₆): δ 4.60 (bs, 3H), 5.18 (s, 2H), 6.68 (d, 1H), 6.87 (d, 1H), 7.41 (t, 1H), 7.48 (t, 2H), 7.59 (d, 2H), 7.74 (d, 1H), 8.23 (d, 1H), 8.25 (s, 1H). (Contains 10% of *cis* isomer).
MS m/e MH⁺ 322.

### Intermediate 5

### tert-Butyl (5-formyl-4-phenyl-1H-imidazol-1-yl)acetate

Prepared according to general method E from 4-phenyl-1H-imidazole-5-carbaldehyde (516mg, 3mmol) and *tert*-butyl chloroacetate (0.43ml), 3.01mmol). Purification by flash chromatography on silica, eluting with 1:1 EtOAc: iso-hexane gave the title compound as a yellow oil. (597mg, 70%).
¹H NMR (DMSO-d₆): δ 1.42 (s, 9H), 5.05 (s, 2H), 7.42-7.55 (m, 3H), 7.73 (d, 2H), 8.05 (s, 1H), 9.81 (s, 1H).
MS m/e MH⁺ 287.

### Example 24

### 4-{(E)-2-[4-phenyl-1-(2-pyrrolidin-1-ylethyl)-1H-imidazol-5-yl]vinyl}pyrimidin-2-amine

Prepared according to general method B from 4-phenyl-1-(2-pyrrolidin-1-ylethyl)-1*H-*imidazole-5-carbaldehyde (Intermediate 6) (269mg, 1mmol) and 4-methylpyrimidin-2-amine (109mg, 1mmol). Purification by organic work-up, followed by scavenging out unreacted aldehyde with sulfonylhydrazine resin gave the title compound as a yellow solid (37mg, 10%).
¹H NMR (DMSO-d₆): δ 1.70-2.10 (m, 4H), 2.95-3.13 (m, 2H), 3.55-3.64 (m, 4H), 4.60 (bs, 5H), 4.86 (t, 3H), 7.00 (d, 1H), 7.42-7.63 (m, 6H), 7.95 (d, 1H), 8.37 (d, 1H), 8.86 (bs, 1H). (Contains 30% of *cis* isomer, 10% of N-3 regioisomer).
MS m/e MH⁺ 361.

### Intermediate 6

### 4-phenyl-1-(2-pyrrolidin-1-ylethyl)-1H-imidazole-5-carbaldehyde

Prepared according to general method E from 1-(2-chloroethyl)pyrrolidine hydrochloride (510mag, 3mmol) and 4-phenyl-1*H*-imidazole-5-carbaldehyde (516mg, 3mmol). Purification by flash chromatography on silica, eluting with 1:1 EtOAc: iso-hexane gave the title compound as a yellow oil, as a 10:1 mixture of regioisomers. (270mg, 34%).
¹H NMR (DMSO-d₆): δ 1.61-1.68 (m, 4H), ~2.5 (m, obscured by DMSO peak, 4H), 2.74 (t, 2H), 4.40 (t, 2H), 7.41-7.47 (m, 3H), 7.71 (d, 2H), 8.08 (s, 1H), 9.80 (s, 1H). (Contains 10% of N3 regioisomer).
MS m/e MH⁺ 270.

### Example 25

### N-benzyl-4-[(E)-2-(6-phenylimidazo[2,1-b][1,3]thiazol-5-yl)vinyl]pyrimidin-2-amine

Prepared according to general method D from 5-[(*E*)-2-(2-chloropyrimidin-4-yl)vinyl]-6-phenylimidazo[2,1-*b*][1,3]thiazole (Example 4) (50mg, 0.148mmol)) and benzylamine (0.05ml, 0.46mmol) with NMP as solvent. Trituration with cyclohexane gave the title compound as a yellow solid. (20mg, 33%).
¹H NMR (DMSO-d₆): δ 4.45 (d, 2H), 6.65 (d, 1H), 7.00 (d, 1H), 7.10-7.25, (m, 6H), 7.38-7.55 (m, 3H), 7.65 (d, 2H), 8.04 (d, 1H), 8.22 (d, 1H), 8.48 (d, 1H).
MS m/e MH⁺ 410.

### Example 26

### N-methyl-4-[(E)-2-(6-phenylimidazo[2,1-b][1,3]thiazol-5-yl)vinyl]pyrimidin-2-amine

Prepared according to general method D from 5-[(*E*)-2-(2-chloropyrimidin-4-yl)vinyl]-6-phenylimidazo[2,1-*b*][1,3]thiazole (Example 4) (50mg, 0.148mmol) and methylamine (as 2M in THF) (0.75ml, 1.5mmol) with NMP as solvent. Purification by flash chromatography on silica, eluting with 1:1 EtOAc: iso-hexane, gave the title compound as a yellow solid. (15mg, 30%)
¹H NMR (CDCl₃): δ 3.02 (d, 3H), 5.02 (bq, 1H), 6.52 (d, 1H), 6.66 (d, 1H), 6.99 (d, 1H), 7.41 (t, 1H), 7.48 (t, 2H), 7.75 (d, 2H), 7.87 (d, 1H), 8.08 (d, 1H), 8.24 (d, 1H). (Contains 12% *cis* isomer).
MS m/e MH⁺ 334.

### Example 27

### N-(1-phenylethyl)-4-[(E)-2-(6-phenylimidazo[2,1-b][1,3]thiazol-5-yl)vinyl]pyrimidin-2 amine

Prepared according to general method D from 5-[(*E*)-2-(2-chloropyrimidin-4-yl)vinyl]-6-phenylimidazo[2,1-*b*][1,3]thiazole (Example 4) (50mg, 0.148mmol) and (1-phenylethyl)amine (0.5ml, 3.9mmol) with NMP as solvent. Purification by flash chromatography on silica, eluting with 1:1 EtOAc/ iso-hexane, gave the title compound as a yellow solid. (18mg, 29%).
¹H NMR (CDCl₃): δ 1.55 (d, 3H), 5.15-5.27 (m, 1H), 5.40 (bq, 1H), 6.47 (d, 1H), 6.61 (d, 1H), 6.98 (d, 1H), 7.17-7.55 (m, 8H), 7.72 (d, 2H), 7.82 (d, 1H), 8.03 (d, 1H), 8.20 (d, 1H). (Contains 40% starting amine).
MS m/e MH⁺ 424.

### Example 28

### N-phenyl-4-[(E)-2-(6-phenylimidazo[2,1-b][1,3]thiazol-5-yl)vinyl]pyrimidin-2-amine

Prepared according to general method D from 5-[(*E*)-2-(2-chloropyrimidin-4-yl)vinyl]-6-phenylimidazo[2,1-*b*][1,3]thiazole (Example 4) (50mg, 0.148mmol) and aniline (0.04ml, 0.45mmol) with NMP as solvent. Purification by flash chromatography on silica, eluting with 1:1 EtOAc: iso-hexane, gave the title compound as a yellow solid. (40mg, 67%).
¹H NMR (DMSO-d₆): δ 6.86-6.92 (m, 2H), 7.10-7.15 (m, 3H), 7.46-7.56 (m, 4H), 7.68-7.75 (m, 4H), 8.11 (d, 1H), 8.42 (d, 1H), 8.54 (d, 1H), 9.46 (s, 1H).
MS m/e MH⁺ 396.

### Example 29

### 4-[(E)-2-(6-phenylimidazo[2,1-b][1,3]thiazol-5-yl)vinyl]pyrimidin-2-ol

Concentrated hydrochloric acid (0.5 ml) was added to a suspension of 6-phenylimidazo[2,1-*b*][1,3]thiazole-5-carbaldehyde (1.14 g, 5 mmol) and 4-methylpyrimidin-2-ol hydrochloride (732mg, 5mmol) in acetonitrile (10.0 ml) and water (2.0 ml). The resulting mixture was heated to 95°C for 18 hours, cooled and poured into water, sonicating to dissolve and basified with addition of 2MNaOH. The aqueous was washed with EtOAc, acidified and brought to pH8 by addition of saturated NaHCO₃ solution. Extracted into EtOAc, dried and the solvent evaporated to give the title compound as a yellow solid. (315mg, 20%).
¹H NMR (CDCl₃): δ 1.65 (bs, 1H), 6.40 (d, 1H), 6.71 (d, 1H), 6.99 (d, 1H), 7.35-7.53 (m, 3H), 7.67-7.72 (m, 2H), 7.88-8.07 (m, 3H).
MS m/e MH⁺ 321.

### Example 30

### 4-[(E)-2-(6-phenylimidazo[2,1-b][1,3]thiazol-5-yl)vinyl]pyrimidine

Prepared according to general method A from 6-phenylimidazo[2,1-*b*][1,3]thiazole-5-carbaldehyde (46mg, 0.2mmol) and 4-Methylpyrimidine (19mg, 0.2mmol). Purification by preparative reverse phase HPLC (Acetonitrile:water:TFA, 90:10;0.1) gave the title compound as an orange solid. (35mg, 57%).
MS m/e MH⁺ 305.

### Example 31

### 4-methyl-6-[(E)-2-(6-phenylimidazo[2,1-b][1,3]thiazol-5-yl)vinyl]pyrimidin-2-amine

Prepared according to general method A from 6-phenylimidazo[2,1-*b*][1,3]thiazole-5-carbaldehyde (46mg, 0.2mmol) and 4,6-dimethylpyrimidin-2-amine (24.6mg, 0.2mmol) but using sodium ethoxide in ethanol instead of LDA/THF. Purification by preparative reverse phase HPLC (Acetonitrile:water:TFA, 90:10;0.1) gave the title compound as a yellow solid. (6.4mg, 10%).
MS m/e MH⁺ 334.

### Example 32

### 6-[(E)-2-(6-phenylimidazo[2,1-b][1,3]thiazol-5-yl)vinyl]pyrimidin-4-amine

Prepared according to general method A from 6-phenylimidazo[2,1-*b*][1,3]thiazole-5-carbaldehyde (52.4mg, 0.23mmol) and 6-methylpyrimidin-4-amine (25mg, 0.23mmol) but using butyl lithium instead of LDA. Purification by preparative reverse phase HPLC (Acetonitrile:water:TFA, 90:10;0.1) gave the title compound as a yellow solid. (15.7mg, 21%).
MS m/e MH⁺ 320.

### Example 33

### Methyl (2Z)-2-(2-aminopyrimidin-4-yl)-3-(6-phenylimidazo[2,1-b][1,3]thiazol-5-yl)prop-2-enoate.

Prepared according to general method C from 6-phenylimidazo[2,1-*b*][1,3]thiazole-5-carbaldehyde (123mg, 0.54mmol) and methyl (2-aminopyrimidin-4-yl)acetate (90mg; 0.54mmol). Purification by flash chromatography on silica, eluting with 2:98 methanol: chloroform, gave the title compound as a yellow powder. (223mg, 100%).
MS m/e MH⁺ 378.

### Example 34

### (2Z)-2-(2-aminopyrimidin-4-yl)-3-(6phenylimidazo[2,1-b][1,3]thiazol-5-yl)prop-2-enoic acid.

Aqueous 1N NaOH (0.3ml) was added to methyl (2Z)-2-(2-aminopyrimidin-4-yl)-3-(6-phenylimidazo[2,1-*b*][1,3]thiazol-5-yl)prop-2-enoate (Example 33) (105mg, 0.28mmol) in MeOH (20ml). After 24 hours at ambient temperature, analytical RPHPLC confirmed reaction not complete but worked up by preparative RPHPLC (Acetonitrile:water:TFA, 90:10;0.1) to give the title compound as a yellow solid. (28mg, 29%.)
MS m/e (M-1) 362.

### Example 35

### (2Z)-2-(2-aminopyrimidin-4-yl)-N-[4-(4-methylpiperazin-1-yl)cyclohexyl]-3-(6-phenylimidazo[2,1-b][1,3]thiazol-5-yl)prop-2-enamide.

HATU (7.6mg, 40µmol) and TEA (14 µlitre, 100µmol) were added to a solution of 4-(4-methylpiperazin-1-yl)cyclohexanamine (Intermediate 2) (7.9mg, 40µmole) and (2*Z*)-2-(aminopyrimidin-4-yl)-3-(6-phenylimidazo[2,3-*b*][1,3]thiazol-5-yl)prop-2-enoic acid (example 35) (14mg,39µmole) in DMA (2ml). After 16 hours at ambient temperature isolation by preparative RPHPLC (Acetonitrile:water:TFA, 90:10;0.1) gave the title compound as a yellow powder. (24.6mg, 99%).
MS m/e MH⁺ 543.

### Example 36

### 2-(2-aminopyrimidin-4-yl)-N-methyl-3-(6-phenylimidazo[2,1-b][1,3]thiazol-5-yl)acrylamide

HATU (7.6mg, 40µmol) and TEA (14 µlitre, 100µmol) were added to a solution of methylamine (as 2M in THF) (20 µlitre, 100 µmol) and (2Z)-2-(aminopyrimidin-4-yl)-3-(6-phenylimidazo[2,3-*b*][1,3]thiazol-5-yl)prop-2-enoic acid (example 34) (14mg,39µmol) in DMA (2ml). After 16 hours at ambient temperature isolation by preparative RPHPLC (Acetonitrile:water:TFA, 90:10;0.1) gave the title compound as a yellow powder. (13.5mg, 92%).
MS m/e MH⁺ 377.

### Example 37

### 2-(2-aminopyrimidin-4-yl)-3-(6-phenylimidazo[2,1-b][1,3]thiazol-5-yl)prop-2-en-1-ol.

DIBAL in DCM (1M, 0.87ml, 0.87mmol) was added to a solution of methyl (2*Z*)-2-(2-aminopyrimidin-4-yl)-3-(6-phenylimidazo[2,1-*b*][1,3]thiazol-5-yl)prop-2-enoate (example 33) (164mg,0.465mmol) in DCM (5ml) at -70°. After 2 hours at -70°, the reaction was quenched by addition of aqueous HCl (1M, 10ml) and then evaporated. Preparative RPHPLC (Acetonitrile:water:TFA, 90:10;0.1) gave the title compound as a yellow solid as a mixture of *E* and *Z* isomers. (29.8mg, 20%.)
MS m/e MH⁺ 350.
The saturated ester was also present in the reaction product and was isolated, yield 34mg, 21%
MS m/e MH⁺ 380

### Example 38

### 4-[(E)-2-(4-phenyl-1H-imidazol-5-yl)ethenyl]pyridine.

Prepared according to general method B from ethyl 4-pyridylacetate (66mg, 0.4mmol) and 5-phenylimidazo-4-carboxaldehyde (69mg, 0.4mmol). Preparative RPHPLC (Acetonitrile:water:TFA, 90:10;0.1) gave the title compound as a colourless gum. (30mg, 30%).
MS m/e MH⁺ 248.

### Example 39

### 4-[(E)-2-(4-phenyl-1H-imidazol-5-yl)ethenyl]pyrimidine.

Prepared according to general method B from 4-methylpyrimidine (38mg, 0.4mmol) and 4-pheny-1*H*-imidazole-5-carbaldehyde (69mg, 0.4mmol). Crystallised on workup to give the title compound as a colourless solid. (90mg, 90%).
MS m/e MH⁺ 249.

### Example 40

### 4-[(E)-2-(1-methyl-4-phenyl-1H-imidazol-5-yl)ethenyl]pyrimidine.

Prepared according to general method B from 4-methylpyrimidine (31mg, 0.33mmol) and 1-methyl-4-phenyl-1-*H*-imidazole-5-carbaldehyde (Intermediate 3) (61mg, 0.33mmol). Preparative RPHPLC (Acetonitrile:water:TFA, 90:10;0.1) gave the title compound as an hygroscopic yellow solid. (50mg, 57%.)
MS m/e MH⁺ 263.

### Example 41

### 4-[(Z)-2-(1-methyl-4-phenyl-1H-imidazol-5-yl)vinyl]pyridine

Prepared according to general method B from ethyl pyridin-4-ylacetate (31mag, 0.33mmol) and 1-methyl-4-phenyl-1-*H*-imidazole-5-carbaldehyde (Intermediate 3) (61mg, 0.33mmol). Preparative RPHPLC (Acetonitrile:water:TFA, 90:10;0.1) gave the title compound as a yellow solid. (69mg, 80%.)
MS m/e MH⁺ 262.

### Example 42

### 4-[(E)-2-(1-methyl-4-phenyl-1H-imidazol-5-yl)ethenyl]pyrimidin-2-ol.

Prepared according to general method B from 4-methyl-2-hydroxypyrimidine (55mg, 0.5mmol) and 1-methyl-4-phenyl-1*H*-imidazole-5-carbaldehyde (Intermediate 3) (93mg, 0.5mmol). Preparative RPHPLC (Acetonitrile:water:TFA, 90:10;0.1) gave the title compound as a yellow solid. (140mg, 100%).
MS m/e (M-1) 277.

### Example 43

### N-methyl-4-[(E)-2-(1-methyl-4-phenyl-1-H-imidazol-5-yl)ethenyl]pyrimidin-2-amine.

Prepared according to general method B from *N*-4-dimethylpyrimidin-2-amine (62mg,0.5mmol) and 1-methyl-4-phenyl-1*H*-imidazole-5-carbaldehyde (93mg,0.5mmol). Preparative RPHPLC (Acetonitrile:water:TFA, 90:10;0.1) gave the title compound as a yellow solid. (126mg, 86%).
MS m/e MH⁺ 292.

### Example 44

### 4-[(1-methyl-4-phenyl-1H-imidazol-5-yl)ethynyl]-2-(methylthio)pyrimidine

A solution of 4-iodo-2-(methylthio)pyrimidine (WO 99/31061. p151) (0.94g, 3.74mmol), copper(I) iodide (0.026g, 0.136mmol) and dichlorobis(triphenylphosphine)palladium(II) (0.065g, 0.093mmol) in triethylamine (6.3ml) and THF (15ml) was degassed then stirred at RT, under Nitrogen, for 1 hr. 5-ethynyl-1-methyl-4-phenyl-1*H*-imidazole (Intermediate 8)(0.68g, 3.74mmol) was added over 45 mins and stirring continued for 90 mins. Diluted with ethyl acetate (50ml), filtered and the filtrate evaporated to give a brown oil. Purification by flash chromatography on silica, eluting with 99:1 Dichloromethane:methanol gave the title compound as a colourless oil. (1.05g, 92%).
¹H NMR (CDCl₃): δ 2.60 (s, 3H), 3.82 (s, 3H), 7.05 (d, 1H), 7.34 (t, 1H), 7.44 (t, 2H), 7.59 (s, 1H), 8.13 (d, 2H), 8.50 (d, 1H).
MS m/e MH⁺ 307.

### Intermediate 7

### 5-ethynyl-1-methyl-4-phenyl-1H-imidazole

ⁿButyl lithium (1.6M in hexanes) (7.93ml, 12.6mmol) was added to a stirred solution of Trimethylsilyldiazomethane (2M in hexanes) (7.26ml, 14.5mmol), at -78°C, under nitrogen. Stirred at -78°C for 30 mins then 1-methyl-4-phenyl-1*H*-imidazole-5-carbaldehyde (Intermediate 3) (1.8g, 9.68mmol) in THF (10ml) added over 5 mins. Stirred at -78°C for 1hr and at 0°C for 1hr. NH₄Cl_{(aq)} (30ml) was added and extracted into diethyl ether (100ml), dried over MgSO₄, filtered and evaporated to give a brown oil. Purification by flash chromatography on silica, eluting with 99:1 Dichloromethane:methanol gave the title compound as a colourless solid. (0.856g, 49%).
¹H NMR (CDCl₃): δ 3.72 (s, 3H), 3.75 (s, 1H), 7.30 (t, 1H), 7.40 (t, 2H), 7.47 (s, 1H), 8.10 (d, 2H)
MS m/e MH⁺ 183.

### Example 45

### 4-(1-Methyl-4-phenyl-1H-imidazoyl-5-yl)ethynyl)pyrimidin-2-amine

A solution of 4-[(1-methyl-4-phenyl-1*H*-imidazol-5-yl)ethynyl]-2-(methylsulfonyl)pyrimidine (Intermediate 9) (0.5g,1.48mmol) in 1,4-Dioxane (15ml) and 880 Ammonia solution (10ml) was heated in a microwave oven at 150°C for 30 mins. The solvents were evaporated and ethyl acetate (50ml) added, washed with brine (50ml), dried over MgSO₄, filtered and evaporated to give a yellow oil. Trituration with dichloromethane gave the title compound as a yellow solid. (0.19g, 47%).
¹H NMR (CDCl₃): δ 3.80 (s, 3H), 5.12 (s, 2H), 6.78 (d, 1H), 7.33 (t, 1H), 7.43 (t, 2H), 7.57 (s, 1H), 8.12 (d, 2H), 8.31 (d, 1H).
MS m/e MH⁺ 276.

### Intermediate 8

### 4-[(1-methyl-4-phenyl-1H-imidazol-5-yl)ethynyl]-2-(methylsulfonyl)pyrimidine

Oxone (6.33g, 10.29mmol) in water (42ml) was added to a solution of 4-[(1-methyl-4-phenyl-1*H*-imidazol-5-yl)ethynyl]-2-(methylthio)pyrimidine (Example 44) (1.05g, 3.43mmol) in methanol (42ml) and stirred at RT for 3 hrs. The solvents were evaporated and aqueous sodium bicarbonate (20ml) added. Extracted into ethyl acetate (50ml), dried over Magnesium Sulfate, filtered and evaporated to give the title compound as a yellow solid. (0.5g, 43%.)
¹H NMR (CDCl₃): δ 3.40 (s, 3H), 3.84 (s, 3H), 7.39 (t, 1H), 7.48 (t, 2H), 7.57 (d, 1H), 7.62 (s, 1H), 8.11 (d, 2H), 8.88 (d, 1H).
MS m/e MH⁺339

### Example 46

### N-methyl-4-[(1-methyl-4-phenyl-1H-imidazol-5-yl)ethynyl]pyrimidin-2-amine

Peracetic acid (39% in Acetic acid) (0.025ml, 0.14mmol), was added to 4-[(1-methyl-4-phenyl-1*H*-imidazol-5-yl)ethynyl]-2-(methylthio)pyrimidine (Example 44) (0.043g, 0.14mmol) in DMF (0.3ml) and stirred at RT for 30 mins. The solvents were evaporated to give a brown oil. 0.045g. LCMS: A mixture of 4-[(1-methyl-4-phenyl-1H-imidazol-5-yl)ethynyl]-2-(methylsulfinyl)pyrimidine (65%) MH⁺ 323 and 4-[(1-methyl-4-phenyl-1*H-*imidazol-5-yl)ethynyl]-2-(methylsulfonyl)pyrimidine (35%) MH⁺ 339. Used directly as a crude mixture.

Methylamine (2M in THF) (0.5ml, 0.25mmol) was added to the crude mixture of the sulfone and sulfoxide obtained above (0.045g, 0.14mmol) with stirring. Heated at reflux for 90 mins and the solvents evaporated. Purification by flash chromatography on silica, eluting with 99:1 Dichloromethane:methanol gave the title compound as a yellow solid. (16mg, 40%.)
¹H NMR (CDCl₃): δ 3.04 (d, 3H), 3.79 (s, 3H), 5.30 (bs, 1H), 6.69 (d, 1H), 7.34 (t, 1H), 7.44 (t, 2H), 7.57 (s, 1H), 8.17 (d, 2H), 8.31 (d, 1H).
MS m/e MH⁺ 290.

### Example 47

### 4-[(1-methyl-4-phenyl-1H-imidazol-5-yl)ethynyl]pyrimidine

A solution of 4-Bromopyrimidine (intermediate 10) (0.043g, 0.22mmol), copper(I)iodide (1.53mg, 0.008mmol), dichlorobis(triphenylphosphine)palladium(II) (0.004g, 0.0057mmol) in triethylamine (0.38ml) and THF (1ml) was degassed then stirred at RT, under Nitrogen, for 5 mins. 5-ethynyl-1-methyl-4-phenyl-1*H*-imidazole (Intermediate 8) (0.04g, 0.22mmol)) was added and stirring continued for 30 mins. Diluted with ethyl acetate (50ml), filtered and the filtrate evaporated to give a yellow oil. Purification by flash chromatography on silica, eluting with 24:1 Dichloromethane:methanol gave the title compound as a yellow solid. (0.05g, 87%.)
¹H NMR (CDCl₃): δ 3.61 (s, 3H), 7.33 (t, 1H), 7.45 (m, 3H), 7.57 (s, 1H), 8.09 (d, 2H), 8.72 (d, 1H), 9.22 (s, 1H).
MS m/e MH⁺ 261.

### Intermediate 9

### 4-Bromopyrimidine.

Phosphorus oxybromide (15.7g, 54.8mmol) was stirred and heated at 80°C until molten. 4(3*H*)-Pyrimidone (4.8g, 50mmol) was added and heated to 115°C for 4 hrs. Cooled to RT, added to 50% saturated aqueous sodium bicarbonate solution (500ml) and extracted into Dichloromethane (4x250ml). The combined organics were washed with water, dried over Magnesium Sulfate, filtered and the solvent evaporated to give the title compound as a brown solid. 3.37g, 42%.
¹H NMR (CDCl₃): δ 7.55 (d, 1H), 8.51 (d, 1H), 8.95 (s, 1H).

## Claims

1. A compound of the Formula I: 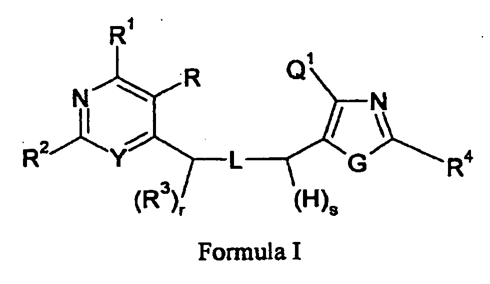 wherein:
**-L-** represents a double bond and r and s each represent 1 or -L- represents a triple bond and r and s each represent 0;
**G** is selected from O, S and NR⁵;
**Y** is selected from N and CR⁶;
**Q¹** is selected from aryl and heteroaryl,
and wherein **Q¹** is optionally substituted by one or more substituents, which may be the same or different, selected from halogeno, trifluoromethyl, trifluoromethoxy, cyano, nitro, hydroxy, amino, carboxy, carbamoyl, (1-6C)alkyl, (2-8C)alkenyl, (2-8C)alkynyl, (1-6C)alkoxy, (2-6C)alkenyloxy, (2-6C)alkynyloxy, (1-6C)alkylthio, (1-6C)alkylsulfinyl, (1-6C)alkylsulfonyl, (1-6C)alkylamino, di-[(1-6C)alkyl]amino, (1-6C)alkoxycarbonyl, N-(1-6C)alkylcarbamoyl, N,N-di-[(1-6C)alkyl]carbamoyl, (2-6C)alkanoyl, (2-6C)alkanoyloxy, (2-6C)alkanoylamino, N-(1-6C)alkyl-(2-6C)alkanoylamino, (3-6C)alkenoylamino, N-(1-6C)alkyl-(3-6C)alkenoylamino, (3-6C)alkynoylamino, N-(1-6C)alkyl-(3-6C)alkynoylamino, N-(1-6C)alkylsulfamoyl, N,N-di-[(1-6C)alkyl]sulfamoyl, (1-6C)alkanesulfonylamino, N-(1-6C)alkyl-(1-6C)alkanesulfonylamino, from a group of the formula:
-X¹-R⁷
wherein X¹ is a direct bond or is selected from O and N(R⁸), wherein R⁸ is hydrogen or (1-6C)alkyl, and R⁷ is halogeno-(1-6C)alkyl, hydroxy-(1-6C)alkyl, (1-6C)alkoxy-(1-6C)alkyl, cyano-(1-6C)alkyl, amino-(1-6C)alkyl, (1-6C)alkylamino-(1-6C)alkyl or di-[(1-6C)alkyl]amino-(1-6C)alkyl, and from a group of the formula:
-X²-Q²
wherein X² is a direct bond or is selected from O, S, SO, SO₂, N(R⁹), CO, CH(OR⁹), CON(R⁹), N(R⁹)CO, N(R⁹)CON(R⁹), SO₂N(R⁹), N(R⁹)SO₂, C(R⁹)₂O, C(R⁹)₂S and N(R⁹)C(R⁹)₂, wherein R⁹ is hydrogen or (1-6C)alkyl, and Q² is aryl, aryl-(1-6C)alkyl, heteroaryl, heteroaryl-(1-6C)alkyl, heterocyclyl or heterocyclyl-(1-6C)alkyl which optionally bears 1, 2 or 3 substituents, which may be the same or different, selected from trifluoromethyl, trifluoromethoxy, cyano, nitro, hydroxy, amino, carboxy, carbamoyl, (1-6C)alkyl, (2-8C)alkenyl, (2-8C)alkynyl, (1-6C)alkoxy, (2-6C)alkenyloxy, (2-6C)alkynyloxy, (1-6C)alkylthio, (1-6C)alkylsulfinyl, (1-6C)alkylsulfonyl, (1-6C)alkylamino, di-[(1-6C)alkyl]amino, (1-6C)alkoxycarbonyl, N-(1-6C)alkylcarbamoyl, N,N-di-[(1-6C)alkyl]carbamoyl, (2-6C)alkanoyl, (2-6C)alkanoyloxy, (2-6C)alkanoylamino, N-(1-6C)alkyl-(2-6C)alkanoylamino, N-(1-6C)alkylsulfamoyl, N,N-di-[(1-6C)alkyl]sulfamoyl, (1-6C)alkanesulfonylamino and N-(1-6C)alkyl-(1-6C)alkanesulfonylamino, or from a group of the formula:
-X³-R¹⁰
wherein X³ is a direct bond or is selected from O and N(R¹¹), wherein R¹¹ is hydrogen or (1-6C)alkyl, and R¹⁰ is halogeno-(1-6C)alkyl, hydroxy-(1-6C)alkyl, (1-6C)alkoxy-(1-6C)alkyl, cyano-(1-6C)alkyl, amino-(1-6C)alkyl, (1-6C)alkylamino-(1-6C)alkyl or di-[(1-6C)alkyl]amino-(1-6C)alkyl,
and any heterocyclyl group within Q² optionally bears 1 or 2 oxo or thioxo substituents;
**R** is selected from hydrogen, amino, hydroxy, halogeno, (1-6C)alkyl, (1-6C)alkoxy, (1-6C)alkylamino, di-[(1-6C)alkyl]amino, carboxy, (1-6C)alkoxycarbonyl and *N*-(heterocyclyl(3-8C)cyclolkyl)carbamoyl;
**R¹** is selected from hydrogen, halogeno, trifluoromethyl, trifluoromethoxy, cyano, nitro, hydroxy, amino, mercapto, carbamoyl, (1-6C)alkyl, (2-8C)alkenyl, (2-8C)alkynyl, (1-6C)alkoxy, (2-6C)alkenyloxy, (2-6C)alkynyloxy, (1-6C)alkylthio, (1-6C)alkylsulfinyl, (1-6C)alkylsulfonyl, (1-6C)alkylamino, di-[(1-6C)alkyl]amino, (1-6C)alkoxycarbonyl, N-(1-6C)alkylcarbamoyl, N,N-di-[(1-6C)alkyl]carbamoyl, (2-6C)alkanoyl, (2-6C)alkanoyloxy, (2-6C)alkanoylamino, N-(1-6C)alkyl-(2-6C)alkanoylamino, (3-6C)alkenoylamino, N-(1-6C)alkyl-(3-6C)alkenoylamino, (3-6C)alkynoylamino, N-(1-6C)alkyl-(3-6C)alkynoylamino, N-(1-6C)alkylsulfamoyl, N,N-di-[(1-6C)alkyl]sulfamoyl, (1-6C)alkanesulfonylamino and N-(1-6C)alkyl-(1-6C)alkanesulfonylamino;
**R²** is selected from hydrogen, halogeno, amino, hydroxy, (1-6C)alkyl, (1-6C)alkoxy, (1-6C)alkylthio, (1-6C)alkylamino, di-[(1-6C)alkyl]amino, aryl(1-6C)alkylamino, arylamino, heterocyclyl and (2-6C)alkanoylamino;
**R³** is selected from hydrogen, (1-6C)alkyl, hydroxy(1-6C)alkyl, carboxy, (1-6C)alkoxycarbonyl, carbamoyl, N-(1-6C)alkylcarbamoyl, N,N-di-[(1-6C)alkyl]carbamoyl and *N*-(heterocyclyl(3-8C)cycloalkyl)carbamoyl;
**R⁵** is, independently, as defined for R⁴ and R⁶, provided that R⁵ is not halogeno;
**R⁴** and **R⁶** which may be the same or different, are selected from hydrogen, halogeno, trifluoromethyl, trifluoromethoxy, cyano, isocyano, nitro, hydroxy, mercapto, amino, formyl, carboxy, carbamoyl, sulfamoyl, (1-6C)alkyl, (2-8C)alkenyl, (2-8C)alkynyl, (1-6C)alkoxy, (2-6C)alkenyloxy, (2-6C)alkynyloxy, (1-6C)alkylthio, (1-6C)alkylsulfinyl, (1-6C)alkylsulfonyl, (1-6C)alkylamino, di-[(1-6C)alkyl]amino, (1-6C)alkoxycarbonyl, N-(1-6C)alkylcarbamoyl, N,N-di-[(1-6C)alkyl]carbamoyl, (2-6C)alkanoyl, (2-6C)alkanoyloxy, (2-6C)alkanoylamino, N-(1-6C)alkyl-(2-6C)alkanoylamino, (3-6C)alkenoylamino, N-(1-6C)alkyl-(3-6C)alkenoylamino, (3-6C)alkynoylamino, N-(1-6C)alkyl-(3-6C)alkynoylamino, N-(1-6C)alkylsulfamoyl, N,N-di-[(1-6C)alkyl]sulfamoyl, (1-6C)alkanesulfonylamino and N-(1-6C)alkyl-(1-6C)alkanesulfonylamino, or from a group of the formula:
Q⁴-X⁵-
wherein X⁵ is a direct bond or is selected from O, S, SO, SO₂, N(R¹²), CO, CH(OR¹²), CON(R¹²), N(R¹²)CO, SO₂N(R¹²), N(R¹²)SO₂, OC(R¹²)₂, SC(R¹²)₂ and N(R¹²)C(R¹²)₂, wherein R¹² is hydrogen or (1-6C)alkyl, and Q⁴ is aryl, aryl-(1-6C)alkyl, (3-7C)cycloalkyl, (3-7C)cycloalkyl-(1-6C)alkyl, (3-7C)cycloalkenyl, (3-7C)cycloalkenyl-(1-6C)alkyl, heteroaryl, heteroaryl-(1-6C)alkyl, heterocyclyl or heterocyclyl-(1-6C)alkyl,
and wherein adjacent carbon atoms in any (2-6C)alkylene chain within an R⁴, R⁵ or R⁶ substituent are optionally separated by the insertion into the chain of a group selected from O, S, SO, SO₂, N(R¹³), CO, CH(OR¹³), CON(R¹³), N(R¹³)CO, SO₂N(R¹³), N(R¹³)SO₂, CH=CH and C≡C wherein R¹³ is hydrogen or (1-6C)alkyl,
and wherein any CH₂=H- or HC≡C- group within an R⁴, R⁵ or R⁶ substituent optionally bears at the terminal CH₂= or HC≡ position a substituent selected from halogeno, carboxy, carbamoyl, (1-6C)alkoxycarbonyl, N-(1-6C)alkylcarbamoyl, N,N-di-[(1-6C)alkyl]carbamoyl, amino-(1-6C)alkyl, (1-6C)alkylamino-(1-6C)alkyl and di-[(1-6C)alkyl]amino-(1-6C)alkyl or from a group of the formula:
Q⁵-X⁶-
wherein X⁶ is a direct bond or is selected from CO and N(R¹⁴)CO, wherein R¹⁴ is hydrogen or (1-6C)alkyl, and Q⁵ is aryl, aryl-(1-6C)alkyl, heteroaryl, heteroaryl-(1-6C)alkyl, heterocyclyl or heterocyclyl-(1-6C)alkyl,
and wherein any CH₂ or CH₃ group within a R⁴, R⁵ or R⁶ substituent optionally bears on each said CH₂ or CH₃ group one or more halogeno or (1-6C)alkyl substituents or a substituent selected from hydroxy, cyano, amino, carboxy, carbamoyl, (1-6C)alkoxy, (1-6C)alkylthio, (1-6C)alkylsulfinyl, (1-6C)alkylsulfonyl, (1-6C)alkylamino, di-[(1-6C)alkyl]amino, (1-6C)alkoxycarbonyl, N-(1-6C)alkylcarbamoyl, N,N-di-[(1-6C)alkyl]carbamoyl, (2-6C)alkanoyl, (2-6C)alkanoyloxy, (2-6C)alkanoylamino, N-(1-6C)alkyl-(2-6C)alkanoylamino, N-(1-6C)alkylsulfamoyl, N,N-di-[(1-6C)alkyl]sulfamoyl, (1-6C)alkanesulfonylamino and N-(1-6C)alkyl-(1-6C)alkanesulfonylamino, or from a group of the formula :
-X⁷-Q⁶
wherein X⁷ is a direct bond or is selected from O, S, SO, SO₂, N(R¹⁵), CO, CH(OR¹⁵), CON(R¹⁵), N(R¹⁵)CO, SO₂N(R¹⁵), N(R¹⁵)SO₂, C(R¹⁵)₂O, C(R¹⁵)₂S and N(R¹⁵)C(R¹⁵)₂, wherein R¹⁵ is hydrogen or (1-6C)alkyl, and Q⁶ is aryl, aryl-(1-6C)alkyl, (3-7C)cycloalkyl, (3-7C)cycloalkyl-(1-6C)alkyl, (3-7C)cycloalkenyl, (3-7C)cycloalkenyl-(1-6C)alkyl, heteroaryl, heteroaryl-(1-6C)alkyl, heterocyclyl or heterocyclyl-(1-6C)alkyl,
and wherein any aryl, heteroaryl, heterocyclyl, cycloalkyl or cycloalkenyl group within a substituent on R⁴, R⁵ or R⁶ optionally bears 1 or more substituents, which may be the same or different, selected from halogeno, trifluoromethyl, trifluoromethoxy, cyano, nitro, hydroxy, amino, carboxy, carbamoyl, (1-6C)alkyl, (2-8C)alkenyl, (2-8C)alkynyl, (1-6C)alkoxy, (2-6C)alkenyloxy, (2-6C)alkynyloxy, (1-6C)allcylthio, (1-6C)alkylsulfinyl, (1-6C)alkylsulfonyl, (1-6C)alkylamino, di-[(1-6C)alkyl]amino, (1-6C)alkoxycarbonyl, N-(1-6C)alkylcarbamoyl, N,N-di-[(1-6C)alkyl]carbamoyl, (2-6C)alkanoyl, (2-6C)alkanoyloxy, (2-6C)alkanoylamino, N-(1-6C)alkyl-(2-6C)alkanoylamino, N-(1-6C)alkylsulfamoyl, N,N-di-[(1-6C)alkyl]sulfamoyl, (1-6C)alkanesulfonylamino, N-(1-6C)alkyl-(1-6C)alkanesulfonylamino, from a group of the formula:
-X⁸-R¹⁶
wherein X⁸ is a direct bond or is selected from O and N(R¹⁷), wherein R¹⁷ is hydrogen or (1-6C)alkyl, and R¹⁶ is halogeno-(1-6C)alkyl, hydroxy-(1-6C)alkyl, (1-6C)alkoxy-(1-6C)alkyl, cyano-(1-6C)alkyl, amino-(1-6C)alkyl, (1-6C)alkylamino-(1-6C)alkyl, di-[(1-6C)alkyl]amino-(1-6C)alkyl, (2-6C)alkanoylamino-(1-6C)alkyl or (1-6C)alkoxycarbonylamino-(1-6C)alkyl, and from a group of the formula :
-X⁹-Q⁷
wherein X⁹ is a direct bond or is selected from O, S, SO, SO₂, N(R¹⁸), CO, CH(OR¹⁸), CON(R¹⁸), N(R¹⁸)CO, SO₂N(R¹⁸), N(R¹⁸)SO₂, C(R¹⁸)₂O, C(R¹⁸)₂S and N(R¹⁸)C(R¹⁸)₂, wherein R¹⁸ is hydrogen or (1-6C)alkyl, and Q⁷ is aryl, aryl-(1-6C)alkyl, (3-7C)cycloalkyl, (3-7C)cycloalkyl-(1-6C)alkyl, (3-7C)cycloalkenyl, (3-7C)cycloalkenyl-(1-6C)alkyl, heteroaryl, heteroaryl-(1-6C)alkyl heterocyclyl or heterocyclyl-(1-6C)alkyl which optionally bears 1 or 2 substituents, which may be the same or different, selected from halogeno, trifluoromethyl, trifluoromethoxy, cyano, nitro, hydroxy, amino, carboxy, carbamoyl, (1-6C)alkyl, (2-8C)alkenyl, (2-8C)alkynyl, (1-6C)alkoxy, (2-6C)alkenyloxy, (2-6C)alkynyloxy, (1-6C)alkylthio, (1-6C)alkylsulfinyl, (1-6C)alkylsulfonyl, (1-6C)alkylamino, di-[(1-6C)alkyl]amino, (1-6C)alkoxycarbonyl, N-(1-6C)alkylcarbamoyl, N,N-di-[(1-6C)alkyl]carbamoyl, (2-6C)alkanoyl, (2-6C)alkanoyloxy, (2-6C)alkanoylamino, N-(1-6C)alkyl-(2-6C)alkanoylamino, N-(1-6C)alkylsulfamoyl, N,N-di-[(1-6C)alkyl]sulfamoyl, (1-6C)alkanesulfonylamino and N-(1-6C)alkyl-(1-6C)alkanesulfonylamino,
or when G is NR⁵, R⁴ and R⁵ together with the atoms to which they are attached form a fused 5- or 6- membered heteroaryl or heterocyclyl ring, and wherein said fused 5- or 6-membered ring optionally bears one or more substituents as defined for R⁴,
and any fused 5- or 6- membered heterocyclyl ring so formed optionally bears 1 or 2 oxo or thioxo substituents,
and wherein any heterocyclyl group within any R⁴, R⁵ or R⁶ substituent optionally bears 1 or 2 oxo or thioxo substituents;
or a pharmaceutically-acceptable salt thereof.
with the proviso that the following compound is excluded:
4-[2-(6-phenylimidazo[2,1-b][1,3]thiazol-5-yl)vinyl]pyrimidin-2-amine.

2. A compound according to Claim 1 as represented by Formula II 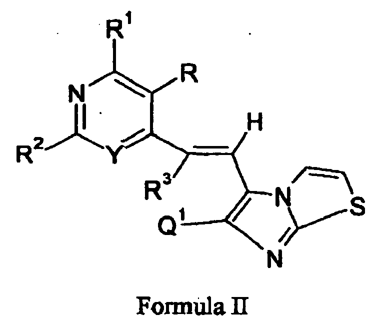 wherein:
**Y, Q¹, R, R¹, R²** and **R³** are as defined in Claim 1;
or a pharmaceutically-acceptable salt thereof.

3. A compound according to Claim 1 as represented by Formula III 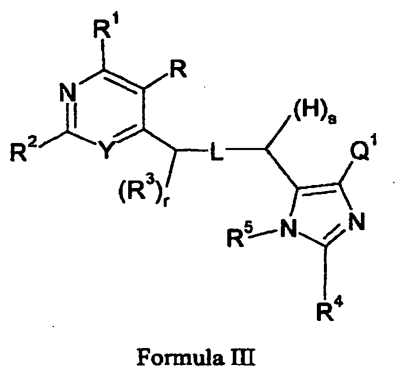 wherein:
**Y, Q¹, L, R, R¹, R², R³, R⁴, R⁵, r** and **s** are as defined in Claim 1;
or a pharmaceutically-acceptable salt thereof.

4. A compound according to any one of the preceding claims wherein R is selected from hydrogen, halogeno, carboxy, (1-6C)alkoxycarbonyl and
*N*-(heterocyclyl(3-8C)cycloalkyl)carbamoyl

5. A compound according to any one of the preceding claims wherein **R¹** is selected from hydrogen, amino and (1-6C)alkyl;

6. A compound according to any one of the preceding claims wherein **R²** is selected from hydrogen, halogeno, hydroxy, amino, (1-6C)alkylthio, (1-6C)alkylamino, di-[(1-6C)alkyl]amino, aryl(1-6C)alkylamino, arylamino, heterocyclyl, (2-6C)alkanoylamino.

7. A compound according to any one of the preceding claims wherein **R³** is selected from hydrogen, carboxy, (1-6C)alkoxycarbonyl, hydroxy(1-6C)alkyl, N-(1-6C)alkylcarbamoyl and N-(heterocyclyl(3-8C)cycloalkyl)carbamoyl.

8. A compound according to any one of the preceding claims wherein **R⁴** is hydrogen.

9. A compound according to any one of the preceding claims wherein **R⁵** is selected from (1-6C)alkyl, aryl(1-6C)alkyl, carboxy(1-6C)alkyl, heterocyclyl(1-6C)alkyl and amino(1-6C)alkyl wherein the amino group is optionally substituted by one or more (1-6C)alkyl.

10. A compound according to any one of the preceding claims wherein **Q¹** is phenyl optionally substituted by halogeno or trifluoromethyl.

11. A pharmaceutical composition which comprises a compound according to any one of claims 1 to 10, or a pharmaceutically acceptable salt thereof, in association with a pharmaceutically-acceptable diluent or carrier.

12. A compound according to any one of claims 1 to 10, or a pharmaceutically-acceptable salt thereof, as defined in claim 1, for use as a medicament

13. Use of a compound according to any one of claims 1 to 10, or a pharmaceutically acceptable salt thereof, in the manufacture of a medicament for use as a Tie2 receptor tyrosine kinase inhibitor in a warm-blooded animal.

14. Use of a compound according to any one of claims 1 to 10, or a pharmaceutically acceptable salt thereof, in the manufacture of a medicament for use in the production of an anti-angiogenic effect in a warm-blooded animal.

15. Use of a compound according to any one of claims 1 to 10, or a pharmaceutically acceptable salt thereof, in the manufacture of a medicament for use in the treatment of cancers in a warm-blooded animal.

16. The use according to claim 15 wherein the cancers are selected from leukaemia, breast, lung, colon, rectal, stomach, prostate, bladder, pancreas, ovarian, lymphoma, testicular, neuroblastoma, hepatic, bile duct, renal cell, uterine, thyroid and skin cancer.

## Patentansprüche

1. Verbindungen der Formel I: 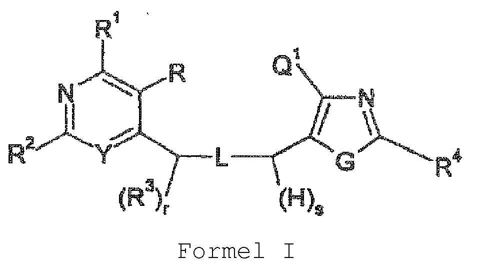 wobei:
**-L-** für eine Doppelbindung steht und r und s jeweils für 1 stehen oder -L- für eine Dreifachbindung steht und r und s jeweils für 0 stehen;
**G** ausgewählt ist aus O, S und NR⁵;
**Y** ausgewählt ist aus N und CR⁶;
**Q¹** ausgewählt ist aus Aryl und Heteroaryl,
und wobei **Q¹** gegebenenfalls substituiert ist durch einen oder mehrere Substitutenen, die gleich oder verschieden sein können, ausgewählt aus Halogen, Trifluormethyl, Trifluormethoxy, Cyano, Nitro, Hydroxy, Amino, Carboxyl, Carbamoyl, (1-6C)-Alkyl, (2-8C)-Alkenyl, (2-8C)-Alkinyl, (1-6C)-Alkoxy, (2-6C)-Alkenyloxy, (2-6C)Alkinyloxy, (1-6C)-Alkylthio, (1-6C)-Alkylsulfinyl, (1-6C)-Alkylsulfonyl, (1-6C)-Alkylamino, Di-[(1-6C)alkyl]amino, (1-6C)-Alkoxycarbonyl, N-(1-6C)-Alkylcarbamoyl, N,N-Di-[(1-6C)alkyl]carbamoyl, (2-6C)-Alkanoyl, (2-6C)-Alkanoyloxy, (2-6C)Alkanoylamino, N-(1-6C)-Alkyl-(2-6C)-alkanoylamino, (3-6C)-Alkenoylamino, N-(1-6C)-Alkyl-(3-6C)-Alkenoylamino, (3-6C)-Alkinoylamino, N-(1-6C)-Alkyl-(3-6C)-alkinoylamino, N-(1-6C)-Alkylsulfamoyl, N,N-Di-[(1-6C)-alkyl]sulfamoyl, (1-6C)-Alkansulfonylamino, N-(1-6C)-Alkyl-(1-6C)-alkansulfonylamino, aus einer Gruppe der Formel:
-X¹-R⁷,
wobei X¹ für eine direkte Bindung steht oder ausgewählt ist aus O und N(R⁸), wobei R⁸ für Wasserstoff oder (1-6C)-Alkyl steht, und R⁷ für Halogen-(1-6C)-alkyl, Hydroxy-(1-6C)-alkyl, (1-6C)-Alkoxy-(1-6C)-alkyl, Cyano-(1-6C)-alkyl, Amino-(1-6C)-alkyl, (1-6C)-Alkylamino-(1-6C)-alkyl oder Di[(1-6C)-alkyl]amino-(1-6C)-alkyl steht, und aus einer Gruppe der Formel:
-X²-Q²,
wobei X² für eine direkte Bindung steht oder ausgewählt ist aus 0, S, SO, SO₂, N(R⁹), CO, CH(OR⁹), CON(R⁹), N(R⁹)CO, N(R⁹)CON(R⁹), SO₂N(R⁹), N(R⁹)SO₂, C(R⁹)₂O, C(R⁹)₂S und N(R⁹)C(R⁹)₂, wobei R⁹ für Wasserstoff oder (1-6C)-Alkyl steht, und Q² für Aryl, Aryl-(1-6C)-alkyl, Heteroaryl, Heteroaryl-(1-6C)-alkyl, Heterocyclyl oder Heterocyclyl-(1-6C)-alkyl steht, das gegebenenfalls 1, 2 oder 3 Substituenten trägt, die gleich oder verschieden sein können, ausgewählt aus Trifluormethyl, Trifluormethoxy, Cyano, Nitro, Hydroxy, Amino, Carboxyl, Carbamoyl, (1-6C)-Alkyl, (2-8C)-Alkenyl, (2-8C)-Alkinyl, (1-6C)-Alkoxy, (2-6C)-Alkenyloxy, (2-6C)-Alkinyloxy, (1-6C)-Alkylthio, (1-6C)-Alkylsulfinyl, (1-6C)-Alkylsulfonyl, (1-6C)-Alkylamino, Di-[(1-6C)-alkyl]amino, (1-6C)-Alkoxycarbonyl, N-(1-6C)-Alkylcarbamoyl, N,N-Di-[(1-6C)-alkyl]carbamoyl, (2-6C)-Alkanoyl, (2-6C)-Alkanoyloxy, (2-6C)-Alkanoylamino, N-(1-6C)-Alkyl-(2-6C)-alkanoylamino, N- (1-6C) - Alkylsulfamoyl, N,N-Di-[(1-6C)-alkyl]sulfamoyl, (1-6C)-Alkansulfonylamino und N-(1-6C)-Alkyl-(1-6C)-alkansulfonylamino, oder aus einer Gruppe der Formel:
-X³-R¹⁰,
wobei X³ für eine direkte Bindung steht oder ausgewählt ist aus 0 und N(R¹¹), wobei R¹¹ für Wasserstoff oder (1-6C)-Alkyl steht und R¹⁰ für Halogen-(1-6C)-alkyl, Hydroxy-(1-6C)-alkyl, (1-6C)-Alkoxy-(1-6C)-alkyl, Cyano-(1-6C)-alkyl, Amino-(1-6C)-alkyl, (1-6C)-Alkylamino-(1-6C)-alkyl oder Di-[(1-6C)-alkyl]amino-(1-6C)-alkyl steht, und eine Heterocyclylgruppe innerhalb von Q² gegebenenfalls 1 oder 2 Oxo- oder Thioxosubstituenten trägt;
**R** ausgewählt ist aus Wasserstoff, Amino, Hydroxy, Halogen, (1-6C)-Alkyl, (1-6C)-alkoxy, (1-6C)-Alkylamino, Di-[(1-6C)-alkyl]amino, Carboxyl, (1-6C)-Alkoxycarbonyl und N-(Heterocyclyl-(3-8C)-cycloalkyl)carbamoyl;
**R¹** ausgewählt ist aus Wasserstoff, Halogen, Trifluormethyl, Trifluormethoxy, Cyano, Nitro, Hydroxy, Amino, Mercapto, Carbamoyl, (1-6C)-Alkyl, (2-8C)-Alkenyl, (2-8C)-Alkinyl, (1-6C)-Alkoxy, (2-6C)-Alkenyloxy, (2-6C)-Alkinyloxy, (1-6C)-Alkylthio, (1-6C)-Alkylsulfinyl, (1-6C)-Alkylsulfonyl, (1-6C)-Alkylamino, Di-[(1-6C)-alkyl]amino, (1-6C)-alkoxycarbonyl, N-(1-6C)-Alkylcarbamoyl, N,N-Di-[(1-6C)-alkyl]carbamoyl, (2-6C)-Alkanoyl, (2-6C)-Alkanoyloxy, (2-6C)-Alkanoylamino, N-(1-6C)-Alkyl-(2-6C)-alkanoylamino, (3-6C)-Alkenoylamino, N-(1-6C)-Alkyl-(3-6C)-alkenoylamino, (3-6C)-Alkinoylamino, N-(1-6C)-Alkyl-(3-6C)-alkinoylamino, N-(1-6C)-Alkylsulfamoyl, N,N-Di-[(1-6C)-Alkyl]sulfamoyl, (1-6C)-Alkansulfonylamino und N-(1-6C)-Alkyl-(1-6C)-alkansulfonylamino;
**R²** ausgewählt ist aus Wasserstoff, Halogen, Amino, Hydroxy, (1-6C)-Alkyl, (1-6C)-Alkoxy, (1-6C)-Alkylthio, (1-6C)-Alkylamino, Di-[(1-6C)-alkyl]amino, Aryl-(1-6C)-alkylamino, Arylamino, Heterocyclyl und (2-6C)-alkanoylamino;
**R³** ausgewählt ist aus Wasserstoff, (1-6C)-Alkyl, Hydroxy-(1-6C)-alkyl, Carboxyl, (1-6C)-Alkoxycarbonyl, Carbamoyl, N-(1-6C)-Alkylcarbamoyl, N,N-Di[(1-6C)-alkyl]carbamoyl und N-(Heterocyclyl-(3-8C)-cycloalkyl)carbamoyl;
**R⁵** unabhängig wie für R⁴ und R⁶ definiert ist, mit der Maßgabe, daß R⁵ nicht für Halogen steht;
**R⁴** und **R⁶,** die gleich oder verschieden sein können, ausgewählt sind aus Wasserstoff, Halogen, Trifluormethyl, Trifluormethoxy, Cyano, Isocyano, Nitro, Hydroxy, Mercapto, Amino, Formyl, Carboxyl, Carbamoyl, Sulfamoyl, (1-6C)-Alkyl, (2-8C)-Alkenyl, (2-8C)-Alkinyl, (1-6C)-Alkoxy, (2-6C)-Alkenyloxy, (2-6C)-Alkinyloxy, (1-6C)-Alkylthio, (1-6C)-Alkylsulfinyl, (1-6C)-Alkylsulfonyl, (1-6C)-Alkylamino, Di-[(1-6C)-alkyl]amino, (1-6C)-Alkoxycarbonyl, N-(1-6C)-Alkylcarbamoyl, N,N-Di-[(1-6C)-alkyl]carbamoyl, (2-6C)-Alkanoyl, (2-6C)-Alkanoyloxy, (2-6C)-Alkanoylamino, N-(1-6C)-Alkyl-(2-6C)-alkanoylamino, (3-6C)-Alkenoylamino, N-(1-6C)-Alkyl-(3-6C)-alkenoylamino, (3-6C)-Alkinoylamino, N-(1-6C)-Alkyl-(3-6C)-alkinoylamino, N-(1-6C)-Alkylsulfamoyl, N,N-Di-[(1-6C)-alkyl]sulfamoyl, (1-6C)-Alkansulfonylamino und N-(1-6C)-Alkyl-(1-6C)-alkansulfonylamino, oder aus einer Gruppe der Formel:
Q⁴-X⁵-,
wobei X⁵ für eine direkte Bindung steht oder ausgewählt ist aus O, S, SO, SO₂, N(R¹²), CO, CH(OR¹²), CON(R¹²), N(R¹²)CO, SO₂N(R¹²), N(R¹²)SO₂, OC(R¹²)₂, SC(R¹²)₂ und N(R¹²)C(R¹²)₂,
wobei R¹² für Wasserstoff oder (1-6C)-Alkyl steht und Q⁴ für Aryl, Aryl-(1-6C)-alkyl, (3-7C)-Cycloalkyl, (3-7C)-Cycloalkyl-(1-6C)-alkyl, (3-7C)-Cycloalkenyl, (3-7C)-Cycloalkenyl-(1-6C)-alkyl, Heteroaryl, Heteroaryl-(1-6C)-alkyl, Heterocyclyl oder Heterocyclyl-(1-6C)-alkyl steht,
und wobei benachbarte Kohlenstoffatome in einer (2-6C)-Alkylenkette in einem R⁴-, R⁵- oder R⁶-Substituenten gegebenenfalls durch Einfügen einer Gruppe ausgewählt aus O, S, SO, SO₂, N (R¹³), CO, CH(OR¹³), CON(R¹³), N(R¹³)CO, SO₂N(R¹³), N(R¹³)SO₂, CH=CH und C=C, wobei R¹³ für Wasserstoff oder (1-6C)-Alkyl steht, in die Kette getrennt sind,
und wobei eine Ch₂=CH- oder HC≡C-Gruppe in einem R⁴-, R⁵- oder R⁶-Substituenten gegebenenfalls an der terminalen CH₂=- oder HC≡-Position einen Substituenten ausgewählt aus Halogen, Carboxyl, Carbamoyl, (1-6C)-Alkoxycarbonyl, N-(1-6C)-Alkylcarbamoyl, N,N-Di-[(1-6C)-alkyl]carbamoyl, Amino-(1-6C)-alkyl, (1-6C)-Alkylamino-(1-6C)-alkyl und Di-[(1-6C)-alkyl]amino-(1-6C)-alkyl oder aus einer Gruppe der Formel:
Q⁵-X⁶-,
wobei X⁶ für eine direkte Bindung steht oder aus CO und N(R¹⁴)CO ausgewählt ist, wobei R¹⁴ für Wasserstoff oder (1-6C)-Alkyl steht und Q⁵ für Aryl, Aryl-(1-6C)-alkyl, Heteroaryl, Heteroaryl-(1-6C)-alkyl, Heterocyclyl oder Heterocyclyl-(1-6C)-alkyl steht, trägt,
und wobei eine CH₂- oder CH₃-Gruppe in einem R⁴-, R⁵- oder R⁶-Substituenten gegebenenfalls an einer CH₂- oder CH₃-Gruppe einen oder mehrere Halogen- oder (1-6C)-Alkyl-Substituenten oder einen Substituenten ausgewählt aus Hydroxy, Cyano, Amino, Carboxyl, Carbamoyl, (1-6C)-Alkoxy, (1-6C)-Alkylthio, (1-6C)-Alkylsulfinyl, (1-6C)-Alkylsulfonyl, (1-6C)-Alkylamino, Di-[(1-6C)-alkyl]amino, (1-6C)-Alkoxycarbonyl, N-(1-6C)-Alkylcarbamoyl, N,N-Di-[(1-6C)-alkyl]carbamoyl, (2-6C)-Alkanoyl, (2-6C)-Alkanoyloxy, (2-6C)-Alkanoylamino, N-(1-6C)-Alkyl-(2-6C)-alkanoylamino, N-(1-6C)-Alkylsulfamoyl, N,N-Di-[(1-6C)-alkyl]sulfamoyl, (1-6C)-Alkansulfonylamino und N-(1-6C)-Alkyl-(1-6C)-alkansulfonylamino oder aus einer Gruppe der Formel:
-X⁷-Q⁶,
wobei X⁷ für eine direkte Bindung steht oder ausgewählt ist aus 0, S, SO, SO₂, N(R¹⁵), CO, CH(OR¹⁵), CON(R¹⁵), N(R¹⁵)CO, SO₂N(R¹⁵), N(R¹⁵)SO₂, C(R¹⁵)₂O, C(R¹⁵)₂S und N(R¹⁵)C(R¹⁵)₂, wobei R¹⁵ für Wasserstoff oder (1-6C)-Alkyl steht und Q⁶ für Aryl, Aryl-(1-6C)-alkyl, (3-7C)-Cycloalkyl, (3-7C)-Cycloalkyl-(1-6C)-alkyl, (3-7C)-Cycloalkenyl, (3-7C)-Cycloalkenyl-(1-6C)-alkyl, Heteroaryl, Heteroaryl-(1-6C)-alkyl, Heterocyclyl oder Heterocyclyl-(1-6C)-alkyl, trägt,
und wobei eine Aryl-, Heteroaryl-, Heterocyclyl-, Cycloalkyl- und Cycloalkenylgruppe in einem Substituenten an R⁴, R⁵ oder R⁶ gegebenenfalls einen oder mehrere Substituenten trägt, die gleich oder verschieden sein können, ausgewählt aus Halogen, Trifluormethyl, Trifluormethoxy, Cyano, Nitro, Hydroxy, Amino, Carboxyl, Carbamoyl, (1-6C)-Alkyl, (2-8C)-Alkenyl, (2-8C)-Alkinyl, (1-6C)Alkoxy, (2-6C)-Alkenyloxy, (2-6C)-Alkinyloxy, (1-6C)-Alkylthio, (1-6C)-Alkylsulfinyl, (1-6C)-Alkylsulfonyl, (1-6C)-Alkylamino, Di-[(1-6C)-alkyl]amino, (1-6C)-Alkoxycarbonyl, N-(1-6C)-Alkylcarbamoyl, N,N-Di-[(1-6C-alkyl]carbamoyl, (2-6C)-Alkanoyl, (2-6C)-Alkanoyloxy, (2-6C)-Alkanoylamino, N-(1-6C)-Alkyl-(2-6C)-alkanoylamino, N-(1-6C)-Alkylsulfamoyl, N,N-Di-[(1-6C)-alkyl]sulfamoyl, (1-6C)-Alkansulfonylamino, N-(1-6C)-Alkyl-(1-6C)-alkansulfonylamino, aus einer Gruppe der Formel:
-X⁸-R¹⁶,
wobei X⁸ für eine direkte Bindung steht oder ausgewählt ist aus O und N(R¹⁷), wobei R¹⁷ für Wasserstoff oder (1-6C)-Alkyl steht und R¹⁶ für Halogen-(1-6C)-alkyl, Hydroxy-(1-6C)-alkyl, (1-6C)-Alkoxy-(1-6C)-alkyl, Cyano-(1-6C)-alkyl, Amino-(1-6C)-alkyl, (1-6C)-Alkylamino-(1-6C)-alkyl, Di-[(1-6C)-alkyl]amino-(1-6C)-alkyl, (2-6C)-Alkanoylamino-(1-6C)-alkyl oder (1-6C)-Alkoxycarbonylamino-(1-6C)-alkyl steht, und aus einer Gruppe der Formel:
-X⁹-Q⁷,
wobei X⁹ für eine direkte Bindung steht oder ausgewählt ist aus 0, S, SO, SO₂, N(R¹⁸), CO, CH(OR¹⁸), CON(R¹⁸), N(R¹⁸)CO, SO₂(N(R¹⁸), N(R¹⁸)SO₂, C(R¹⁸)₂O, C(R¹⁸)₂S und N(R¹⁸)C(R¹⁸)₂, wobei R¹⁸ für Wasserstoff oder (1-6C)-Alkyl steht und Q⁷ für Aryl, Aryl-(1-6C)-alkyl, (3-7C)-Cycloalkyl, (3-7C)-Cycloalkyl-(1-6C)-alkyl, (3-7C)-Cycloalkenyl, (3-7C)-Cycloalkenyl-(1-6C)-alkyl, Heteroaryl, Heteroaryl-(1-6C)-alkyl, Heterocyclyl oder Heterocyclyl-(1-6C)-alkyl steht, welches gegebenenfalls 1 oder 2 Substituenten trägt, die gleich oder verschieden sein können, ausgewählt aus Halogen, Trifluormethyl, Trifluormethoxy, Cyano, Nitro, Hydroxy, Amino, Carboxyl, Carbamoyl, (1-6C)-Alkyl, (2-8C)-Alkenyl, (2-8C)-Alkinyl, (1-6C)-Alkoxy, (2-6C)-Alkenyloxy, (2-6C)-Alkinyloxy, (1-6C)-Alkylthio, (1-6C)-Alkylsulfinyl, (1-6C)-Alkylsulfonyl, (1-6C)-Alkylamino, Di-[(1-6C)-alkyl]amino, (1-6C)-Alkoxycarbonyl, N-(1-6C)-Alkylcarbamoyl, N,N-Di-[(1-6C)-alkyl]carbamoyl, (2-6C)-Alkanoyl, (2-6C)-Alkanoyloxy, (2-6C)-Alkanoylamino, N-(1-6C)-Alkyl-(2-6C)-alkanoylamino, N-(1-6C)-Alkylsulfamoyl, N,N-Di-[(1-6C)-alkyl]sulfamoyl, (1-6C)-Alkansulfonylamino und N-(1-6C)-Alkyl-(1-6C)-Alkansulfonylamino,
oder, wenn G für NR⁵ steht, R⁴ und R⁵ zusammen mit den Atomen, an die sie gebunden sind, einen kondensierten 5- oder 6-gliedrigen Heteroaryl- oder Heterocyclylring bilden, und wobei dieser kondensierte 5- oder 6-gliedrige Ring gegebenenfalls einen oder mehrere wie für R⁴ definierte Substituenten trägt,
und der so gebildete kondensierte 5- oder 6-gliedrige Heterocyclylring gegebenenfalls 1 oder 2 Oxo- oder Thioxosubstituenten trägt,
und wobei eine Heterocyclylgruppe in einem R⁴-, R⁵- oder R⁶-Substituenten gegebenenfalls 1 oder 2 Oxo- oder Thioxosubstituenten trägt;
und deren pharmazeutisch annehmbare Salze,
mit der Maßgabe, daß die folgende Verbindung ausgeschlossen ist:
4-[2-(6-Phenylimidazo[2,1-b][1,3]thiazol-5-yl)vinyl]pyrimidin-2-amin.

2. Verbindungen nach Anspruch 1, wiedergegeben durch die Formel II 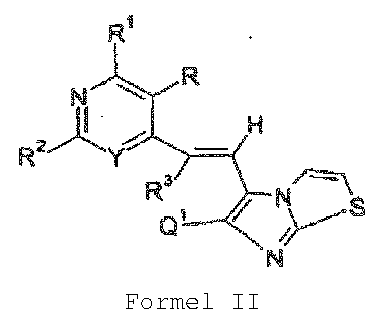 wobei:
**Y, Q¹, R, R¹, R²** und **R³** wie in Anspruch 1 definiert sind;
und deren pharmazeutisch annehmbare Salze.

3. Verbindungen nach Anspruch 1, wiedergegeben durch die Formel III 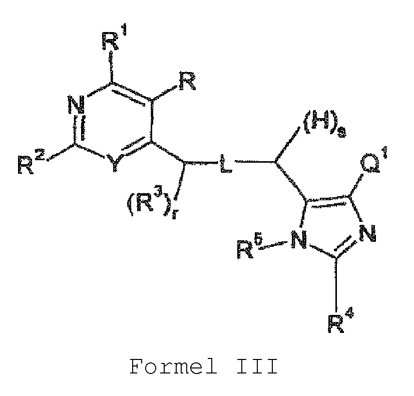 wobei:
**Y, Q¹, L, R, R¹, R², R³, R⁴, R⁵, r** und **s** wie in Anspruch 1 definiert sind;
und deren pharmazeutisch annehmbare Salze.

4. Verbindungen nach einem der vorhergehenden Ansprüche, wobei **R** ausgewählt ist aus Wasserstoff, Halogen, Carboxyl, (1-6C)-Alkoxycarbonyl und N-(Heterocyclyl-(3-8C)-cycloalkyl)carbamoyl.

5. Verbindungen nach einem der vorhergehenden Ansprüche, wobei **R¹** ausgewählt ist aus Wasserstoff, Amino und (1-6C)-Alkyl.

6. Verbindungen nach einem der vorhergehenden Ansprüche, wobei **R²** ausgewählt ist aus Wasserstoff, Halogen, Hydroxy, Amino, (1-6C)-Alkylthio, (1-6C)-Alkylamino, Di-[(1-6C)-alkyl]amino, Aryl-(1-6C)-alkylamino, Arylamino, Heterocyclyl, (2-6C)-Alkanoylamino.

7. Verbindungen nach einem der vorhergehenden Ansprüche, wobei **R³** ausgewählt ist aus Wasserstoff, Carboxyl, (1-6C)-Alkoxycarbonyl, Hydroxy(1-6C)-alkyl, N-(1-6C)-Alkylcarbamoyl und N-(Heterocyclyl-(3-8C)-cycloalkyl)carbamoyl.

8. Verbindungen nach einem der vorhergenden Ansprüche, wobei **R⁴** für Wasserstoff steht.

9. Verbindungen nach einem der vorhergehenden Ansprüche, wobei **R⁵** ausgewählt ist aus (1-6C)-Alkyl, Aryl-(1-6C)-alkyl, Carboxyl-(1-6C)-alkyl, Heterocyclyl-(1-6C)-alkyl und Amino-(1-6C)-alkyl, wobei die Aminogruppe gegebenenfalls durch eins oder mehrere (1-6C)-Alkyl substituiert ist.

10. Verbindungen nach einem der vorhergenden Ansprüche, wobei **Q¹** für gegebenenfalls durch Halogen oder Trifluormethyl substituiertes Phenyl steht.

11. Pharmazeutische Zusammensetzung, enthaltend eine Verbindung nach einem der Ansprüche 1 bis 10 oder ein pharmazeutisch annehmbares Salz davon zusammen mit einem pharmazeutisch annehmbaren Verdünnungsmittel oder einem pharmazeutisch annehmbaren Träger.

12. Verbindungen nach einem der Ansprüche 1 bis 10 und deren pharmazeutisch annehmbare Salze, wie in Anspruch 1 definiert, zur Verwendung als Medikament.

13. Verwendung einer Verbindung nach einem der Ansprüche 1 bis 10 oder eines pharmazeutisch annehmbaren Salzes davon bei der Herstellung eines Medikaments zur Verwendung als Tie2-Rezeptortyrosinkinaseinhibitor in einem Warmblüter.

14. Verwendung einer Verbindung nach einem der Ansprüche 1 bis 10 oder eines pharmazeutisch annehmbaren Salzes davon bei der Herstellung eines Medikaments zur Verwendung beim Hervorrufen einer antiangiogenen Wirkung in einem Warmblüter.

15. Verwendung einer Verbindung nach einem der Ansprüche 1 bis 10 oder eines pharmazeutisch annehmbaren Salzes davon bei der Herstellung eines Medikaments zur Verwendung bei der Behandlung von Krebs in einem Warmblüter.

16. Verwendung nach Anspruch 15, wobei die Krebsart ausgewählt ist aus Leukämie, Brustkrebs, Lungenkrebs, Kolonkarzinom, Rektalkarzinom, Magenkrebs, Prostatakrebs, Blasenkrebs, Bauchspeicheldrüsenkrebs, Eierstockkrebs, Lymphom, Hodenkrebs, Neuroblastom, Leberkarzinom, Karzinom der Gallenwege, Nierenzellenkarzinom, Gebärmutterkrebs, Schilddrüsenkrebs und Hautkrebs.

## Revendications

1. Composé de formule I : 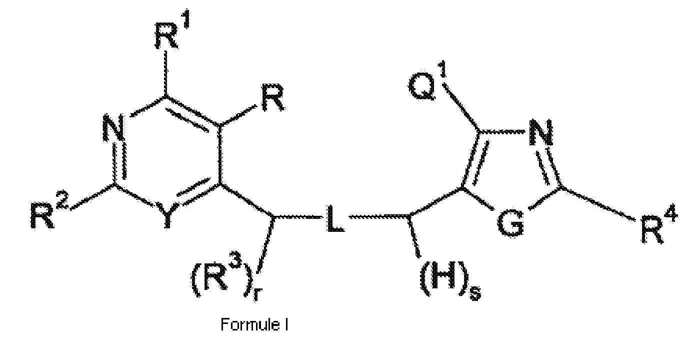 où
-L- représente une double liaison et r et s représentent chacun 1 ou -L- représente une triple liaison et r et s représentent chacun 0 ;
G est choisi parmi O, S et NR⁵ ;
Y est choisi parmi N et CR⁶ ;
Q¹ est choisi parmi aryle et hétéroaryle,
et où Q¹ est éventuellement substitué par un ou plusieurs substituants, qui peuvent être identiques ou différents, choisis parmi halogéno, trifluorométhyle, trifluorométhoxy, cyano, nitro, hydroxy, amino, carboxy, carbamoyle, (1-6C)alkyle, (2-8C)alcényle, (2-8C)alcynyle, (1-6C)alcoxy, (2-6C)alcényloxy, (2-6C)alcynyloxy, (1-6C)alkylthio, (1-6C)alkylsulfinyle, (1-6C)alkylsulfonyle, (1-6C)alkylamino, di-[(1-6C)alkyl]amino, (1-6C)alcoxycarbonyle, N-(1-6C)alkylcarbamoyle, N,N-di[(1-6C)alkyl]carbamoyle, (2-6C)alcanoyle, (2-6C)alcanoyloxy, (2-6C)alcanoylamino, N-(1-6C)alkyl-(2-6C)alcanoylamino, (3-6C)alcénoylamino, N-(1-6C)alkyl-(3-6C)alcénoylamino, (3-6C)alcynoylamino, N-(1-6C)alkyl-(3-6C)alcynoylamino, N-(1-6C)alkylsulfamoyle, N,N-di-[(1-6C)alkyl]sulfamoyle, (1-6C)alcanesulfonylamino, N-(1-6C)alkyl-(1-6C)alcanesulfonylamino, parmi un groupe de formule :
-X¹-R⁷
où X¹ représente une liaison directe ou est choisi parmi O et N(R⁸), où R⁸ représente hydrogène ou (1-6C)alkyle, et R⁷ représente halogéno-(1-6C)alkyle, hydroxy-(1-6C)alkyle, (1-6C)alcoxy-(1-6C)alkyle, cyano-(1-6C)alkyle, amino-(1-6C)alkyle, (1-6C)alkylamino-(1-6C)alkyle ou di-[(1-6C)alkyl]amino-(1-6C)alkyle, et parmi un groupe de formule :
-X²-Q²
où X² représente une liaison directe ou est choisi parmi O, S, SO, SO₂, N(R⁹), CO, CH(OR⁹), CON(R⁹), N(R⁹)CO, N(R⁹)CON(R⁹), SO₂N(R⁹), N(R⁹)SO₂, C(R⁹)₂O, C(R⁹)₂S et N(R⁹)C(R⁹)₂, où R⁹ représente hydrogène ou (1-6C)alkyle, et Q² représente aryle, aryl-(1-6C)alkyle, hétéroaryle, hétéroaryl-(1-6C)alkyle, hétérocyclyle ou hétérocyclyl-(1-6C)alkyle qui porte éventuellement 1, 2 ou 3 substituants qui peuvent être identiques ou différents, choisis parmi trifluorométhyle, trifluorométhoxy, cyano, nitro, hydroxy, amino, carboxy, carbamoyle, (1-6C)alkyle, (2-8C)alcényle, (2-8C)alcynyle, (1-6C) alcoxy, (2-6C) alcényloxy, (2-6C) alcynyloxy, (1-6C)alkylthio, (1-6C)alkylsulfinyle, (1-6C)alkylsulfonyle, (1-6C)alkylamino, di-[(1-6C)alkyl]amino, (1-6C)alcoxycarbonyle, N-(1-6C)alkylcarbamoyle, N,N-di[(1-6C)alkyl]carbamoyle, (2-6C)alcanoyle, (2-6C)alcanoyloxy, (2-6C)alcanoylamino, N-(1-6C)alkyl-(2-6C)alcanoylamino, N-(1-6C)alkylsulfamoyle, N,N-di[(1-6C)alkyl]sulfamoyle, (1-6C)alcanesulfonylamino et N-(1-6C)alkyl-(1-6C)alcanesulfonylamino, ou parmi un groupe de formule :
-X³-R¹⁰
où X³ représente une liaison directe ou est choisi parmi O et N(R¹¹), où R¹¹ représente hydrogène ou (1-6C)alkyle, et R¹⁰ représente halogéno-(1-6C)alkyle, hydroxy-(1-6C)alkyle, (1-6C)alcoxy-(1-6C)alkyle, cyano-(1-6C)alkyle, amino-(1-6C)alkyle, (1-6C)alkylamino-(1-6C)alkyle ou di-[(1-6C)alkyl]amino-(1-6C)alkyle, et n'importe quel groupe hétérocyclyle dans Q² porte éventuellement 1 ou 2 substituants oxo ou thioxo :
R est choisi parmi hydrogène, amino, hydroxy, halogéno, (1-6C)alkyle, (1-6C)alcoxy, (1-6C)alkylamino, di-[(1-6C)alkyl]amino, carboxy, (1-6C)alcoxycarbonyle et N-(hétérocyclyl(3-8C)cycloalkyl)carbamoyle ;
R¹ est choisi parmi hydrogène, halogéno, trifluorométhyle, trifluorométhoxy, cyano, nitro, hydroxy, amino, mercapto, carbamoyle, (1-6C)alkyle, (2-8C)alcényle, (2-8C)alcynyle, (1-6C) alcoxy, (2-6C) alcényloxy, (2-6C) alcynyloxy, (1-6C)alkylthio, (1-6C)alkylsulfinyle, (1-6C)alkylsulfonyle, (1-6C)alkylamino, di[(1-6C)alkyl]amino, (1-6C)alcoxycarbonyle, N-(1-6C)alkylcarbamoyle, N,N-di-[(1-6C)alkyl]carbamoyle, (2-6C)alcanoyle, (2-6C)alcanoyloxy, (2-6C)alcanoylamino, N-(1-6C)alkyl-(2-6C)alcanoylamino, (3-6C)alcénoylamino, N-(1-6C)alkyl-(3-6C)alcénoylamino, (3-6C)alcynoylamino, N-(1-6C)alkyl-(3-6C)alcynoylamino, N-(1-6C)alkylsulfamoyle, N,N-di-[(1-6C)alkyl]sulfamoyle, (1-6C)alcanesulfonylamino et N-(1-6C)alkyl-(1-6C)alcanesulfonylamino ;
R² est choisi parmi hydrogène, halogéno, amino, hydroxy, (1-6C)alkyle, (1-6C)alcoxy, (1-6C)alkylamino, (1-6C)alkylamino, di-[(1-6C)alkyl]amino, aryl(1-6C)alkylamino, arylamino, hétérocyclyle et (2-6C)alcanoylamino ;
R³ est choisi parmi hydrogène, (1-6C)alkyle, hydroxy(1-6C)alkyle, carboxy, (1-6C)alcoxycarbonyle, carbamoyle, N-(1-6C)alkylcarbamoyle, N,N-di-[(1-6C)alkyl]carbamoyle et N-(hétérocyclyl(3-8C)cycloalkyl)carbamoyle ;
R⁵ est, indépendamment, tel que défini pour R⁴ et R⁶, à condition que R⁵ ne représente pas halogéno ;
R⁴ et R⁶ qui peuvent être identiques ou différents, sont choisis parmi hydrogène, halogéno, trifluorométhyle, trifluorométhoxy, cyano, isocyano, nitro, hydroxy, mercapto, amino, formyle, carboxy, carbamoyle, sulfamoyle, (1-6C)alkyle, (2-8C)alcényle, (2-8C)alcynyle, (1-6C) alcoxy, (2-6C) alcényloxy, (2-6C) alcynyloxy, (1-6C)alkylthio, (1-6C)alkylsulfinyle, (1-6C)alkylsulfonyle, (1-6C)alkylamino, di-[(1-6C)alkyl]amino, (1-6C)alcoxycarbonyle, N-(1-6C)alkylcarbamoyle, N,N-di-[(1-6C)alkyl]carbamoyle, (2-6C)alcanoyle, (2-6C)alcanoyloxy, (2-6C)alcanoylamino, N-(1-6C)alkyl-(2-6C)alcanoylamino, (3-6C)alcénoylamino, N-(1-6C)alkyl-(3-6C)alcénoylamino, (3-6C)alcynoylamino, N-(1-6C)alkyl-(3-6C)alcynoylamino, N-(1-6C)alkylsulfamoyle, N,N-di-[(1-6C)alkyl]sulfamoyle, (1-6C)alcanesulfonylamino et N-(1-6C)alkyl-(1-6C)alcanesulfonylamino, ou parmi un groupe de formule :
Q⁴-X⁵-
où X⁵ représente une liaison directe ou est choisi parmi O, S, SO, SO₂, N(R¹²), CO, CH(OR¹²), CON(R¹²)' N(R¹²)CO, SO₂N(R¹²), N(R¹²)SO₂, OC(R¹²)₂, SC(R¹²)₂ et N(R¹²)C(R¹²)₂,
où R¹² représente hydrogène ou (1-6C)alkyle, et Q⁴ représente aryle, aryl-(1-6C)alkyle, (3-7C)cycloalkyle, (3-7C)cycloalkyl-(1-6C)alkyle, (3-7C)cycloalcényle, (3-7C)cycloalcényl-(1-6C)alkyle, hétéroaryle, hétéroaryl-(1-6C)alkyle, hétérocyclyle ou hétérocyclyl-(1-6C)alkyle,
et où des atomes de carbone adjacents dans n'importe quelle chaîne (2-6C)alkylène dans un substituant R⁴, R⁵ ou R⁶ sont éventuellement séparés par l'insertion dans la chaîne d'un groupe choisi parmi O, S, SO, SO₂, N(R¹³), CO, CH(OR¹³), CON(R¹³), N(R¹³)CO, SO₂N(R¹³), N(R¹³)SO₂, CH=CH et C≡C où R¹³ représente hydrogène ou (1-6C)alkyle,
et où n'importe quel groupe CH₂=CH- ou HC≡C- dans les substituants R⁴, R⁵ ou R⁶ porte éventuellement au niveau de l'extrémité CH₂= ou HC≡ un substituant choisi parmi halogéno, carboxy, carbamoyle, (1-6C)alcoxycarbonyle, N-(1-6C)alkylcarbamoyle, N,N-di-[(1-6C)alkyl]carbamoyle, amino-(1-6C)alkyle, (1-6C)alkylamino-(1-6C)alkyle et di-[(1-6C)alkyl]amino-(1-6C)alkyle ou parmi un groupe de formule :
Q⁵-X⁶-
où X⁶ représente une liaison directe ou est choisi parmi CO et N(R¹⁴)CO, où R¹⁴ représente hydrogène ou (1-6C)alkyle, et Q⁵ représente aryle, aryl-(1-6C)alkyle, hétéroaryle, hétéroaryl-(1-6C)alkyle, hétérocyclyle ou hétérocyclyl-(1-6C)alkyle,
et où n'importe quel groupe CH₂ ou CH₃ dans un substituant R⁴, R⁵ ou R⁶ porte éventuellement, sur chaque groupe CH₂ ou CH₃, un ou plusieurs substituants halogéno ou (1-6C)alkyle ou un substituant choisi parmi hydroxy, cyano, amino, carboxy, carbamoyle, (1-6C)alcoxy, (1-6C)alkylthio, (1-6C)alkylsulfinyle, (1-6C)alkylsulfonyle, (1-6C)alkylamino, di-[(1-6C)alkyl]amino, (1-6C)alcoxycarbonyle, N-(1-6C)alkylcarbamoyle, N,N-di-[(1-6C)alkyl]carbamoyle, (2-6C)alcanoyle, (2-6C)alcanoyloxy, (2-6C)alcanoylamino, N-(1-6C)alkyl-(2-6C)alcanoylamino, N-(1-6C)alkylsulfamoyle, N,N-di-[(1-6C)alkyl]sulfamoyle, (1-6C)alcanesulfonylamino et N-(1-6C)alkyl-(1-6C)alcanesulfonylamino, ou parmi un groupe de formule :
-X⁷-Q⁶
où X⁷ représente une liaison directe ou est choisi parmi O, S, SO, SO₂, N(R¹⁵), CO, CH(OR¹⁵), CON(R¹⁵), N(R¹⁵)CO, SO₂N(R¹⁵), N(R¹⁵)SO₂, C(R¹⁵)₂O, C(R¹⁵)₂S et N (R¹⁵)C(R¹⁵)₂,
où R¹⁵ représente hydrogène ou (1-6C)alkyle, et Q⁶ représente aryle, aryl-(1-6C)alkyle, (3-7C)cycloalkyle, (3-7C)cycloalkyl-(1-6C)alkyle, (3-7C)cycloalcényle, (3-7C)cycloalcényl-(1-6C)alkyle, hétéroaryle, hétéroaryl-(1-6C)alkyle, hétérocyclyle ou hétérocyclyl-(1-6C)alkyle,
et où n'importe quel groupe aryle, hétéroaryle, hétérocyclyle, cycloalkyle ou cycloalcényle dans un substituant sur R⁴, R⁵ ou R⁶ porte éventuellement 1 ou plusieurs substituants, qui peuvent être identiques ou différents, choisis parmi halogéno, trifluorométhyle, trifluorométhoxy, cyano, nitro, hydroxy, amino, carboxy, carbamoyle, (1-6C)alkyle, (2-8C)alcényle, (2-8C)alcynyle, (1-6C)alcoxy, (2-6C)alcényloxy, (2-6C)alcynyloxy, (1-6C)alkylthio, (1-6C)alkylsulfinyle, (1-6C)alkylsulfonyle, (1-6C)alkylamino, di-[(1-6C)alkyl]amino, (1-6C)alcoxycarbonyle, N-(1-6C)alkylcarbamoyle, N,N-di-[(1-6C)alkyl]carbamoyle, (2-6C)alcanoyle, (2-6C)alcanoyloxy, (2-6C)alcanoylamino, N-(1-6C)alkyl-(2-6C)alcanoylamino, N-(1-6C)alkylsulfamoyle, N,N-di[(1-6C)alkyl]sulfamoyle, (1-6C)alcanesulfonylamino, N-(1-6C)alkyl-(1-6C)alcanesulfonylamino, parmi un groupe de formule :
-X⁸-R¹⁶
où X⁸ représente une liaison directe ou est choisi parmi O et N(R¹⁷), où R¹⁷ représente hydrogène ou (1-6C)alkyle, et R¹⁶ représente halogéno-(1-6C)alkyle, hydroxy-(1-6C)alkyle, (1-6C)alcoxy-(1-6C)alkyle, cyano-(1-6C)alkyle, amino-(1-6C)alkyle, (1-6C)alkylamino-(1-6C)alkyle, di-[(1-6C)alkyl]amino-(1-6C)alkyle, (2-6C)alcanoylamino-(1-6C)alkyle ou (1-6C)alcoxycarbonylamino-(1-6C)alkyle, et parmi un groupe de formule :
-X⁹-Q⁷
où X⁹ représente une liaison directe ou est choisi parmi 0, S, SO, SO₂, N (R¹⁸), CO, CH (OR¹⁸), CON(R¹⁸), N(R¹⁸)CO, SO₂N(R¹⁸), N(R¹⁸)SO₂, C(R¹⁸)₂O, C(R¹⁸)₂S et N (R¹⁸)C(R¹⁸)₂,
où R¹⁸ représente hydrogène ou (1-6C)alkyle, et Q⁷ représente aryle, aryl-(1-6C)alkyle, (3-7C)cycloalkyle, (3-7C)cycloalkyl-(1-6C)alkyle, (3-7C)cycloalcényle, (3-7C)cycloalcényl-(1-6C)alkyle, hétéroaryle, hétéroaryl-(1-6C)alkyle, hétérocyclyle ou hétérocyclyl-(1-6C)alkyle qui porte éventuellement 1 ou 2 substituants, qui peuvent être identiques ou différents, choisis parmi halogéno, trifluorométhyle, trifluorométhoxy, cyano, nitro, hydroxy, amino, carboxy, carbamoyle, (1-6C)alkyle, (2-8C)alcényle, (2-8C)alcynyle, (1-6C)alcoxy, (2-6C)alcényloxy, (2-6C)alcynyloxy, (1-6C)alkylthio, (1-6C)alkylsulfinyle, (1-6C)alkylsulfonyle, (1-6C)alkylamino, di-[(1-6C)alkyl]amino, (1-6C)alcoxycarbonyle, N-(1-6C)alkylcarbamoyle, N,N-di-[(1-6C)alkyl]carbamoyle, (2-6C)alcanoyle, (2-6C)alcanoyloxy, (2-6C)alcanoylamino, N-(1-6C)alkyl-(2-6C)alcanoylamino, N-(1-6C)alkylsulfamoyle, N,N-di-[(1-6C)alkyl]sulfamoyle, (1-6C)alcanesulfonylamino et N-(1-6C)alkyl-(1-6C)alcanesulfonylamino,
ou, lorsque G représente NR⁵, R⁴ et R⁵ forment, ensemble avec les atomes auxquels ils sont attachés, un cycle hétéroaryle ou hétérocyclyle condensé à 5 ou 6 chaînons, et où ledit cycle condensé à 5 ou 6 chaînons porte éventuellement un ou plusieurs substituants tel que définis pour R⁴,
et n'importe quel cycle hétérocyclyle condensé à 5 ou 6 chaînons ainsi forme porte éventuellement 1 ou 2 substituants oxo ou thioxo,
et où n'importe quel groupe hétérocyclyle dans n'importe quel substituant R⁴, R⁵ ou R⁶ porte éventuellement 1 ou 2 substituants oxo ou thioxo ; ou un sel pharmaceutiquement acceptable de celui-ci, à condition que le composé suivant soit exclu : 4-[2-(6-phénylimidazo[2,1-b][1,3]thiazol-5-yl)vinyl]pyrimidin-2-amine.

2. Composé selon la revendication 1 tel que représenté par la formule II 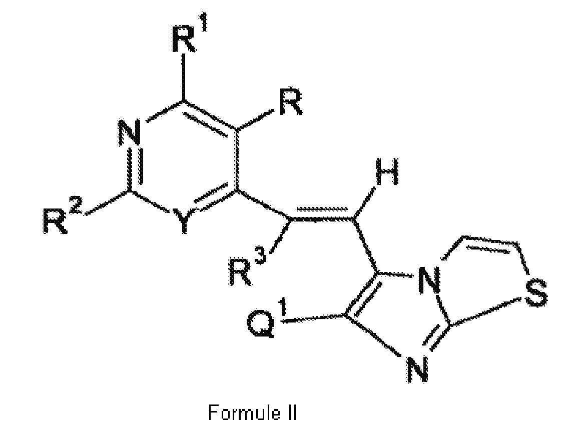 où Y, Q¹, R, R¹, R² et R³ sont tels définis dans la revendication 1 ou un sel pharmaceutiquement acceptable de celui-ci.

3. Composé selon la revendication 1 tel que représenté par la formule III 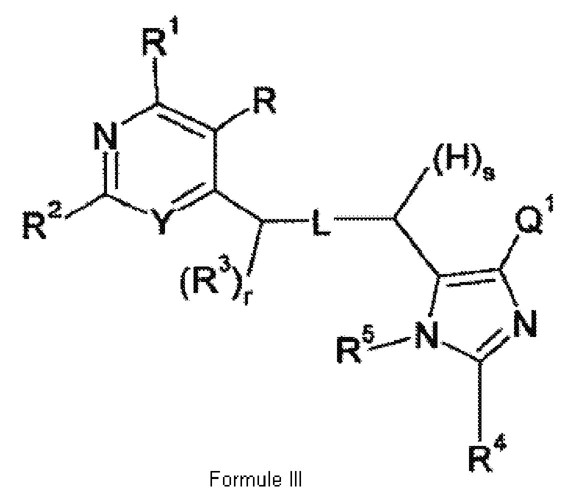 où
Y, Q¹, L, R, R¹, R², R³, R⁴, R⁵, et s sont tels définis dans la revendication 1 ; ou un sel pharmaceutiquement acceptable de celui-ci.

4. Composé selon l'une quelconque des revendications précédentes, où R est choisi parmi hydrogène, halogéno, carboxy, (1-6C)alcoxycarbonyle et N-(hétérocyclyl(3-8C)cycloalkyl)carbamoyle.

5. Composé selon l'une quelconque des revendications précédentes, où R¹ est choisi parmi hydrogène, amino et (1-6C)alkyle.

6. Composé selon l'une quelconque des revendications précédentes, où R² est choisi parmi hydrogène, halogéno, hydroxy, amino, (1-6C)alkylthio, (1-6C)alkylamino, di-[(1-6C)alkyl]amino, aryl(1-6C)alkylamino, arylamino, hétérocyclyle, (2-6C)alcanoylamino.

7. Composé selon l'une quelconque des revendications précédentes, où R³ est choisi parmi hydrogène, carboxy, (1-6C)alcoxycarbonyle, hydroxy(1-6C)alkyle, N-(1-6C)alkylcarbamoyle et N-(hétérocyclyl(3-8C)cycloalkyl)carbamoyle.

8. Composé selon l'une quelconque des revendications précédentes, où R⁴ représente hydrogène.

9. Composé selon l'une quelconque des revendications précédentes, où R⁵ est choisi parmi (1-6C)alkyle, aryl(1-6C)alkyle, carboxy(1-6C)alkyle, hétérocyclyl(1-6C)alkyle et amino(1-6C)alkyle, où le groupe amino est éventuellement substitué par un ou plusieurs groupes (1-6C)alkyle.

10. Composé selon l'une quelconque des revendications précédentes, où Q¹ représente phényle, éventuellement substitué par halogéno ou trifluorométhyle.

11. Composition pharmaceutique qui comprend un composé selon l'une quelconque des revendications 1 à 10, ou un sel pharmaceutiquement acceptable de celui-ci en association avec un diluant ou un support pharmaceutiquement acceptable.

12. Composé selon l'une quelconque des revendications 1 à 10, ou un sel pharmaceutiquement acceptable de celui-ci, tel que défini dans la revendication 1, destiné à une utilisation comme médicament.

13. Utilisation d'un composé selon l'une quelconque des revendications 1 à 10, ou un sel pharmaceutiquement acceptable de celui-ci, dans la préparation d'un médicament destiné à une utilisation comme inhibiteur de la tyrosine kinase d'un récepteur Tie2 chez un animal à sang chaud.

14. Utilisation d'un composé selon l'une quelconque des revendications 1 à 10, ou d'un sel pharmaceutiquement acceptable de celui-ci, dans la préparation d'un médicament destiné à la production d'un effet anti-angiogénique chez un animal à sang chaud.

15. Utilisation d'un composé selon l'une quelconque des revendications 1 à 10, ou d'un sel pharmaceutiquement acceptable de celui-ci, dans la préparation d'un médicament destiné à une utilisation dans le traitement de cancers chez un animal à sang chaud.

16. Utilisation selon la revendication 15 où les cancers sont choisis parmi la leucémie, le cancer du sein, du poumon, du colon, rectal, de l'estomac, de la prostate, de la vessie, du pancréas, de l'ovaire, les lymphomes, le cancer des testicules, le neuroblastome, le cancer du foie, du tractus biliaire, des cellules rénales, de l'utérus, de la thyroïde et de la peau.
